# EUROPEAN PATENT SPECIFICATION

(11) **EP 1 453 471 B1**
(45) Date of publication and mention of the grant of the patent: **05.01.2011**
(21) Application number: 02806695.9
(22) Date of filing: 07.11.2002
(51) Int. Cl.: C12Q 1/37, A61K 39/00, C07K 14/18

(54) **EXPRESSION VECTORS ENCODING EPITOPES OF ANTIGENS AND METHODS FOR THEIR DESIGN**
FÜR EPITOPE VON ANTIGENEN KODIERENDE EXPRESSIONSVEKTOREN UND VERFAHREN ZU DEREN KONZEPTION
VECTEURS D'EXPRESSION CODANT POUR DES EPITOPES D'ANTIGENES ET PROCEDES PERMETTANT LEUR CONCEPTION

(30) Priority: 07.11.2001 US 336968 P
(43) Date of publication of application: 08.09.2004
(60) Divisional application: 10180603.2
(73) Proprietor: Mannkind Corporation, Valencia, California 91355 (US)
(72) Inventor: SIMARD, John J.L., Vancouver, British Columbia Z6G1A6 (CA); DIAMOND, David C., West Hills, CA 91304 (US); QUI, Zhiyong, Los Angeles, CA 90066 (US); LEI, Xiang-Dong, West Hills, CA 91307 (US)
(74) Representative: Maiwald Patentanwalts GmbH
(86) International application number: PCT/US2002/036098
(87) International publication number: WO 2003/063770

(56) References cited:
- EP-A1- 1 118 860
- WO-A-01/82963
- WO-A-03/011331
- WO-A1-99/24596
- DATABASE GENESEQ [Online] Derwent; 16 July 2001 (2001-07-16), SCHADENDORF ET AL.: "Listeria expression vector for immunotherapy, particularly of malignant melanoma, comprises a DNA sequence encoding tumor-associated antigens." XP002401738 retrieved from GENESEQ Database accession no. AAB86042
- MOREL S. ET AL.: 'Processing of some antigens by the standard proteasome but not by the immunoproteasome results in poor presentation by drendritic cells' IMMUNITY vol. 12, January 2000, pages 107 - 117, XP002183661
- VAN DEN EYNDE B. ET AL.: 'Differential processing of class-I-restricted epitopes by the standard proteasome and the immunoproteasome' CURRENT OPINION IN IMMUNOLOGY vol. 13, April 2001, pages 147 - 153, XP004257777
- VALMORI D. ET AL.: 'Induction of potent antitumor CTL responses by recombinant vaccinia encoding a Melan-A peptide analogue' JOURNAL OF IMMUNOLOGY vol. 164, January 2000, pages 1125 - 1131, XP002139352

## Description

### Background of the Invention

### Field of the Invention

The invention disclosed herein is directed to methods for the design of epitope-encoding vectors for use in compositions, including for example, pharmaceutical compositions capable of inducing an immune response in a subject to whom the compositions are administered. The invention is further directed to the vectors themselves. The epitope(s) expressed using such vectors can stimulate a cellular immune response against a target cell displaying the epitope(s).

### Description of the Related Art

The immune system can be categorized into two discrete effector arms. The first is innate immunity, which involves numerous cellular components and soluble factors that respond to all infectious challenges. The other is the adaptive immune response, which is customized to respond specifically to precise epitopes from infectious agents. The adaptive immune response is further broken down into two effector arms known as the humoral and cellular immune systems. The humoral arm is centered on the production of antibodies by B-lymphocytes while the cellular arm involves the killer cell activity of cytotoxic T Lymphocytes.

Cytotoxic T Lymphocytes (CTL) do not recognize epitopes on the infectious agents themselves. Rather, CTL detect fragments of antigens derived from infectious agents that are displayed on the surface of infected cells. As a result antigens are visible to CTL only after they have been processed by the infected cell and thus displayed on the surface of the cell.

The antigen processing and display system on the surface of cells has been well established. CTL recognize short peptide antigens, which are displayed on the surface in noncovalent association with class I major histocompatibility complex molecules (MHC). These class I peptides are in turn derived from the degradation of cytosolic proteins.

### Summary of the Invention

Disclosed herein are expression cassettes, for example, for use in vaccine vectors, which encode one or more embedded housekeeping epitopes, and methods for designing and testing such expression cassettes. Housekeeping epitopes can be liberated from the translation product of such cassettes through proteolytic processing by the immunoproteasome of professional antigen presenting cells (pAPC). Disclosed herein are sequences flanking the housekeeping epitope(s) that can be altered to promote cleavage by the immunoproteasome at the desired location(s). Housekeeping epitopes, their uses, and identification are described in U.S. Patent Application Nos. 09/560,465 and 09/561,074 entitled EPITOPE SYNCHRONIZATION IN ANTIGEN PRESENTING CELLS, and METHOD OF EPITOPE DISCOVERY, respectively; both of which were filed on April 28, 2000.

Examples of housekeeping epitopes are disclosed in provisional U.S. Patent Applications entitled EPITOPE SEQUENCES, Nos. 60/282,211, filed on April 6, 2001; 60/337,017, filed on November 7, 2001; 60/363210 filed 3/7/02; and 60/409,123, filed on September 5, 2002; and U.S. Application No. 10/117,937, filed on April 4, 2002, which is also entitled EPITOPE SEQUENCES.

Disclosed herein are housekeeping epitope(s) that can be flanked by arbitrary sequences or by sequences incorporating residues known to be favored in immunoproteasome cleavage sites. As used herein the term "arbitrary sequences" refers to sequences chosen without reference to the native sequence context of the epitope, their ability to promote processing, or immunological function. In further embodiments of the invention multiple epitopes can be arrayed head-to-tail. These arrays can be made up entirely of housekeeping epitopes. Likewise, the arrays can include alternating housekeeping and immune epitopes. Alternatively, the arrays can include housekeeping epitopes flanked by immune epitopes, whether complete or distally truncated. Further, the arrays can be of any other similar arrangement. There is no restriction on placing a housekeeping epitope at the terminal positions of the array. The vectors can additionally contain authentic protein coding sequences or segments thereof containing epitope clusters as a source of immune epitopes. The term "authentic" refers to natural protein sequences.

Epitope clusters and their uses are described in U.S. Patent application Nos. 09/561,571 entitled EPITOPE CLUSTERS, filed on April 28, 2000; 10/005,905, entitled EPITOPE SYNCHRONIZATION IN ANTIGEN PRESENTING CELLS, filed on November 7, 2001; and 10/026,066, filed on December 7, 2001, also entitled EPITOPE SYNCHRONIZATION IN ANTIGEN PRESENTING CELLS.

Disclosed herein are methods of screening the constructs to determine whether the housekeeping epitope is liberated. In constructs containing multiple housekeeping epitopes, embodiments can include screening to determine which epitopes are liberated. Also described herein is, a vector containing an embedded epitope that can be used to immunize HLA transgenic mice and the resultant CTL can be tested for their ability to recognize target cells presenting the mature epitope. Target cells expressing immunoproteasome can be transformed with the vector. The target cell may express immunoproteasome either constitutively, because of treatment with interferon (IFN), or through genetic manipulation, for example. CTL that recognize the mature epitope can be tested for their ability to recognize these target cells. The embedded epitope can be prepared as a synthetic peptide. The synthetic peptide then can be subjected to digestion by an immunoproteasome preparation *in vitro* and the resultant fragments can be analyzed to determine the sites of cleavage. Such polypeptides, recombinant or synthetic, from which embedded epitopes can be successfully liberated, can also be incorporated into immunogenic compositions.

The invention disclosed herein relates to the identification of a polypeptide suitable for epitope liberation. Disclosed herein is a method of identifying a polypeptide suitable for epitope liberation including, for example, the steps of identifying an epitope of interest; providing a substrate polypeptide sequence including the epitope, wherein the substrate polypeptide permits processing by a proteasome; contacting the substrate polypeptide with a composition including the proteasome, under conditions that support processing of the substrate polypeptide by the proteasome; and assaying for liberation of the epitope.

The epitope can be embedded in the substrate polypeptide, and in some aspects the substrate polypeptide can include more than one epitope, for example. Also, the epitope can be a housekeeping epitope.

It is disclosed that, the substrate polypeptide can be a synthetic peptide. Optionally, the substrate polypeptide can be included in a formulation promoting protein transfer. Alternatively, the substrate polypeptide can be a fusion protein. The fusion protein can further include a protein domain possessing protein transfer activity. Further, the contacting step can include immunization with the substrate polypeptide.

It is described herein that the substrate polypeptide can be encoded by a polynucleotide. The contacting step can include immunization with a vector including the polynucleotide, for example. The immunization can be carried out in an HLA-transgenic mouse or any other suitable animal, for example. Alternatively, the contacting step can include transforming a cell with a vector including the polynucleotide. It is described that the transformed cell can be a target cell that is targeted by CTL for purposes of assaying for proper liberation of epitope.

The proteasome processing can take place intracellularly, either *in vitro* or *in vivo.* Further, the proteasome processing can take place in a cell-free system.

The assaying step can include a technique selected from the group including, but not limited to, mass spectrometry, N-terminal pool sequencing, HPLC, and the like. Also, the assaying step can include a T cell target recognition assay. The T cell target recognition assay can be selected from the group including, but not limited to, a cytolytic activity assay, a chromium release assay, a cytokine assay an ELISPOT assay, tetramer analysis, and the like.

It is described herein that, the amino acid sequence of the substrate polypeptide including the epitope can be arbitrary. Also, the substrate polypeptide in which the epitope is embedded can be derived from an authentic sequence of a target-associated antigen. Further, the substrate polypeptide in which the epitope is embedded can be conformed to a preferred immune proteasome cleavage site flanking sequence.

It is described herein that the substrate polypeptide can include an array of additional epitopes. Members of the array can be arranged head-to-tail, for example. The array can include more than one housekeeping epitope. The more than one housekeeping epitope can include copies of the same epitope. The array can include a housekeeping and an immune epitope, or alternating housekeeping and immune epitopes, for example. Also, the array can include a housekeeping epitope positioned between two immune epitopes in an epitope battery. The array can include multiple epitope batteries, so that there are two immune epitopes between each housekeeping epitope in the interior of the array. Optionally, at least one of the epitopes can be truncated distally to its junction with an adjacent epitope. The truncated epitopes can be immune epitopes, for example. The truncated epitopes can have lengths selected from the group including, but not limited to, 9, 8, 7, 6, 5, 4 amino acids, and the like.

It is described herein that, the substrate polypeptide can include an array of epitopes and epitope clusters. Members of the array can be arranged head-to-tail, for example.

It is described herein that, the proteasome can be an immune proteasome.

Also described are vectors including a housekeeping epitope expression cassette. The housekeeping epitope(s) can be derived from a target-associated antigen, and the housekeeping epitope can be liberatable, that is capable of liberation, from a translation product of the cassette by immunoproteasome processing.

It is described herein that the expression cassette can encode an array of two or more epitopes or at least one epitope and at least one epitope cluster. The members of the array can be arranged head-to-tail, for example. Also, the members of the array can be arranged head-to-tail separated by spacing sequences, for example. Further, the array can include a plurality of housekeeping epitopes. The plurality of housekeeping epitopes can include more than one copy of the same epitope or single copies of distinct epitopes, for example. The array can include at least one housekeeping epitope and at least one immune epitope. Also, the array can include alternating housekeeping and immune epitopes. Further, the array includes a housekeeping epitope sandwiched between two immune epitopes so that there are two immune epitopes between each housekeeping epitope in the interior of the array. The immune epitopes can be truncated distally to their junction with the adjacent housekeeping epitope.

It is described herein that the expression cassette further encodes an authentic protein sequence, or segment thereof, including at least one immune epitope. Optionally, the segment can include at least one epitope cluster. The housekeeping epitope expression cassette and the authentic sequence including at least one immune epitope can be encoded in a single reading frame or transcribed as a single mRNA species, for example. Also, the housekeeping epitope expression cassette and the authentic sequence including at least one immune epitope may not be transcribed as a single mRNA species.

In yet another aspect, the vector can include a DNA molecule or an RNA molecule. The vector can encode, SEQ ID NO. 4. Also, the vector can include SEQ ID NO. 9.

It is disclosed herein that the target-associated antigen can be an antigen derived from or associated with a tumor or an intracellular parasite, and the intracellular parasite can be, for example, a virus, a bacterium, a protozoan, or the like.

Also described herein are vectors including a housekeeping epitope identified according to any of the methods disclosed herein, claimed or otherwise. For example, also described is a vector encoding a substrate polypeptide that includes a housekeeping epitope by any of the methods described herein.

It is disclosed herein that the housekeeping epitope can be liberated from the cassette translation product by immune proteasome processing

Also described are methods of activating a T cell. The methods can include, for example, the steps of contacting a vector including a housekeeping epitope expression cassette with an APC. The housekeeping epitope can be derived from a target-associated antigen, for example, and the housekeeping epitope can be liberatable from a translation product of the cassette by immunoproteasome processing. The methods can further include contacting the APC with a T cell. The contacting of the vector with the APC can occur *in vitro* or *in vivo.*

Also disclosed herein is a substrate polypeptide including a housekeeping epitope wherein the housekeeping epitope can be liberated by immunoproteasome processing in a pAPC.

Also described is a method of activating a T cell comprising contacting a substrate polypeptide including a housekeeping epitope with an APC wherein the housekeeping epitope can be liberated by immunoproteasome processing and contacting the APC with a T cell.

### Brief Description of the Drawings

Figure 1. An illustrative drawing depicting pMA2M.

Figure 2. Assay results showing the % of specific lysis of ELAGIGILTV pulsed and unpulsed T2 target cells by mock immunized CTL.

Figure 3. Assay results showing the % of specific lysis of ELAGIGILTV pulsed and unpulsed T2 target cells by pVAXM3 immunized CTL.

Figure 4. Assay results showing the % of specific lysis of ELAGIGILTV pulsed and unpulsed T2 target cells by pVAXM2 immunized CTL.

Figure 5. Assay results showing the % of specific lysis of ELAGIGILTV pulsed and unpulsed T2 target cells by pVAXM1 immunized CTL.

Figure 6. Illustrates a sequence of SEQ ID NO. 22 from which the NY-ESO-I₁₅₇₋₁₆₅ epitope is liberated by immunoproteasomal processing.

Figure 7. Shows the differential processing by immunoproteasome and housekeeping protcasome of the SLLMWITQC epitope (SEQ ID NO. 12) in its native context where the cleavage following the C residue is more efficiently produced by housekeeping than immunoproteasome.

Figure 8. 8A: Shows the results of the human immunoproteasome digest of SEQ ID NO. 31. 8B: Shows the comparative results of mouse versus human immunoproteasome digestion of SEQ ID NO. 31.

Figure 9. Shows the differential processing of SSX-2₃₁₋₆₈ by housekeeping and immunoproteasome.

### Detailed Description of the Preferred Embodiment

### Definitions

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell costimulatory molecules and is able to induce a T cell response. Well characterized pAPCs include dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNOPROTEASOME - a proteasome normally active in pAPCs; the immunoproteasome is also active in some peripheral cells in infected tissues or following exposure to interferon.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptides presented on the surface of cells, the peptides being non-covalently bound to the binding cleft of class I MHC, such that they can interact with T cell receptors (TCR). Epitopes presented by class I MHC may be in immature or mature form. "Mature" refers to an MHC epitope in distinction to any precursor ("immature") that may include or consist essentially of a housekeeping epitope, but also includes other sequences in a primary translation product that are removed by processing, including without limitation, alone or in any combination, proteasomal digestion, N-terminal trimming, or the action of exogenous enzymatic activities. Thus, a mature epitope may be provided embedded in a somewhat longer polypeptide, the immunological potential of which is due, at least in part, to the embedded epitope; or in its ultimate form that can bind in the MHC binding cleft to be recognized by TCR, respectively.

MHC EPITOPE - a polypeptide having a known or predicted binding affinity for a mammalian class I or class II major histocompatibility complex (MHC) molecule.

HOUSEKEEPING EPITOPE - In a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions. Exemplary housekeeping epitopes are provided in U.S. Application No. 10/117,937, filed on April 4, 2002; and U.S. Provisional Application Nos. 60/282,211, filed on April 6, 2001; 60/337,017, filed on November 7, 2001; 60/363210 filed 3/7/02; and 60/409,123, filed on September 5, 2002; all of which are entitled EPITOME SEQUENCES.

IMMUNE EPITOPE - In a preferred embodiment, an immune epitope is defined as a polypeptide fragment that is an MHC epitope, and that is displayed on a cell in which immunoproteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - a cell to be targeted by the vaccines and methods of the invention. Examples of target cells according to this definition include but are not necessarily limited to a neoplastic cell and a cell harboring an intracellular parasite, such as, for example, a virus, a bacterium, or a protozoan. Target cells can also include cells that are targeted by CTL as a part of assays to determine or confirm proper epitope liberation and processing by a cell expressing immunoproteasome, to determine T cell specificity or immunogenicity for a desired epitope.. Such cells may be transformed to express the substrate or liberation sequence, or the cells can simply be pulsed with peptide/epitope.

TARGET-ASSOCIATED ANTIGEN (TAA) - a protein or polypeptide present in a target cell.

TUMOR-ASSOCIATED ANTIGENS (TuAA) - a TAA, wherein the target cell is a neoplastic cell.

HLA EPITOPE - a polypeptide having a known or predicted binding affinity for a human class I or class II HLA complex molecule.

ANTIBODY - a natural immunoglobulin (Ig), poly- or monoclonal, or any molecule composed in whole or in part of an Ig binding domain, whether derived biochemically or by use of recombinant DNA. Examples include *inter alia*, F(ab), single chain Fv, and Ig variable region-phage coat protein fusions.

ENCODE - an open-ended term such that a nucleic acid encoding a particular amino acid sequence can consist of codons specifying that (poly)peptide, but can also comprise additional sequences either translatable, or for the control of transcription, translation, or replication, or to facilitate manipulation of some host nucleic acid construct.

SUBSTANTIAL SIMILARITY - this term is used to refer to sequences that differ from a reference sequence in an inconsequential way as judged by examination of the sequence. Nucleic acid sequences encoding the same amino acid sequence are substantially similar despite differences in degenerate positions or modest differences in length or composition of any non-coding regions. Amino acid sequences differing only by conservative substitution or minor length variations are substantially similar. Additionally, amino acid sequences comprising housekeeping epitopes that differ in the number of N-terminal flanking residues, or immune epitopes and epitope clusters that differ in the number of flanking residues at either terminus, are substantially similar. Nucleic acids that encode substantially similar amino acid sequences are themselves also substantially similar.

FUNCTIONAL SIMILARITY - this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino acid substitutions (and thus do not meet the substantial similarity definition). Pairs of antibodies, or TCRs, that recognize the same epitope can be functionally similar to each other despite whatever structural differences exist. In testing for functional similarity of immunogenicity one would generally immunize with the "altered" antigen and test the ability of the elicited response (Ab, CTL, cytokine production, etc.) to recognize the target antigen. Accordingly, two sequences may be designed to differ in certain respects while retaining the same function. Such designed sequence variants are among the embodiments of the present invention.

EXPRESSION CASSETTE - a polynucleotide sequence encoding a polypeptide, operably linked to a promoter and other transcription and translation control elements, including but not limited to enhancers, termination codons, internal ribosome entry sites, and polyadenylation sites. The cassette can also include sequences that facilitate moving it from one host molecule to another.

EMBEDDED EPITOPE - an epitope contained within a longer polypeptide, also can include an epitope in which either the N- terminus or the C-terminus is embedded such that the epitope is not in an interior position.

MATURE EPITOPE - a peptide with no additional sequence beyond that present when the epitope is bound in the MHC peptide-binding cleft.

EPITOPE CLUSTER - a polypeptide, or a nucleic acid sequence encoding it, that is a segment of a native protein sequence comprising two or more known or predicted epitopes with binding affinity for a shared MHC restriction element, wherein the density of epitopes within the cluster is greater than the density of all known or predicted epitopes with binding affinity for the shared MHC restriction element within the complete protein sequence, and as disclosed in U.S. Patent Application No. 09/561,571 entitled EPITOPE CLUSTERS.

Substrate or liberation sequence- a designed or engincered sequence comprising or encoding a housekeeping epitope (according to the first of the definitions offered above) embedded in a larger sequence that provides a context allowing the housekeeping epitope to be liberated by immunoproteasomal processing, directly or in combination with N-terminal trimming or other processes.

Degradation of cytosolic proteins takes place via the ubiquitin-dependent multi-catalytic multi-subunit protease system known as the proteasome. The proteasome degrades cytosolic proteins generating fragments that can then be translocated from the cytosol into the endoplasmic reticulum (ER) for loading onto class I MHC. Such protein fragments shall be referred to as class I peptides. The peptide loaded MHC are subsequently transported to the cell surface where they can be detected by CTL.

The multi-catalytic activity of the proteasome is the result of its multi-subunit structure. Subunits are expressed from different genes and assembled post-translationally into the proteasome complex. A key feature of the proteasome is its bimodal activity, which enables it to exert its protease, or cleavage function, with two discrete kinds of cleavage patterns. This bimodal action of the proteasome is extremely fundamental to understanding how CTL are targeted to recognize peripheral cells in the body and how this targeting requires synchronization between the immune system and the targeted cells.

The housekeeping proteasome is constitutively active in all peripheral cells and tissues of the body. The first mode of operation for the housekeeping proteasome is to degrade cellular protein, recycling it into amino acids. Proteasome function is therefore a necessary activity for cell life. As a corollary to its housekeeping protease activity, however, class I peptides generated by the housekeeping proteasome are presented on all of the peripheral cells of the body.

The proteasome's second mode of function is highly exclusive and occurs specifically in pAPCs or as a consequence of a cellular response to interferons (IFNs). In its second mode of activity the proteasome incorporates unique subunits, which replace the catalytic subunits of the constitutive housekeeping proteasome. This "modified" proteasome has been called the immunoproteasome, owing to its expression in pAPC and as a consequence of induction by IFN in body cells.

APC define the repertoire of CTL that recirculate through the body and are potentially active as killer cells. CTL are activated by interacting with class I peptide presented on the surface of a pAPC. Activated CTL are induced to proliferate and caused to recirculate through the body in search of diseased cells. This is why the CTL response in the body is defined specifically by the class I peptides produced by the pAPC. It is important to remember that pAPCs express the immunoproteasome, and that as a consequence of the bimodal activity of the proteasome, the cleavage pattern of proteins (and the resultant class I peptides produced) are different from those in peripheral body cells which express housekeeping proteasome. The differential proteasome activity in pAPC and peripheral body cells, therefore, is important to consider during natural infection and with therapeutic CTL vaccination strategies.

All cells of the body are capable of producing IFN in the event that they are infected by a pathogen such as a virus. IFN production in turn results in the expression of the immunoproteasome in the infected cell. Viral antigens are thereby processed by the immunoproteasome of the infected cell and the consequent peptides are displayed with class I MHC on the cell surface. At the same time, pAPC are sequestering virus antigens and are processing class I peptides with their immunoproteasome activity, which is normal for the pAPC cell type. The CTL response in the body is being stimulated specifically by the class I peptides produced by the pAPC. Fortunately, the infected cell is also producing class I peptides from the immunoproteasome, rather than the normal housekeeping proteasome. Thus, virus-related class I peptides are being produced that enable detection by the ensuing CTL response. The CTL immune response is induced by pAPC, which normally produce different class I peptides compared to peripheral body cells, owing to different proteasome activity. Therefore, during infection there is epitope synchronization between the infected cell and the immune system.

This is not the case with tumors and chronic viruses, which block the interferon system. For tumors there is no infection in the tumor cell to induce the immunoproteasome expression, and chronic virus infection either directly or indirectly blocks immunoproteasome expression. In both cases the diseased cell maintains its display of class I peptides derived from housekeeping proteasome activity and avoids effective surveillance by CTL.

In the case of therapeutic vaccination to eradicate tumors or chronic infections, the bimodal function of the proteasome and its differential activity in APC and peripheral cells of the body is significant. Upon vaccination with protein antigen, and before a CTL response can occur, the antigen must be acquired and processed into peptides that are subsequently presented on class I MHC on the pAPC surface. The activated CTL recirculate in search of cells with similar class I peptide on the surface. Cells with this peptide will be subjected to destruction by the cytolytic activity of the CTL. If the targeted diseased cell does not express the immunoproteasome, which is present in the pAPC, then the epitopes are not synchronized and CTL fail to find the desired peptide target on the surface of the diseased cell.

Preferably, therapeutic vaccine design takes into account the class I peptide that is actually present on the target tissue. That is, effective antigens used to stimulate CTL to attack diseased tissue are those that are naturally processed and presented on the surface of the diseased tissue. For tumors and chronic infection this generally means that the CTL epitopes are those that have been processed by the housekeeping proteasome. In order to generate an effective therapeutic vaccine, CTL epitopes are identified based on the knowledge that such epitopes are, in fact, produced by the housekeeping proteasome system. Once identified, these epitopes, embodied as peptides, can be used to successfully immunize or induce therapeutic CTL responses against housekeeping proteasome expressing target cells in the host.

However, in the case of DNA vaccines, there can be an additional consideration. The immunization with DNA requires that APCs take up the DNA and express the encoded proteins or peptides. It is possible to encode a discrete class I peptide on the DNA. By immunizing with this construct, APCs can be caused to express a housekeeping epitope, which is then displayed on class I MHC on the surface of the cell for stimulating an appropriate CTL response. Constructs for generation of proper termini of housekeeping epitopes have been described in U.S. Patent application No. 09/561,572 entitled EXPRESSION VECTORS ENCODING EPITOPES OF TARGET-ASSOCIATED ANTIGENS, filed on April 28, 2000.

Disclosed herein are expression cassettes that encode one or more embedded housekeeping epitopes, and methods for designing and testing such expression cassettes. The expression cassettes and constructs can encode epitopes, including housekeeping epitopes, derived from antigens that are associated with targets. Housekeeping epitopes can be liberated from the translation product(s) of the cassettes. For example, it is described that the housekeeping epitope(s) can be flanked by arbitrary sequences or by sequences incorporating residues known to be favored in immunoproteasome cleavage sites. It is described herein that multiple epitopes can be arrayed head-to-tail. It is disclosed herein that these arrays can be made up entirely of housekeeping epitopes. Likewise, the arrays can include alternating housekeeping and immune epitopes. Alternatively, the arrays can include housekeeping epitopes flanked by immune epitopes, whether complete or distally truncated. It is described herein that each housekeeping epitope can be flanked on either side by an immune epitope, such that an array of such arrangements has two immune epitopes between each housekeeping epitope. Further, the arrays can be of any other similar arrangement. There is no restriction on placing a housekeeping epitope at the terminal positions of the array. The vectors can additionally contain authentic protein coding sequences or segments thereof containing epitope clusters as a source of immune epitopes.

Several disclosures make reference to polyepitopes or string-of-bead arrays. *See,* for example, WO0119408A1, March 22, 2001; WO9955730A2, November 4, 1999; W00040261A2, July 13, 2000; WO9603144A1, February 8, 1996; EP1181314A1, February 27, 2002; WO0123577A3, April 5; US6074817, June 13, 2000; US5965381, October 12, 1999; WO9741440A1, November 6, 1997; US6130066, October 10, 2000; US6004777, December 21, 1999; US5990091, November 23, 1999; WO9840501A1, September 17, 1998; WO9840500A1, September 17, 1998; WO0118035A2, March 15, 2001; WO02068654A2, September 6, 2002; WO0189281A2, November 29, 2001; WO0158478A, August 16, 2001; EP1118860A1, July 25, 2001; WO0111040A1, February 15, 2001; WO0073438A1, December 7, 2000; WO0071158A1, November 30, 2000; WO0066727A1, November 9, 2000; WO0052451A1, September 8, 2000; WO0052157A1, September 8, 2000; WO0029008A2, May 25,2000; WO0006723A1, February 10, 2000. Additional disclosures, include Palmowski MJ, et al - J Immunol 2002;168(9):4391-8; Fang ZY, et al - Virology 2001;291(2):272-84; Firat H, et al - J Gene Med 2002;4(1):38-45; Smith SG, et al - Clin Cancer Res 2001;7(12):4253-61; Vonderheide RH, et al - Clin Cancer Res 2001; 7(11):3343-8; Firat H, et al - Eur J Immunol 2001;31(10):3064-74; Le TT, et al - Vaccine 2001;19(32):4669-75; Fayolle C, et al - J Virol 2001;75(16):7330-8; Smith SG - Curr Opin Mol Ther 1999;1(1):10-5; Firat H, et al - Eur J Immunol 1999;29(10):3112-21; Mateo L, et al - J Immunol 1999;163(7):4058-63; Heemskerk MH, et al - Cell Immunol 1999;195(1):10-7; Woodberry T, et al - J Virol 1999;73(7):5320-5; Hanke T, et al - Vaccine 1998;16(4):426-35; Thomson SA, et al - J Immunol 1998;160(4):1717-23; Toes RE, et al - Proc Natl Acad Sci USA 1997;94(26):14660-5; Thomson SA, et al - J Immunol 1996;157(2):822-6; Thomson SA, et al - Proc Natl Acad Sci USA 1995;92(13):5845-9; Street MD, et al - Immunology 2002;106(4):526-36; Hirano K, et al - Histochem Cell Biol 2002;117(1):41-53; Ward SM, et al - Virus Genes 2001;23(1):97-104; Liu WJ, et al - Virology 2000;273(2):374-82; Gariglio P, et al - Arch Med Res 1998;29(4):279-84; Suhrbier A - Immunol Cell Biol 1997;75(4):402-8; Fomsgaard A, et al - Vaccine 1999;18(7-8):681-91; An LL, et al - J Virol 1997;71(3):2292-302; Whitton JL, et al - J Virol 1993;67(1):348-52; Ripalti A, et al - J Clin Microbiol 1994;32(2):358-63; and Gilbert, S.C., et al., Nat. Biotech. 15:1280-1284, 1997.

One important feature that the disclosures in the preceding paragraph all share is their lack of appreciation for the desirability of regenerating housekeeping epitopes when the construct is expressed in a pAPC. This understanding was not apparent until the present invention. Embodiments of the invention include sequences, that when processed by an immune proteasome, liberate or generate a housekeeping epitope. Accordingly, while the preceding references contain disclosures relating to polyepitope arrays, none is enabling of the technology necessary to provide or select a polyepitope capable of liberating a housekeeping epitope by action of an immunoproteasome in a pAPC. In contrast, the present disclosure is based upon a recognition of the desirability of achieving this result. Accordingly, also described herein is any nucleic acid construct that encodes a polypeptide containing at least one housekeeping epitope provided in a context that promotes its generation via immunoproteasomal activity, whether the housekeeping epitope is embedded in a string-of-beads array or some other arrangement. Also described herein are uses of one or more of the nucleic acid constructs or their products that are specifically disclosed in any one or more of the above-listed references. Such uses include, for example, screening a polyepitope for proper liberation context of a housekeeping epitope and/or an immune epitope, designing an effective immunogen capable of causing presentation of a housekeeping epitope and/or an immune epitope on a pAPC, immunizing a patient, and the like. Also disclosed is the use of only a subset of such nucleic acid constructs or a single such construct, while specifically excluding one or more other such constructs, for any of the purposes disclosed herein.

Also described herein are methods, uses, therapies, methods, uses, therapies, and compositions directed to various types of targets. Such targets can include, for example, neoplastic cells such as those listed below, for example; and cells infected with any virus, bacterium, protozoan, fungus, or other agents, examples of which are listed below in Tables 1-5, or which are disclosed in any of the references listed above. Also described is the use of only a subset of such neoplastic cells and infected cells listed below, in Tables 1-5, or in any of the references disclosed herein, or a single one of the neoplastic cells or infected cells, while specifically excluding one or more other such neoplastic cells or infected cells, for any of the purposes disclosed herein. The following are examples of neoplastic cells that can be targeted: human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non Hodgkin's disease), multiple myeloma, Waldenstrom's macroglobulinemia, heavy chain disease, hepatocellular cancer, brain cancer, stomach cancer, liver cancer, and the like. Examples of infectious agents that infect the target cells can include the following: adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, human T cell leukemia virus II, *Chlamydia, Listeria, Salmonella, Legionella, Brucella, Coxiella, Rickettsia, Mycobacterium, Leishmania, Trypanasoma, Toxoplasma, Plasmodium,* and the like. Exemplary infectious agents and neoplastic cells are also included in Tables 1-5 below.

Furthermore the targets can include neoplastic cells described in or cells infected by agents that are described in any of the following references: Jäger, E. et al., "Granulocyte-macrophage-colony-stimulating factor enhances immune responses to melanoma-associated peptides in vivo," Int. J Cancer, 67:54-62 (1996); Kündig, T.M., Althage, A., Hengartner, H. & Zinkernagel, R.M., "A skin test to assess CD8+ cytotoxic T cell activity," Proc. Natl. Acad Sci. USA, 89:7757-76 (1992); Bachmann, M.F. & Kundig, T.M., "In vitro vs. in vivo assays for the assessment of T- and B-cell function," Curr. Opin. Immunol., 6:320-326 (1994); Kundig et al., "On the role of antigen in maintaining cytotoxic T cell memory," Proceedings of the National Academy of Sciences of the United States of America, 93:9716-23 (1996); Steinmann, R.M., "The dendritic cells system and its role in immunogenicity," Annual Review of Immunology 9:271-96 (1991); Inaba, K. et al., "Identification of proliferating dendritic cell precursors in mouse blood," Journal of Experimental Medicine, 175:1157-67 (1992); Young, J.W. & Inaba, K., "Dendritic cells as adjuvants for class I major histocompatibility complex-restricted anti-tumor immunity," Journal of Experimental Medicine, 183:7-11 (1996); Kuby, Janis, Immunology, Second Edition, Chapter 15, W.H. Freeman and Company (1991); Austenst, E., Stahl, T., and de Gruyter, Walter, Insulin Pump Therapy, Chapter 3, Berlin, New York (1990); Remington, The Science and Practice of Pharmacy, Nineteenth Edition, Chapters 86-88; Cleland, Jeffery L. and Langer, Robert (Editor), "Formulation and delivery of proteins and peptides," American Chemical Society (ACS Symposium Series, No. 567) (1994); Dickinson, Becton, which is fixed using Tegadenn transparent dressing Tegaderm™ 1624, 3M, St. Paul, MN 55144, USA; Santus, Giancarlo and Baker, Richard, "Osmotic drug delivery: A review of the patent literature," Journal of Controlled Release, 35:1-21 (1995); Rammensee, U.S. Patent No. 5,747,269, issued May 05, 1998; Magruder, U.S. Patent No. 5,059,423, issued October 22, 1991; Sandbrook, U.S. Patent No. 4,552,651, issued November 25, 1985; Eckenhoff et al., U.S. Patent No. 3,987,790, issued October 26, 1976; Theeuwes, U.S. Patent No. 4,455,145, issued June 19, 1984; Roth et al. U.S. Patent No. 4,929,233, issued May 29 1990; van der Bruggen et al., U.S. Patent No. 5,554,506, issued September 10, 1996; Pfreundschuh, U.S. Patent No. 5,698,396, issued December 16, 1997; Magruder, U.S. Patent No. 5,110,596, issued May 5, 1992; Eckenhoff, U.S. Patent No. 4,619,652, issued October 28, 1986; Higuchi et al., U.S. Patent No. 3,995,631, issued December 07, 1976; Maruyama, U.S. Patent No. 5,017,381, issued May 21, 1991; Eckenhoff, U.S. Patent No. 4,963,141, issued October 16, 1990; van der Bruggen et al., U.S. Patent No. 5,558,995, issued September 24, 1996; Stolzenberg et al. U.S. Patent No. 3,604,417, issued September 14, 1971; Wong et al., U.S. Patent No. 5,110,597, issued May 05, 1992; Eckenhoff, U.S. Patent No. 4,753,651, issued June 28, 1988; Theeuwes, U.S. Patent No. 4,203,440, issued May 20, 1980; Wong et al. U.S. Patent No. 5,023,088, issued June 11, 1991; Wong et al., U.S. Patent No. 4,976,966, issued December 11, 1990; Van den Eynde et al., U.S. Patent No. 5,648,226, issued July 15, 1997; Baker et al., U.S. Patent No. 4,838,862, issued June 13, 1989; Magruder, U.S. Patent No. 5,135,523, issued August 04, 1992; Higuchi et al., U.S. Patent No. 3,732,865, issued May 15, 1975, ; Theeuwes, U.S. Patent No. 4,286,067, issued August, 25 1981; Theeuwes et al., U.S. Patent No. 5,030,216, issued July 09, 1991; Boon et al., U.S. Patent No. 5,405,940, issued April 11, 1995; Faste, U.S. Patent No. 4,898,582, issued February 06, 1990; Eckenhoff, U.S. Patent No. 5,137,727, issued August 11, 1992; Higuchi et al., U.S. Patent No. 3,760,804, issued September 25, 1973; Eckenhoff et al., U.S. Patent No. 4,300,558, issued November 12, 1981; Magruder et al., U.S. Patent No. 5,034,229, issued July 23, 1991; Boon et al., U.S. Patent No. 5,487,974, issued January 30, 1996; Kam et al., U.S. Patent No. 5,135,498, issued August 04, 1992; Magruder et al., U.S. Patent No. 5,174,999, issued December 29, 1992; Higuchi, U.S. Patent No. 3,760,805, September 25, 1973; Michaels, U.S. Patent No. 4,304,232, issued December 08, 1981; Magruder et al., U.S. Patent No. 5,037,420, issued October 15, 1991; Wolfel et al., U.S. Patent No. 5,530,096, issued June 25, 1996; Athadye et al., U.S. Patent No. 5,169,390, issued December 08, 1992; Balaban et al., U.S. Patent No. 5,209,746, issued May 11, 1993; Higuchi, U.S. Patent No. 3,929,132, issued December 30, 1975; Michaels, U.S. Patent No. 4,340,054, issued July 20, 1982; Magruder et al., U.S. Patent No. 5,057,318, issued October 15, 1991; Wolfel et al., U.S. Patent No. 5,519,117, issued May 21, 1996; Athadye et al., U.S. Patent No. 5,257,987, issued November 02, 1993; Linkwitz et al., U.S. Patent No. 5,221,278, issued June 22, 1993; Nakano et al., U.S. Patent No. 3,995,632, issued December 07, 1976; Michaels, U.S. Patent No. 4,367,741, issued January 11, 1983; Eckenhoff, U.S. Patent No. 4,865,598, issued September 12, 1989; Lethe et al., U.S. Patent No. 5,774,316, issued April 28, 1998; Eckenhoff, U.S. Patent No. 4,340,048, issued July 20, 1982; Wong, U.S. Patent No. 5,223,265, issued June 29, 1993; Higuchi et al., U.S. Patent No. 4,034,756, issued July 12, 1977; Michaels, U.S. Patent No. 4,450,198, issued May 22, 1984; Eckenhoff et al., U.S. Patent No. 4,865,845, issued September 12, 1989; Melief et. al., U.S. Patent No. 5,554,724, issued September 10, 1996; Eckenhoff et al., U.S. Patent No. 4,474,575, issued October 02, 1984; Theeuwes, U.S. Patent No. 3,760,984, issued September 25, 1983; Eckenhoff, U.S. Patent No. 4,350,271, issued September 21, 1982; Eckenhoff et al., U.S. Patent No. 4,855,141, issued August 08, 1989; Zingerman, U.S. Patent No. 4,872,873, issued October 10, 1989; Townsend et al., U.S. Patent No. 5,585,461, issued December 17, 1996; Carulli, J.P. et al., J. Cellular Biochem Suppl., 30/31:286-96 (1998); Türeci, Ö., Sahin, U., and Pfreundschuh, M., "Serological analysis of human tumor antigens: molecular definition and implications," Molecular Medicine Today, 3:342 (1997); Rammensee et al., MHC Ligands and Peptide Motifs, Landes Bioscience Austin, TX, 224-27, (1997); Parker et al., "Scheme for ranking potential HLA-A2 binding peptides based on independent binding of individual peptide side-chains," J. Immunol. 152:163-175; Kido & Ohshita, Anal. Biochem., 230:41-47 (1995); Yamada et al., J. Biochem. (Tokyo), 95:1155-60 (1984); Kawashima et al., Kidney Int., 54:275-8 (1998); Nakabayshi & Ikezawa, Biochem. Int. 16:1119-25 (1988); Kanaseki & Ohkuma, J. Biochem. (Tokyo), 110:541-7 (1991); Wattiaux et al., J. Cell Biol., 78:349-68 (1978); Lisman et al., Biochem. J., 178:79-87 (1979); Dean, B., Arch. Biochem. Biophys., 227:154-63 (1983); Overdijk et al., Adv. Exp. Med. Biol., 101:601-10 (1978); Stromhaug et al., Biochem. J., 335:217-24 (1998); Escola et al., J. Biol. Chem., 271:27360-05 (1996); Hammond et al., Am. J. Physiol., 267:F516-27 (1994); Williams & Smith, Arch. Biochem. Biophys., 305:298-306 (1993); Marsh, M., Methods Cell Biol., 31:319-34 (1989); Schmid & Mellman, Prog. Clin. Biol. Res., 270:35-49 (1988); Falk, K. et al., Nature, 351:290, (1991); Ausubel et al., Short Protocols in Molecular Biology, Third Edition, Unit 11.2 (1997); hypertext transfer protocol address 134.2.96.221/scripts/hlaserver.dll/EpPredict.htm; Levy, Morel, S. et al., Immunity 12:107-117 (2000); Seipelt et al., Virus Research, 62:159-68, 1999; Storkus et al., U.S. Patent No. 5,989,565, issued November 23, 1999; Morton, U.S. Patent No. 5,993,828, issued November 30, 1999; Virus Research 62:159-168, (1999); Simard et al., U.S. Patent Application No. 10/026066, filed December 07, 2001; Simard et al., U.S. Patent Application No. 09/561571, filed April 28, 2000; Simard et al., U.S. Patent Application No. 09/561572, filed April 28, 2000; Miura et al., WO 99/01283, January 14, 1999; Simard et al., U.S. Patent Application No. 09/561074, filed April 28, 2000; Simard et al., U.S. Patent Application No. 10/225568, filed August 20, 2002; Simard et al., U.S. Patent Application No. 10/005905, filed November 07, 2001; Simard et al., U.S. Patent Application No. 09/561074, filed April 28, 2000.

Also disclosed herein are methods, uses, therapies, and compositions relating to a particular antigen, whether the antigen is derived from, for example, a target cell or an infective agent, such as those mentioned above. Some preferred embodiments employ the antigens listed herein, in Tables 1-5, or in the list below, alone, as subsets, or in any combination. For example, some embodiments exclude use of one or more of those antigens. Other embodiments may exclude any combination or all of those antigens. Several examples of such antigens include MelanA (MART-I), gp100 (Pmel 17), tyrosinase, TPP-1, TRP-2, MAGE-1, MAGE-3, BAGE, GAGE-1, GAGE-2, CEA, RAGE, NY-ESO, SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CAM 17.1, NuMa, K-ras, β-Catenin, CDK4, Mum-1, p16, as well as any of those set forth in the above mentioned references. Other antigens are included in Tables 1-4 below.

Disclosed herein are methods, uses, compositions, and therapies relating to epitopes, including, for example those epitopes listed in Tables 1-5. These epitopes can be useful to flank housekeeping epitopes in screening vectors, for example. Some embodiments include one or more epitopes from Tables 1-5, while other embodiments specifically exclude one or more of such epitopes or combinations thereof.

**Table I**

| Virus | Protein | AA Position | T cell epitope MHC ligand (Antigen) | MHC molecule |
|---|---|---|---|---|
| Adenovirus 3 | E3 9Kd | 30-38 | LIVIGILIL | HLA-A*0201 |
| | | | (SEQ. ID NO.:44) | |
| Adenovirus 5 | EIA | 234-243 | SGPSNTPPEI | H2-Db |
| | | | (SEQ. ID NO.:45) | |
| Adenovirus 5 | EIB | 192-200 | VNIRNCCYI | H2-Db |
| | | | (SEQ. ID NO.:46) | |
| Adenovirus 5 | EIA | 234-243 | SGPSNIPPEI (T>I) | H2-Db |
| | | | (SEQ. ID NO.:47) | |
| CSFV | NS polyprotein | 2276-2284 | ENALLVALF | SLA,haplotype d/d |
| | | | (SEQ. ID NO.:48) | |
| Dengue virus 4 | NS3 | 500-508 | TPEGIIPTL | HLA-B*3501 |
| | | | (SEQ. ID NO.:49) | |
| EBV | LMP-2 | 426-434 | CLGGLLTMV | HLA-A*0201 |
| | | | (SEQ. ID NO.:50) | |
| EBV | EBNA-1 | 480-484 | NIAEGLRAL | HLA-A*0201 |
| | | | (SEQ. ID NO.:51) | |
| EBV | EBNA-1 | 519-527 | NLRRGTALA | HLA-A*0201 |
| | | | (SEQ. ID NO.:52) | |
| EBV | EBNA-1 | 525-533 | ALAIPQCRL | HLA-A*0201 |
| | | | (SEQ. ID NO.:53) | |
| EBV | EBNA-1 | 575-582 | VLKDAIKDL | HLA-A*0201 |
| | | | (SEQ. ID NO.:54) | |
| EBV | EBNA-1 | 562-570 | FMVFLQTHI | HLA-A*0201 |
| | | | (SEQ. ID NO.:55) | |
| EBV | EBNA-2 | 15-23 | HLIVDTDSL | HLA-A*0201 |
| | | | (SEQ. ID N0.:56) | |
| EBV | EBNA-2 | 22-30 | SLGNPSLSV | HLA-A*0201 |
| | | | (SEQ. ID NO.:57) | |
| EBV | EBNA-2 | 126-134 | PLASAMRML | HLA-A*0201 |
| | | | (SEQ. ID NO.:58) | |
| EBV | EBNA-2 | 132-140 | RMLWMANYI | HLA-A*0201 |
| | | | (SEQ. ID NO.:59) | |
| EBV | EBNA-2 | 133-141 | MLWMANYIV | HL.A-A*0201 |
| | | | (SEQ. ID NO.:60) | |
| EBV | EBNA-2 | 151-159 | ILPQGPQTA | HLA-A*0201 |
| | | | (SEQ. ID NO.:61) | |
| EBV | EBNA-2 | 171-179 | PLRPTAPTI | HLA-A*0201 |
| | | | (SEQ. ID NO.:62) | |
| EBV | EBNA-2 | 205-213 | PLPPATLTV | HLA-A*0201 |
| | | | (SEQ. ID NO.:63) | |
| EBV | EBNA-2 | 246-254 | RMHLPVLHV | HLA-A*0201 |
| | | | (SEQ. ID N0.:64) | |
| EBV | EBNA-2 | 287-295 | PMPLPPSQL | HLA-A*0201 |
| | | | (SEQ. ID NO.:65) | |
| EBV | EBNA-2 | 294-302 | QLPPPAAPA | HLA-A*0201 |
| | | | (SEQ. ID NO.:66) | |
| EBV | EBNA-2 | 381-389 | SMPELSPVL | HLA-A*0201 |
| | | | (SEQ. ID NO.:67) | |
| EBV | EBNA-2 | 453-461 | DLDESWDYI | HLA-A*0201 |
| | | | (SEQ. ID NO.:68) | |
| EBV | BZLF1 | 43-51 | PLPCVLWPV | HLA-A*0201 |
| | | | (SEQ. ID NO.:69) | |
| EBV | BZLF1 | 167-175 | SLEECDSEL | HLA-A*0201 |
| | | | (SEQ. ID NO.:70) | |
| EBV | BZLF1 | 176-184 | EIKRYKNRV | HLA-A*0201 |
| | | | (SEQ. ID NO.:71) | |
| EBV | BZLF1 | 195-203 | QLLQHYREV | HLA-A*0201 |
| | | | (SEQ. ID NO.:72) | |
| EBV | BZLF1 | 196-204 | LLQHYREVA | HLA-A*0201 |
| | | | (SEQ. ID NO.:73) | |
| EBV | BZLF1 | 217-225 | LLKQMCPSL | HLA-A*0201 |
| | | | (SEQ. ID NO.:74) | |
| EBV | BZLF1 | 229-237 | SIIPRTPDV | HLA-A*0201 |
| | | | (SEQ. ID NO.:75) | |
| EBV | EBNA-6 | 284-293 | LLDFVRFMGV | HLA-A*0201 |
| | | | (SEQ. ID NO.:76). | |
| EBV | EBNA-3 | 464-472 | SVRDRLARL | HLA-A*0203 |
| | | | (SEQ. ID NO.:77) | |
| EBV | EBNA-4 | 416-424 | IVTDFSVIK | HLA-A*1101 |
| | | | (SEQ. ID NO.:78) | |
| EBV | EBNA-4 | 399-408 | AVFDRKSDAK | HLA-A*0201 |
| | | | (SEQ. ID NO.:79) | |
| EBV | EBNA-3 | 246-253 | RYSIFFDY | HLA-A24 |
| | | | (SEQ. ID NO.:80) | |
| EBV | EBNA-6 | 881-889 | QPRAPIRPI | HLA-B7 |
| | | | (SEQ. ID NO.:81) | |
| EBV | EBNA-3 | 379-387 | RPPIFIRRI. | HLA-B7 |
| | | | (SEQ. ID NO.:82) | |
| EBV | EBNA-1 | 426-434 | EPDVPPGAI | HLA-B7 |
| | | | (SEQ. ID NO.:83) | |
| EBV | EBNA-1 | 228-236 | IPQCRLTPL | HLA-B7 |
| | | | (SEQ. ID NO.:84) | |
| EBV | EBNA-1 | 546-554 | GPGPQPGPL | HLA-B7 |
| | | | (SEQ. ID NO.:85) | |
| EBV | EBNA-1 | 550-558 | QPGPLRESI | HLA-B7 |
| | | | (SEQ. ID NO.:86) | |
| EBV | EBNA-1 | 72-80 | R.PQKRPSCI | HLA-B7 |
| | | | (SEQ. ID NO.:87) | |
| EBV | EBNA-2 | 224-232 | PPTPLLTVL | HLA-B7 |
| | | | (SEQ. ID NO.:88) | |
| EBV | EBNA-2 | 241-249 | TPSPPRMHL | HLA-B7 |
| | | | (SEQ. ID NO.:89) | |
| EBV | EBNA-2 | 244-252 | PPRMHLPVL | HLA-B7 |
| | | | (SEQ. ID NO.:90) | |
| EBV | EBNA-2 | 254-262 | VPDQSMHPL | HLA-B7 |
| | | | (SEQ. ID NO.:91) | |
| EBV | EBNA-2 | 446-454 | PPSIDPADL | HLA-B7 |
| | | | (SEQ. ID NO.:92) | |
| EBV | BZLFI | 44-52 | LPCVLWPVL | HLA-B7 |
| | | | (SEQ. ID NO.:93) | |
| EBV | BZLFI | 222-231 | CPSLDVDSII | HLA-B7 |
| | | | (SEQ. ID NO.:94) | |
| EBV | BZLFI | 234-242 | TPDVLHEDL | HLA.B7 |
| | | | (SEQ. ID NO.:95) | |
| EBV | EBNA-3 | 339-347 | FLRGRAYGL | HLA-B8 |
| | | | (SEQ. ID NO.:96) | |
| EBV | EBNA-3 | 26-34 | QAKWRLQTL | HLA-B8 |
| | | | (SEQ. ID NO.:97) | |
| EBV | EBNA-3 | 325-333 | AYPLHEQHG | HLA-B8 |
| | | | (SEQ. ID NO.:98) | |
| EBV | EBNA-3 | 158-166 | YIKSFVSDA | HLA-B8 |
| | | | (SEQ. ID NO.:99) | |
| EBV | LMP-2 | 236-244 | RRRWRRLTV | HLA-B*2704 |
| | | | (SEQ. ID NO.: 100) | |
| EBV | EBNA-6 | 258-266 | RRIYDLIEL | HLA-B*2705 |
| | | | (SEQ. ID NO.:101) | |
| EBV | EBNA-3 | 458-466 | YPLHEQHGM | HLA-B*3501 |
| | | | (SEQ. ID NO.:102) | |
| EBV | EBNA-3 | 458-466 | YPLHEQHGM | HLA-B*3503 |
| | | | (SEQ. ID NO.:103) | |
| HCV | NS3 | 389-397 | HSKKKCDEL | HLA-B8 |
| | | | (SEQ. ID NO.:104) | |
| HCV | env E | 44-51 | ASRCWVAM | HLA-B*3501 |
| | | | (SEQ. ID NO.:105) | |
| HCV | core protein | 27-35 | GQIVGGVYL | HLA-B*40012 |
| | | | (SEQ. ID NO.:106) | |
| HCV | NSI | 77-85 | PPLTDFDQGW | HLA-B*5301 |
| | | | (SEQ. ID NO.: 107) | |
| HCV | core protein | 18-27 | LMGYIPLVGA | H2-Dd |
| | | | (SEQ. ID NO.:108) | |
| HCV | core protein | 16-25 | ADLMGYIPLV | H2-Dd |
| | | | (SEQ. ID NO.:109) | |
| HCV | NS5 | 409-424 | MSYSWTGALVTPCAEE | H2-Dd |
| | | | (SEQ. ID NO.:110) | |
| HCV | NS1 | 205-213 | KHPDATYSR | Papa-A06 |
| | | | (SEQ. ID NO.:111) | |
| HCV-1 | NS3 | 400-409 | KLVALGINAV | HLA-A*0201 |
| | | | (SEQ. ID NO.: 112) | |
| HCV-1 | NS3 | 440-448 | GDFDSVIDC | Patr-B16 |
| | | | (SEQ. ID NO.: 113) | |
| HCV-1 | env E | 118-126 | GNASRCWVA | Patr-B16 |
| | | | (SEQ. ID NO.: 114) | |
| HCV-1 | NSI | 159-167 | TRPPLGNWF | Patr-B13 |
| | | | (SEQ. ID NO.:115) | |
| HCV-1 | NS3 | 351-359 | VPHPNIEEV | Patr-B13 |
| | | | (SEQ. ID NO.:116) | |
| HCV-1 | NS3 | 438-446 | YTGDFDSVI | Patr-B01 |
| | | | (SEQ. ID NO.: 117) | |
| HCV-1 | NS4 | 328-335 | SWAIKWEY | Patr-Al1 |
| | | | (SEQ. ID NO.:118) | |
| HCV-1 | NSI | 205-213 | KHPDATYSR | Patr-A04 |
| | | | (SEQ. ID NO.: 119) | |
| HCV-1 | NS3 | 440-448 | GDFDSVIDC | Patr-A04 |
| | | | (SEQ. ID NO.: 120) | |
| HIV | gp41 | 583-591 | RYLKDQQLL | HLA A24 |
| | | | (SEQ. ID NO.:121) | |
| HIV | gagp24 | 267-275 | IVGLNKIVR | HLA-A*3302 |
| | | | (SEQ. ID NO.:122) | |
| HIV | gagp24 | 262-270 | EIYKRWllL | HLA-B8 |
| | | | (SEQ. ID NO.: 123) | |
| HIV | gagp24 | 261-269 | GEIYKRWII | HLA-B8 |
| | | | (SEQ. ID NO.: 124) | |
| HIV | gagp17 | 93-101 | EIKDTKEAL | HLA-B8 |
| | | | (SEQ. ID NO.: 125) | |
| HIV | gp41 | 586-593 | YLKDQQLL | HLA-B8 |
| | | | (SEQ. ID NO.:126) | |
| HIV | gagp24 | 267-277 | ILGLNKIVRMY | HLA-B* 1501 |
| | | | (SEQ. ID NO.:127) | |
| HIV | gp41 | 584-592 | ERYLKDQQL | HLA-B14 |
| | | | (SEQ. ID NO.: 128) | |
| HIV | nef | 115-125 | YHTQGYFPQWQ | HLA-B17 |
| | | | (SEQ. ID NO.:129) | |
| HIV | nef | 117-128 | TQGYFPQWQNYT | HLA-B17 |
| | | | (SEQ. ID NO.:130) | |
| HIV | gp120 | 314-322 | GRAFVTIGK | HLA-B*2705 |
| | | | (SEQ. ID NO.:131) | |
| HIV | gagp24 | 263-271 | KRWIILGLN | HLA-B*2702 |
| | | | (SEQ. ID NO.:132) | |
| HIV | nef | 72-82 | QVPLRPMTYK | HLA-B*3501 |
| | | | (SEQ. ID NO.: 133) | |
| HIV | nef | 117-125 | TQGYFPQWQ | HLA-B*3701 |
| | | | (SEQ. ID NO.: 134) | |
| HIV | gagp24 | 143-151 | HQAISPRTI, | HLA-Cw*0301 |
| | | | (SEQ. ID NO.: 135) | |
| HIV | gagp24 | 140-151 | QMVHQAISPRTL | HLA-Cw*0301 |
| | | | (SEQ. ID NO.:136) | |
| HIV | gp120 | 431-440 | MYAPPIGGQI | H2-Kd |
| | | | (SEQ. ID NO.: 137) | |
| HIV | gp160 | 318-327 | RGPGRAFVTI | H2-Dd |
| | | | (SEQ. ID NO.:138) | |
| HIV | gp120 | 17-29 | MPGRAFVTI | H2-Ld |
| | | | (SEQ. ID NO.:139) | |
| HIV-1 | RT | 476-484 | ILKEPVHGV | HLA-A*0201 |
| | | | (SEQ. ID NO.:140) | |
| HIV-1 | nef | 190-198 | AFHHVAREL | HLA-A*0201 |
| | | | (SEQ. ID NO.:141) | |
| HIV-1 | gp160 | 120-128 | KLTPLCVTL | HLA-A*0201 |
| | | | (SEQ. ID NO.:142) | |
| HIV-1 | g]60 | 814-823 | SLLNATDIAV | HLA-A*0201 |
| | | | (SEQ. ID NO.:143) | |
| HIV-1 | RT | 179-187 | VIYQYMDDL | HLA-A*0201 |
| | | | (SEQ. ID NO.:144) | |
| HIV-1 | gagp 17 | 77-85 | SLYNTVATL | HLA-A*0201 |
| | | | (SEQ. ID NO.:145) | |
| HIV-1 | gp160 | 315-329 | RGPGRAFVTl | HLA-A*0201 |
| | | | (SEQ. ID NO.:146) | |
| HIV-1 | gp41 | 768-778 | RLRDLLLIVTR | HLA-A3 |
| | | | (SEQ. ID NO.:147) | |
| HIV-1 | nef | 73-82 | QVPLRPMTYK | HLA-A3 |
| | | | (SEQ. ID NO.:148) | |
| HIV-1 | gp120 | 36-45 | TVYYGVPVWK | HLA-A3 |
| | | | (SEQ. ID NO.:149) | |
| HIV-1 | gagp17 | 20-29 | RLRPGGKKK | HLA-A3 |
| | | | (SEQ. ID NO.:150) | |
| HIV-1 | gp120 | 38-46 | VYYGVPVWK | HLA-A3 |
| | | | (SEQ. ID NO.:151) | |
| HIV-1 | nef | 74-82 | VPLRPMTYK | HLA-a*1101 |
| | | | (SEQ. ID NO.:152) | |
| HIV-1 | gagp24 | 325-333 | AIFQSSMTK | HLA-A*1101 |
| | | | (SEQ. ID NO.:153) | |
| HIV-1 | nef | 73-82 | QVPLRPMTYK | HLA-A*1101 |
| | | | (SEQ. ID NO.:154) | |
| HIV-1 | nef | 83-94 | AAVDLSHFLKEK | HLA-A*1101 |
| | | | (SEQ. ID NO.:155) | |
| HIV-1 | gagp24 | 349-359 | ACQGVGGPGGHK | HLA-A*1101 |
| | | | (SEQ. ID NO.:156) | |
| HIV-1 | gagp24 | 203-212 | ETINEEAAEW | HLA-A25 |
| | | | (SEQ. ID NO.:157) | |
| HIV-1 | nef | 128-137 | TPGPGVRYPL | HLA-B7 |
| | | | (SEQ. ID NO.:158) | |
| HIV-1 | gagp 17 | 24-31 | GGKKKYKL | HLA-B8 |
| | | | (SEQ. ID NO.:159) | |
| HIV-1 | gp120 | 2-10 | RVKEKYQHL | HLA-B8 |
| | | | (SEQ. ID NO.:160) | |
| HIV-1 | gagp24 | 298-306 | DRFYKTLRA | HLA-B 14 |
| | | | (SEQ. ID NO.:161) | |
| HIV-1 | NEF | 132-147 | GVRYPLTFGWCYKLVP | HLA-B18 |
| | | | (SEQ. ID NO.:162) | |
| HIV-1 | gagp24 | 265-24 | KRWIILGLNK | HLA-B*2705 |
| | | | (SEQ. ID NO.:163) | |
| HIV-1 | nef | 190-198 | AFHHVAREL | HLA-B*5201 |
| | | | (SEQ. ID NO.:164) | |
| EBV | EBNA-6 | 335-343 | KEHVIQNAF | HLA-B44 |
| | | | (SEQ. ID NO.:165) | |
| EBV | EBNA-6 | 130-139 | EENLLDFVRF | HLA-B*4403 |
| | | | (SEQ. ID NO.:166) | |
| EBV | EBNA-2 | 42-51 | DTPLIPLTIF | HLA-B51 |
| | | | (SEQ. ID NO.:167) | |
| EBV | EBNA-6 | 213-222 | QNGALAINTF | HLA-1362 |
| | | | (SEQ. ID NO.:168) | |
| EBV | EBNA-3 | 603-611 | RLRAEAGVK | HLA-A3 |
| | | | (SEQ. ID NO.:169) | |
| HBV | sAg | 348-357 | GLSPTVWLSV | HLA-A*0201 |
| | | | (SEQ. ID NO.:170) | |
| HBV | SAg | 335-343 | WLSLLVPFV | HLA-A*0201 |
| | | | (SEQ. ID NO.:171) | |
| HBV | cAg | 18-27 | FLPSDFFPSV | HLA-A*0201 |
| | | | (SEQ. ID NO.:172) | |
| HBV | cAg | 18-27 | FLPSDFFPSV | HLA-A*0202 |
| | | | (SEQ. ID NO.:173) | |
| HBV | cAg | 18-27 | FLPSDFFPSV | HLA-A*0205 |
| | | | (SEQ. ID NO.:174) | |
| HBV | cAg | 18-27 | FLPSDFFPSV | HLA-A*0206 |
| | | | (SEQ. ID NO.:175) | |
| HBV | pol | 575-583 | FLLSLGIHL | HLA-A*020 |
| | | | (SEQ. ID NO.:176) | |
| HBV | pol | 816-824 | SLYADSPSV | HLA-A*020 |
| | | | (SEQ. ID NO.:177) | |
| HBV | pol | 455-463 | GLSRYVARL | HLA-A*0201 |
| | | | (SEQ. ID NO.:178) | |
| HBV | env | 338-347 | LLVPFVQWFV | HLA-A*020 |
| | | | (SEQ. ID NO.:179) | |
| HBV | pol | 642-650 | ALMPLYACI | HLA-A*0201 |
| | | | (SEQ. ID NO.:180) | |
| HBV | env | 378-387 | LLPIFFCLWV | HLA-A*0201 |
| | | | (SEQ. ID NO.:181) | |
| HBV | pol | 538-546 | YMDDWLGA | HLA-A*0201 |
| | | | (SEQ. ID NO.:182) | |
| HBV | env | 250-258 | LLLCLIFLL | HLA-A*0201 |
| | | | (SEQ. ID NO.:183) | |
| HBV | env | 260-269 | LLDYQGMLPV | HLA-A*0201 |
| | | | (SEQ. ID NO.:184) | |
| HBV | env | 370-379 | SIVSPFIPLL | HLA-A*0201 |
| | | | (SEQ. ID NO.:185) | |
| HBV | env | 183-191 | FLLTRILTI | HLA-A*0201 |
| | | | (SEQ. ID NO.:186) | |
| HBV | cAg | 88-96 | YVNVNMGLK | HLA-A* 1101 |
| | | | (SEQ. ID NO.:187) | |
| HBV | cAg | 141-151 | STLPETTVVRR | HLA-A*3101 |
| | | | (SEQ. ID NO.:188) | |
| HBV | cAg | 141-151 | STLPETTVVRR | HLA-A*6801 |
| | | | (SEQ. ID NO.:189) | |
| HBV | cAg | 18-27 | FLPSDFFPSV | HLA-A*6801 |
| | | | (SEQ. ID NO.:190) | |
| HBV | sAg | 28-39 | IPQSLDSWWTSL | H2-Ld |
| | | | (SEQ. ID NO.:191) | |
| HBV | cAg | 93-100 | MGLKFRQL | H2-Kb |
| | | | (SEQ. ID NO.:192) | |
| HBV | preS | 141-149 | STBXQSGXQ | HLA-A*0201 |
| | | | (SEQ. ID NO.:193) | |
| HCMV | gp B | 618-628 | FIAGNSAYEYV | HLA-A*0201 |
| | | | (SEQ. ID NO.:194) | |
| HCMV | El | 978-989 | SDEEFAIVAYTL | HLA-B18 |
| | | | (SEQ. ID NO.:195) | |
| HCMV | pp65 | 397-411 | DDVWTSGSDSDEELV | HLA-b35 |
| | | | (SEQ. ID NO.:196) | |
| HCMV | pp65 | 123-131 | IPSINVHHY | HLA-B*3501 |
| | | | (SEQ. ID NO.:197) | |
| HCMV | pp65 | 495-504 | NLVPMVATVO | HLA-A*0201 |
| | | | (SEQ. ID NO.:198) | |
| HCMV | pp65 | 415-429 | RKTPRVTOGGAMAGA | HLA-B7 |
| | | | (SEQ. ID NO.:199) | |
| HCV | MP | 17-25 | DLMGYIPLV | HLA-A*0201 |
| | | | (SEQ. ID NO.:200) | |
| HCV | MP | 63-72 | LLALLSCLTV | HLA-A*0201 |
| | | | (SEQ. ID NO.:201) | |
| HCV | MP | 105-112 | ILHTPGCV | HLA-A*0201 |
| | | | (SEQ. ID NO.:202) | |
| HCV | env E | 66-75 | QLRRHIDLLV | HLA-A*0201 |
| | | | (SEQ. ID NO.:203) | |
| HCV | env E | 88-96 | DLCGSVFLV | HLA-A*0201 |
| | | | (SEQ. ID NO.:204) | |
| HCV | env E | 172-180 | SMVGNWAKV | HLA-A*0201 |
| | | | (SEQ. ID NO.:205) | |
| HCV | NSI | 308-316 | HLIIQNIVDV | HLA-A*0201 |
| | | | (SEQ. ID NO.:206) | |
| HCV | NSI | 340-348 | FLLLADARV | HLA-A*0201 |
| | | | (SEQ. ID NO.:207) | |
| HCV | NS2 | 234-246 | GLRDLAVAVEPVV | HLA-A*0201 |
| | | | (SEQ. ID NO.:208) | |
| HCV | NSI | 18-28 | SLLAPGAKQNV | HLA-A*0201 |
| | | | (SEQ. ID NO.:209) | |
| HCV | NSI | 19-28 | LLAPGAKQNV | HLA-A*0201 |
| | | | (SEQ. ID NO.:210) | |
| HCV | NS4 | 192-201 | LLFNILGGWV | HLA-A*0201 |
| | | | (SEQ. ID NO.:211) | |
| HCV | NS3 | 579-587 | YLVAYQATV | HLA-A*0201 |
| | | | (SEQ. ID NO.:212) | |
| HCV | core protein | 34-43 | YLLPRRGPRL | HLA-A*0201 |
| | | | (SEQ. ID NO.:213) | |
| HCV | MP | 63-72 | LLALLSCLTI | HLA-A*0201 |
| | | | (SEQ. ID NO.:214) | |
| HCV | NS4 | 174-182 | SLMAFTAAV | HLA-A*0201 |
| | | | (SEQ. ID NO.:215) | |
| HCV | NS3 | 67-75 | CINGVCWTV | HLA-A*0201 |
| | | | (SEQ. ID NO.:216) | |
| HCV | NS3 | 163-171 | LLCPAGHAV | HLA-A*0201 |
| | | | (SEQ. ID NO.:217) | |
| HCV | NS5 | 239-247 | ILDSFDPLV | HLA-A*0201 |
| | | | (SEQ. ID NO.:218) | |
| HCV | NS4A | 236-244 | ILAGYGAGV | HLA-A*0201 |
| | | | (SEQ. ID NO.:219) | |
| HCV | NS5 | 714-722 | GLQDCTMLV | HLA-A*0201 |
| | | | (SEQ. ID NO.:220) | |
| HCV | NS3 | 281-290 | TGAPVTYSTY | HLA-A*0201 |
| | | | (SEQ. ID NO.:221) | |
| HCV | NS4A | 149-157 | HMWNFISGI | HLA-A*0201 |
| | | | (SEQ. ID NO.:222) | |
| HCV | NS5 | 575-583 | RVCEKMALY | HLA-A*0201-A3 |
| | | | (SEQ. ID NO.:223) | |
| HCV | NS1 | 238-246 | TINYTIFK | HLA-A*1101 |
| | | | (SEQ. ID NO.:224) | |
| HCV | NS2 | 109-116 | YISWCLWW | HLA-A23 |
| | | | (SEQ. ID NO.:225) | |
| HCV | core protein | 40-48 | GPRLGVRAT | HLA-B7 |
| | | | (SEQ. ID NO.:226) | |
| HIV-1 | gp120 | 380-388 | SFNCGGEFF | HLA-Cw*0401 |
| | | | (SEQ. ID NO.:227) | |
| HIV-1 | RT | 206-214 | TEMEKEGKI | H2-Kk |
| | | | (SEQ. ID NO.:228) | |
| HIV- 1 | p17 | 18-26 | KIRLRPGGK | HLA-A*0301 |
| | | | (SEQ. ID NO.:229) | |
| HIV-1 | P17 | 20-29 | RLRPGGKKKY | HLA-A*0301 |
| | | | (SEQ. ID NO.:230) | |
| HIV- | RT | 325-333 | AIFQSSMTK | HLA-A*0301 |
| | | | (SEQ. ID NO.:231) | |
| HIV-1 | p17 | 84-92 | TLYCVHQRI | HLA-A11 |
| | | | (SEQ. ID NO.:232) | |
| HIV-1 | RT | 508-517 | IYQEPFKNLK | HLA-A11 |
| | | | (SEQ. ID NO.:233) | |
| HIV-1 | P17 | 28-36 | KYKLKHIVW | HLA-A24 |
| | | | (SEQ. ID NO.:234) | |
| HIV-1 | gp120 | 53-62 | LFCASDAKAY | HLA-A24 |
| | | | (SEQ. ID NO.:235) | |
| HIV-1 | gagp24 | 145-155 | QAISPRTLNAW | HLA-A25 |
| | | | (SEQ. ID NO.:236) | |
| HIV-1 | gagp24 | 167-175 | EVIPMFSAL | HLA-A26 |
| | | | (SEQ. ID NO.:237) | |
| HIV-1 | RT | 593-603 | ETFYVDGAANR | HLA-A26 |
| | | | (SEQ. ID NO.:238) | |
| HIV-1 | gp41 | 775-785 | RLRDLLLIVTR | HLA-A31 |
| | | | (SEQ. ID NO.:239) | |
| HIV-1 | RT | 559-568 | PIQKETWETW | HLA-A32 |
| | | | (SEQ. ID NO.:240) | |
| HCV-1 | gp120 | 419-427 | RIKQIINMW | HLA-A32 |
| | | | (SEQ. ID NO.:241) | |
| HIV-1 | RT | 71-79 | ITLWQRPLV | HLA-A*6802 |
| | | | (SEQ. ID NO.:242) | |
| HIV-1 | RT | 85-93 | DTVLEEMNL | HLA-A*6802 |
| | | | (SEQ. ID NO.:243) | |
| HIV-1 | RT | 71-79 | ITLWQRPLV | HLA-A*7401 |
| | | | (SEQ. ID NO.:244) | |
| H1V-1 | gag p24 | 148-156 | SPRTLNAWV | HLA-B7 |
| | | | (SEQ. ID NO.:245) | |
| HIV-1 | gagp24 | 179-187 | ATPQDLNTM | HLA-B7 |
| | | | (SEQ. ID NO.:246) | |
| HIV-1 | gp120 | 303-312 | RPNNNTRKSI | HLA-B7 |
| | | | (SEQ. ID NO.:247) | |
| HIV-1 | gp41 | 843-851 | IPRRIRQGL | HLA-B7 |
| | | | (SEQ. ID NO.:248) | |
| HIV-1 | p17 | 74-82 | ELRSLYNTV | HLA-B8 |
| | | | (SEQ. ID NO.:249) | |
| HIV-1 | nef | 13-20 | WPTVRERM | HLA-B8 |
| | | | (SEQ. ID NO.:250) | |
| HIV-1 | nef | 90-97 | FLKEKGGL | HLA-B8 |
| | | | (SEQ. ID NO.:251) | |
| HIV-1 | gag p24 | 183-191 | DLNTMLNTV | HLA-B14 |
| | | | (SEQ. ID NO.:252) | |
| HIV-1 | P17 | 18-27 | KIRLRPGGKK | HLA-B27 |
| | | | (SEQ. ID NO.:253) | |
| HIV-1 | p17 | 19-27 | IRLRPGGKK | HLA--827 |
| | | | (SEQ. ID NO.:254) | |
| HIV-1 | gp41 | 791-799 | GRRGWEALKY | HLA-B27 |
| | | | (SEQ. ID NO.:255) | |
| HIV-1 | nef | 73-82 | QVPLRPMTYK | HLA-B27 |
| | | | (SEQ. ID NO.:256) | |
| HIV-1 | GP41 | 590-597 | RYLKDQQL | HLA-B27 |
| | | | (SEQ. ID NO.:257) | |
| HIV-1 | nef | 105-114 | RRQDILDLWI | HLA-B*2705 |
| | | | (SEQ. ID NO.:258) | |
| HIV-1 | nef | 134-141 | RYPLTFGW | HLA-B*2705 |
| | | | (SEQ. ID NO.:259) | |
| H1V-1 | p17 | 36-44 | WASRELERF | HLA-B35 |
| | | | (SEQ. ID NO.:260) | |
| HIV-1 | GAG P24 | 262-270 | TVLDVGDAY | HLA-B35 |
| | | | (SEQ. ID NO.:261) | |
| HIV-1 | gp120 | 42-52 | VPVWKEATTTL | HLA-B35 |
| | | | (SEQ. ID NO.:262) | |
| HIV-1 | Pl7 | 36-44 | NSSKVSQNY | HLA-B35 |
| | | | (SEQ. ID NO.:263) | |
| HIV-1 | gag p24 | 254-262 | PPIPVGDIY | HLA-B35 |
| | | | (SEQ. ID NO.:264) | |
| HIV-1 | RT | 342-350 | HPDIVIYQY | HLA-B35 |
| | | | (SEQ. ID NO.:265) | |
| HIV-1 | gp41 | 611-619 | TAVPWNASW | HLA-B35 |
| | | | (SEQ. ID NO.:266) | |
| HIV-1 | gag | 245-253 | NPVPVGNIY | HLA-B35 |
| | | | (SEQ. ID NO.:267) | |
| HIV-1 | nef | 120-128 | YFPDWQNYT | HLA-B37 |
| | | | (SEQ. ID NO.:268) | |
| HIV-1 | gag p24 | 193-201 | GHQAAMQML | HLA-B42 |
| | | | (SEQ. ID NO.:269) | |
| HIV-1 | p17 | 20-29 | RLRPGGKKKY | HLA-B42 |
| | | | (SEQ. ID NO.:270) | |
| HIV-1 | RT | 438-446 | YPGIKVRQL | HLA-B42 |
| | | | (SEQ. ID NO.:271) | |
| HIV-1 | RT | 591-600 | GAETFYVDGA | HLA-B45 |
| | | | (SEQ. ID NO.:272) | |
| HIV-1 | gag p24 | 325-333 | NANPDCKTI | HLA-B51 |
| | | | (SEQ. ID NO.:273) | |
| HIV-1 | gag p24 | 275-282 | RMYSPTSI | HLA-B52 |
| | | | (SEQ. ID NO.:274) | |
| HIV-1 | gp120 | 42-51 | VPVWKEATTT | HLA-B*5501 |
| | | | (SEQ. ID NO.:275) | |
| HIV-1 | gag p24 | 147-155 | ISPRTLNAW | HLA-B57 |
| | | | (SEQ. ID NO.:276) | |
| HIV-1 | gag p24 | 240-249 | TSTLQEQIGW | HLA-B57 |
| | | | (SEQ. ID NO.:277) | |
| HIV-1 | gag p24 | 162-172 | KAFSPEVIPMF | HLA-B57 |
| | | | (SEQ. ID NO.:278) | |
| HIV-1 | gag p24 | 311-319 | QASQEVKNW | HLA-B57 |
| | | | (SEQ. ID NO.:279) | |
| HIV-1 | gag p24 | 311-319 | QASQDVKNW | HLA-B57 |
| | | | (SEQ. ID NO.:280) | |
| HIV-1 | nef | 116-125 | HTQGYFPDWQ | HLA-B57 |
| | | | (SEQ. ID NO.:281) | |
| HIV-1 | nef | 120-128 | YFPDWQNYT | HLA-B57 |
| | | | (SEQ. ID NO.:282) | |
| HIV-1 | gag p24 | 240-249 | TSTLQEQIGW | HLA-B58 |
| | | | (SEQ. ID NO.:283) | |
| HTV-1 | p17 | 20-29 | RLRPGGKKKY | HLA-B62 |
| | | | (SEQ. ID NO.:284) | |
| HIV-1 | p24 | 268-277 | LGLNKJVRMY | HLA-B62 |
| | | | (SEQ. ID NO.:285) | |
| HIV-1 | RT | 415-426 | LVGKLNWASQIY | HLA-B62 |
| | | | (SEQ. ID NO.:286) | |
| HIV-1 | RT | 476-485 | ILKEPVHGVY | HLA-B62 |
| | | | (SEQ. ID NO.:287) | |
| HIV-1 | nef | 117-127 | TQGYFPDWQNY | HLA-B62 |
| | | | (SEQ. ID NO.:288) | |
| HIV-1 | nef | 84-91 | AVDLSHFL | HLA-B62 |
| | | | (SEQ. ID NO.:289) | |
| HIV-1 | gag p24 | 168-175 | VIPMFSAL | HLA-Cw*0102 |
| | | | (SEQ. ID NO.:290) | |
| HIV-1 | gp120 | 376-384 | FNCGGEFFY | HLA-A29 |
| | | | (SEQ. ID NO.:291) | |
| HIV-1 | gp120 | 375-383 | SFNCGGEFF | HLA-B15 |
| | | | (SEQ. ID NO.:292) | |
| HIV-1 | nef | 136-145 | PLTFGWCYKL | HLA-A*0201 |
| | | | (SEQ. ID NO.:293) | |
| HIV-1 | nef | 180-189 | VLEWRFDSRL | HLA-A*0201 |
| | | | (SEQ. ID NO.:294) | |
| HIV-1 | nef | 68-77 | FPVTPQVPLR | HLA-B7 |
| | | | (SEQ. ID NO.:295) | |
| HN-1 | nef | 128-137 | TPGPGVRYPL | HLA-B7 |
| | | | (SEQ. ID NO.:296) | |
| HIV-1 | gag p24 | 308-316 | QASQEVKNW | HLA-Cw*0401 |
| | | | (SEQ. ID NO.:297) | |
| HIV-1 IIIB | RT | 273-282 | VPLDEDFRKY | HLA-B35 |
| | | | (SEQ. ID NO.:298) | |
| HIV-1 IIIB | RT | 25-33 | NPDIVIYQY | HLA-B35 |
| | | | (SEQ. ID NO.:299) | |
| HIV-1 IIIB | gp41 | 557-565 | RAIEAQAHL | HLA-B51 |
| | | | (SEQ. ID NO.:300) | |
| HIV-1 IIIB | RT | 231-238 | TAFTIPSI | HLA-B51 |
| | | | (SEQ. ID NO.:301) | |
| HIV-1 IIIB | p24 | 215-223 | VHPVHAGPIA | HLA-B*5501 |
| | | | (SEQ. ID NO.:302) | |
| HIV-1 IIIB | gp120 | 156-165 | NCSFNISTSI | HLA-Cw8 |
| | | | (SEQ. ID NO.:303) | |
| HIV-1 IIIB | gp120 | 241-249 | CTNVSTVQC | HLA-Cw8 |
| | | | (SEQ. ID NO.:304) | |
| HIV-1 5F2 | gp120 | 312-320 | IGPGRAFHT | H2-Dd |
| | | | (SEQ. ID NO.:305) | |
| HIV-1 5F2 | pol | 25-33 | NPDIVIYQY | HLA-B*3501 |
| | | | (SEQ. ID NO.:306) | |
| HN-15F2 | pol | 432-441 | EPIVGAETFY | HLA-B*3501 |
| | | | (SEQ. ID NO.:307) | |
| HIV-1 5F2 | pol | 432-440 | EPIVGAETF | HLA-B*3501 |
| | | | (SEQ. ID NO.:308) | |
| HIV-1 5F2 | pol | 6-14 | SPAIFQSSM | HLA-B*3501 |
| | | | (SEQ. ID NO.:309) | |
| HIV-1 5F2 | pol | 59-68 | VPLDKDFRKY | HLA-B*3501 |
| | | | (SEQ. ID NO.:310) | |
| HIV-1 5F2 | pol | 6-14 | IPLTEEAEL | HLA-B*3501 |
| | | | (SEQ. ID NO.:311) | |
| HIV-1 5F2 | nef | 69-79 | RPQVPLRPMTY | HLA-B*3501 |
| | | | (SEQ. ID NO.:312) | |
| HIV-1 5F2 | nef | 66-74 | FPVRPQVPL | HLA-B*3501 |
| | | | (SEQ. ID NO.:313) | |
| HIV-1 5F2 | env | 10-18 | DPNPQEVVL | HLA-B*3501 |
| | | | (SEQ. ID NO.:314) | |
| HIV-1 5F2 | env | 7-15 | RPIVSTQLL | HLA-B*3501 |
| | | | (SEQ. ID NO.:315) | |
| HIV-1 5F2 | pol | 6-14 | IPLTEEAEL | HLA-B51 |
| | | | (SEQ. ID NO.:316) | |
| HIV-1 5F2 | env | 10-18 | DPNPQEVVL | HLA-B51 |
| | | | (SEQ. ID NO.:317) | |
| HIV-1 5F2 | gagp24 | 199-207 | AMQMLKETI | H2-Kd |
| | | | (SEQ. ID NO.:318) | |
| HIV-2 | gagp24 | 182-190 | TPYDrNQML | HLA-B*5301 |
| | | | (SEQ. ID NO.:319) | |
| HIV-2 | gag | 260-269 | RRWIQLGLQKV | HLA-B*2703 |
| | | | (SEQ. ID NO.:320) | |
| HIV-1 5F2 | gp41 | 593-607 | GIWGCSGKLICTTAV | HLA-B17 |
| | | | (SEQ. ID NO.:321) | |
| HIV-1 5F2 | gp41 | 753-767 | ALIWEDLRSLCLFSY | HLA-B22 |
| | | | (SEQ. ID NO.:322) | |
| HPV 6b | E7 | 21-30 | GLHCYEQLV | HLA-A*0201 |
| | | | (SEQ. ID NO.:323) | |
| HPV 6b | E7 | 47-55 | PLKQHFQIV | HLA-A*0201 |
| | | | (SEQ. ID NO.:324) | |
| HPV11 | E7 | 4-12 | RLVTLKDIV | HLA-A*0201 |
| | | | (SEQ. ID NO.:325) | |
| HPV16 | E7 | 86-94 | TLGIVCPIC | HLA-A*0201 |
| | | | (SEQ. ID NO.:326) | |
| HPV16 | E7 | 85-93 | GTLGIVCPI | HLA-A*0201 |
| | | | (SEQ. ID NO.:327) | |
| HPV16 | E7 | 12-20 | MLDLQPETT | HLA-A*0201 |
| | | | (SEQ. ID NO.:328) | |
| HPV16 | E7 | 11-20 | YMLDLQPETT | HLA-A*0201 |
| | | | (SEQ. ID NO.:329) | |
| HPV16 | E6 | 15-22 | RPRKLPQL | HLA-B7 |
| | | | (SEQ. ID NO.:330) | |
| HPV16 | E6 | 49-57 | RAHYNIVTF | HW-Db |
| | | | (SEQ. ID NO.:331) | |
| HSV | gp B | 498-505 | SSIEFARL | H2-Kb |
| | | | (SEQ. ID NO.:332) | |
| HSV-1 | gp C | 480-488 | GIGIGVLAA | HLA-A*020 |
| | | | (SEQ. ID NO.:333) | |
| HSV-1 | ICP27 | 448-456 | DYATLGVGV | H2-Kd |
| | | | (SEQ. ID NO.:334) | |
| Virus | Protein | | T cell epitope MHC ligand | MHC molecule |
| | | | (Antigen) | |
| HSV-1 | ICP27 | 322-332 | LYRTFAGNPRA | H2-Kd |
| | | | (SEQ. ID NO.:335) | |
| HSV-1 | UL39 | 822-829 | QTFDFGRL | H2-Kb |
| | | | (SEQ. ID NO.:336) | |
| HSV-2 | gpC | 446-454 | GAGIGVAVL | HLA-A*0201 |
| | | | (SEQ. ID NO.:337) | |
| HLTV-1 | TAX | 11-19 | LLFGYPVYV | HLA-A*0201 |
| | | | (SEQ. ID NO.:338) | |
| Influenza | MP | 58-66 | GILGFVFTL | HLA-A*0201 |
| | | | (SEQ. ID NO.:339) | |
| Influenza | MP | 59-68 | ILGFVFTLTV | HLA-A*0201 |
| | | | (SEQ. ID NO.:340) | |
| Influenza | NP | 265-273 | ILRGSVAHK | HLA-A3 |
| | | | (SEQ. ID NO.:341) | |
| Influenza | NP | 91-99 | KTGGPIYKR | HLA-A*6801 |
| | | | (SEQ. ID NO.:342) | |
| Influenza | NP | 380-388 | ELRSRYWAI | HLA-B8 |
| | | | (SEQ. ID NO.:343) | |
| Influenza | NP | 381-388 | LRSRYWAI | HLA-B*2702 |
| | | | (SEQ. ID NO.:344) | |
| Influenza | NP | 339-347 | EDLRVLSFI | HLA-B*370 |
| | | | (SEQ. ID NO.:345) | |
| Influenza | NSI | 158-166 | GEISPLPSL | HLA-B44 |
| | | | (SEQ. ID NO.:346) | |
| Influenza | NP | 338-346 | FEDLRVLSF | HLA-B44 |
| | | | (SEQ. ID NO.:347) | |
| Influenza | NSI | 158-166 | GEISPLPSL | HLA-B*4402 |
| | | | (SEQ. ID NO.:348) | |
| Influenza | NP | 338-346 | FEDLRVLSF | HLA-B*4402 |
| | | | (SEQ. ID NO.:349) | |
| Influenza | PBI | 591-599 | VSDGGPKLY | HLA-Al |
| | | | (SEQ. ID NO.:350) | |
| Influenza A | NP | 44-52 | CTELKLSDY | HLA-Al |
| | | | (SEQ. ID NO.:351) | |
| Influenza | NSI | 122-130 | AIMDKNIIL | HLA-A*0201 |
| | | | (SEQ. ID NO.:352) | |
| Influenza A | NSI | 123-132 | IMDKNIILKA | HLA-A*0201 |
| | | | (SEQ. ID NO.:353) | |
| Influenza A | NP | 383-391 | SRYWAIRTR | HLA-B*2705 |
| | | | (SEQ. ID NO.:354) | |
| Influenza A | NP | 147-155 | TYQRTRALV | H2-Kd |
| | | | (SEQ. ID NO.:355) | |
| Influenza A | HA | 210-219 | TYVSVSTSTL | H2-Kd |
| | | | (SEQ. ID NO.:356) | |
| Influenza A | HA | 518-526 | IYSTVASSL | H2-Kd |
| | | | (SEQ. ID NO.:357) | |
| Influenza A | HA | 259-266 | FEANGNLI | H2-Kk |
| | | | (SEQ. ID NO.:358) | |
| Influenza A | HA | 10-18 | IEGGWTGMI | H2-Kk |
| | | | (SEQ. ID NO.:359) | |
| Influenza A | NP | 50-57 | SDYEGRLI | H2-Kk |
| | | | (SEQ. ID NO.:360) | |
| Influenza a | NSI | 152-160 | EEGAIVGEI | H2-Kk |
| | | | (SEQ. ID NO.:361) | |
| Influenza A34 | NP | 336-374 | ASNENMETM | H2Db |
| | | | (SEQ. ID NO.:362) | |
| Influenza A68 | NP | 366-374 | ASNENMDAM | H2Db |
| | | | (SEQ. ID NO.:363) | |
| Influenza B | NP | 85-94 | KLGEFYNQMM | HLA-A*0201 |
| | | | (SEQ. ID NO.:364) | |
| Influenza B | NP | 85-94 | KAGEFYNQMM | HLA-A*0201 |
| | | | (SEQ. ID NO.:365) | |
| Influenza JAP | HA | 204-212 | LYQNVGTYV | H2Kd |
| | | | (SEQ. ID NO.:366) | |
| Influenza JAP | HA | 210-219 | TYVSVGTSTL | H2-Kd |
| | | | (SEQ. ID NO.:367) | |
| Influenza JAP | HA | 523-531 | VYQILATYA | H2-Kd |
| | | | (SEQ. ID NO.:368) | |
| Influenza JAP | HA | 529-537 | IYATVAGSL | H2-Kd |
| | | | (SEQ. ID NO.:369) | |
| Influenza JAP | HA | 210-219 | TYVSVGTSTI(L>I) | H2-Kd |
| | | | (SEQ. ID NO.:370) | |
| Influenza JAP | HA | 255-262 | FESTGNLI | H2-Kk |
| | | | (SEQ. ID NO.:371) | |
| JHMV | cAg | 318-326 | APTAGAFFF | H2-Ld |
| | | | (SEQ. ID NO.:372) | |
| LCMV | NP | 118-126 | RPQASGVYM | H2-Ld |
| | | | (SEQ. ID NO.:373) | |
| LCMV | NP | 396-404 | FQPQNGQFI | H2-Db |
| | | | (SEQ. ID NO.:374) | |
| LCMV | GP | 276-286 | SGVENPGGYCL | H2-Db |
| | | | (SEQ. ID NO.:375) | |
| LCMV | GP | 33-42 | KAVYNFATCG | H2-Db |
| | | | (SEQ. ID NO.:376) | |
| MCMV | pp89 | 168-176 | YPHFMPTNL | H2-Ld |
| | | | (SEQ. ID NO.:377) | |
| MHV | spike protein | 510-518 | CLSWNGPHL | H2-Db |
| | | | (SEQ. ID NO.:378) | |
| MMTV | env gp 36 | 474-482 | SFAVATTAL | H2-Kd |
| | | | (SEQ. ID NO.:379) | |
| MMTV | gag p27 | 425-433 | SYETFISRL | H2-Kd |
| | | | (SEQ. ID NO.:380) | |
| MMTV | env gp73 | 544-551 | ANYDFICV | H2-Kb |
| | | | (SEQ. ID NO.:381) | |
| MuLV | env p15E | 574-581 | KSPWFTTL | H2-Kb |
| | | | (SEQ. ID NO.:382) | |
| MuLV | env gp70 | 189-196 | SSWDFITV | H2-Kb |
| | | | (SEQ. ID NO.:383) | |
| MuLV | gag 75K | 75-83 | CCLCLTVFL | H2-Db |
| | | | (SEQ. ID NO.:384) | |
| MuLV | env gp70 | 423-431 | SPSYVYHQF | H2Ld |
| | | | (SEQ. ID NO.:385) | |
| MV | F protein | 437-447 | SRRYPDAVYLH | HLA-B*2705 |
| | | | (SEQ. ID NO.:386) | |
| Mv | F protein | 438-446 | RRYPDAVYL | HLA-B*2705 |
| Virus | Protein | AA Position | T cell epitope MHC ligand (Antigen) | MHC molecule |
| | | | (SEQ. ID NO.:387) | |
| Mv | NP | 281-289 | YPALGLHEF | H2-Ld |
| | | | (SEQ. ID NO.:388) | |
| Mv | HA | 343-351 | DPVIDRLYL | H2-Ld |
| | | | (SEQ. ID NO.:389) | |
| MV | HA | 544-552 | SPGRSFSYF | H2-Ld |
| | | | (SEQ. ID NO.:390) | |
| Poliovirus | VP1 | 111-118 | TYKDTVQL | H2-kd |
| | | | (SEQ. ID NO.:391) | |
| Poliovirus | VP1 | 208-217 | FYDGFSKVPL | H2-Kd |
| | | | (SEQ. ID NO.:392) | |
| Pseudorabies virus gp | G111 | 455-463 | IAGIGILAI | HLA-A*0201 |
| | | | (SEQ. ID NO.:393) | |
| Rabiesvirus | NS | 197-205 | VEAEIAHQI | H2-Kk |
| | | | (SEQ. ID NO.:394) | |
| Rotavirus | VP7 | 33-40 | IIYRFLLI | H2-Kb |
| | | | (SEQ. ID NO.:395) | |
| Rotavirus | VP6 | 376-384 | VGPVFPPGM | H2-Kb |
| | | | (SEQ. ID NO.:396) | |
| Rotavirus | VP3 | 585-593 | YSGYIFRDL | H2-Kb |
| | | | (SEQ. ID NO.:397) | |
| RSV | M2 | 82-90 | SYIGSINNI | H2-Kd |
| | | | (SEQ. ID NO.:398) | |
| SIV | gagp11C | 179-190 | EGCTPYDTNQML | Mamu-A*01 |
| | | | (SEQ. ID NO.:399) | |
| SV | NP | 324-332 | FAPGNYPAL | H2-Db |
| | | | (SEQ. ID NO.:400) | |
| SV | NP | 324-332 | FAPCTNYPAL | H2-Kb |
| | | | (SEQ. ID NO.:401) | |
| SV40 | T | 404-411 | VVYDFLKC | H2-Kb |
| | | | (SEQ. ID NO.:402) | |
| SV40 | T | 206-215 | SAINNYAQKL | H2-Db |
| | | | (SEQ. ID NO.:403) | |
| SV40 | T | 223-231 | CKGVNKEYL | H2-Db |
| | | | (SEQ. ID NO.:404) | |
| SV40 | T | 489-497 | QGINNLDNL | H2-Db |
| | | | (SEQ. ID NO.:405) | |
| SV40 | T | 492-500 | NNLDNLRDY(L) | H2-Db |
| | | (501) | | |
| | | | (SEQ. ID NO.:406) | |
| SV40 | T | 560-568 | SEFLLEKRI | H2-Kk |
| | | | (SEQ. ID NO.:407) | |
| VSV | NP | 52-59 | RGYVYQGL | H2-Kb |
| | | | (SEQ. ID NO.:408) | |

**Table 2**

| **HLA-A1** | **Position (Antigen)** | **Source** |
|---|---|---|
| T cell epitopes | EADPTGHSY | MAGE-1 161-169 |
| | (SEQ. ID NO.:409) | |
| | VSDGGPNLY | Influenza A PB 1591-599 |
| | (SEQ. ID NO.:410) | |
| | CTELKLSDY | Influenza A NP 44-52 |
| | (SEQ. ID NO.:411) | |
| | EVDPIGHLY | MAGE-3 168-176 |
| | (SEQ. ID NO.:412) | |
| HLA-A201 | MLLSVPLLLG | Calreticulin signal sequence I-10 |
| | (SEQ, ID NO.:413) | |
| | STBXQSGXQ | HBV PRE-S PROTEIN 141-149 |
| | (SEQ. ID NO.:414) | |
| | YMDGTMSQV | Tyrosinase 369-377 |
| | (SEQ. ID NO.:415) | |
| | ILKEPVHGV | HIV - I RT 476-484 |
| | (SEQ. ID NO.:416) | |
| | LLGFVFTLTV | Influenza MP 59-68 |
| | (SEQ. ID NO.:417) | |
| | LLFGYPVYVV | HTLV-1 tax 11-19 |
| | (SEQ. ID NO.:418) | |
| | GLSPTVWLSV | HBV sAg 348-357 |
| | (SEQ. ID NO.:419) | |
| | WLSLLVPFV | HBV sAg 335-343 |
| | (SEQ. ID NO.:420) | |
| | FLPSDFFPSV | HBV cAg 18-27 |
| | (SEQ. ID NO.:421) | |
| | CLG0LLTMV | EBV LMP-2 426-434 |
| | (SEQ. ID NO.:422) | |
| | FLAGNSAYEYV | HCMV gp 618-628B |
| | (SEQ. ID NO.:423) | |
| | KLGEFYNQMM | Influenza BNP 85-94 |
| | (SEQ. ID NO.:424) | |
| | KLVALGINAV | HCV-1 NS3 400-409 |
| | (SEQ. ID NO.:425) | |
| | DLMGYIPLV | HCV MP 17-25 |
| | (SEQ. ID NO.:426) | |
| | RLVTLKDIV | HPV 11 EZ 4-12 |
| | (SEQ. ID NO.:427) | |
| | MLLAVLYCL | Tyrosinase 1-9 |
| | (SEQ. ID NO.:428) | |
| | AAGIGILTV | Melan A\Mart-127-35 |
| | (SEQ. ID NO.:429) | |
| | YLEPGPVTA | Pmel 17/gp 100 480-488 |
| | (SEQ. ID NO.:430) | |
| | ILDGTATLRL | Pmel 17/ gp 100 457-466 |
| | (SEQ. ID NO.:431) | |
| | LLDGTATLRL | Pmel gplOO 457-466 |
| | (SEQ. ID NO.:432) | |
| | ITDQVPFSV | Pmel gp 100 209-217 |
| | (SEQ. ID NO.:433) | |
| | KTWGQYWQV | Pmel gp 100 154-162 |
| | (SEQ. ID NO.:434) | |
| | TITDQVPFSV | Pmel gp 100 208-217 |
| | (SEQ. ID NO.:435) | |
| | AFHIIVAREL | HIV-1 nef 190-198 |
| | (SEQ. ID NO.:436) | |
| | YLNKIQNSL | P. falciparum CSP 334-342 |
| | (SEQ. ID NO.:437) | |
| | MMRKLAILSV | P. falciparum CSP 1 -10 |
| | (SEQ. ID NO.:438) | |
| | KAGEFYNQMM | Influenza BNP 85-94 |
| | (SEQ. ID NO.:439) | |
| | NIAEGLRAL | EBNA-1 480-488 |
| | (SEQ. ID NO.:440) | |
| | NLRRGTALA | EBNA-1 519-527 |
| | (SEQ. ID NO.:441) | |
| | ALAIPQCRL | EBNA-1 525-533 |
| | (SEQ. ID NO.:442) | |
| | VLKDAIKDL | EBNA-1 575-582 |
| | (SEQ. ID NO.:443) | |
| | FMVFLQTHI | EBNA-1 562-570 |
| | (SEQ. ID NO.:444) | |
| | HLIVDTDSL | EBNA-2 15-23 |
| | (SEQ. ID NO.:445) | |
| | SLGNPSLSV | EBNA-2 22-30 |
| | (SEQ. ID NO.:446) | |
| | PLASAMRML | EBNA-2 126-134 |
| | (SEQ. ID NO.:447) | |
| | RMLWMANYI | EBNA-2 132-140 |
| | (SEQ. ID NO.:448) | |
| | MLWMANYIV | EBNA-2 133-141 |
| | (SEQ. ID NO.:449) | |
| | ILPQGPQTA | EBNA-2 151-159 |
| | (SEQ. ID NO.:450) | |
| | PLRPTAPTTI | EBNA-2 171-179 |
| | (SEQ. ID NO.:451) | |
| | PLPPATLTV | EBNA-2 205-213 |
| | (SEQ. ID NO.:452) | |
| | R M H L P V L H V | EBNA-2 246-254 |
| | (SEQ. ID NO.:453) | |
| | PMPLPPSQL | EBNA-2 287-295 |
| | (SEQ. ID NO.:454) | |
| | QLPPPAAPA | EBNA-2 294-302 |
| | (SEQ. ID NO.:455) | |
| | SMPELSPVL | EBNA-2 381-389 |
| | (SEQ. ID NO.:456) | |
| | DLDESWDYI | EBNA-2 453-461 |
| | (SEQ. ID NO.:457) | |
| | P L P C V L W P VV | BZLF1 43-51 |
| | (SEQ. ID NO.:458) | |
| | SLEECDSEL | BZLFI 167-175 |
| | (SEQ, ID NO.:459) | |
| | EIKRYKNRV | BZLFI 176-184 |
| | (SEQ. ID NO.:460) | |
| | QLLQFIYREV | BZLF1 195-203 |
| | (SEQ. ID NO.:461) | |
| | LLQHYREVA | BZLFI 196-204 |
| | (SEQ. ID NO.:462) | |
| | LLKQMCPSL | BZLFI 217-225 |
| | (SEQ. ID NO.:463) | |
| | SIIPRTPDV | BZLFI 229-237 |
| | (SEQ. ID NO.:464) | |
| | AIMDKNIIL | Influenza A NS1 122-130 |
| | (SEQ. ID NO.:465) | |
| | IMDKNIILKA | Influenza A NS1 123-132 |
| | (SEQ. ID NO.:466) | |
| | LLALLSCLTV | HCV MP 63-72 |
| | (SEQ. ID NO.:467) | |
| | ILHTPGCV | HCV MP 105-112 |
| | (SEQ. ID NO.:468) | |
| | QLRRHIDLLV | HCV env E 66-75 |
| | (SEQ, ID NO.:469) | |
| | DLCGSVFLV | HCV env E 88-96 |
| | (SEQ. ID NO.:470) | |
| | SMVGNWAKV | HCV env E 172-180 |
| | (SEQ. ID NO.:471) | |
| | HLHQNIVDV | HCV NSI 308-316 |
| | (SEQ. ID NO.:472) | |
| | FLLLADARV | HCV NSI 340-348 |
| | (SEQ. ID NO.:473) | |
| | GLRDLAVAVEPVV | HCV NS2 234-246 |
| | (SEQ. ID NO.:474) | |
| | SLLAPGAKQNV | HCV NS1 18-28 |
| | (SEQ. ID NO.:475) | |
| | LLAPGAKQNV | HCV NS1 19-28 |
| | (SEQ. ID NO.:476) | |
| | FLLSLGIHL | HBV pol 575-583 |
| | (SEQ. ID NO.:477) | |
| | SLYADSPSV | HBV pol 816-824 |
| | (SEQ. ID NO.:478) | |
| | GLSRYVARL | HBV POL 455-463 |
| | (SEQ. ID NO.:479) | |
| | KIFGSLAFL | HER-2 369-377 |
| | (SEQ. ID NO.:480) | |
| | ELVSEFSRM | HER-2 971-979 |
| | (SEQ. ID NO.:481) | |
| | KLTPLCVTL | HIV- I gp 160 120-128 |
| | (SEQ. ID NO.:482) | |
| | SLLNATDIAV | HIV- I GP 160 814-823 |
| | (SEQ. ID NO.:483) | |
| | VLYRYGSFSV | Pmel gp100 476-485 |
| | (SEQ. ID NO.:484) | |
| | YIGEVLVSV | Non-filament forming class I myosin family (HA-2)** |
| | (SEQ. ID NO.:485) | |
| | LLFNYLGGWV | HCV NS4 192-201 |
| | (SEQ. ID NO.:486) | |
| | LLVPFVQWFW | HBV env 338-347 |
| | (SEQ. ID NO.:487) | |
| | ALMPLYACI | HBV pol 642-650 |
| | (SEQ. ID NO.:488) | |
| | YLVAYQATV | HCV NS3 579-587 |
| | (SEQ. ID NO.:489) | |
| | TLGIVCPIC | HIPV 16 E7 86-94 |
| | (SEQ. ID NO.:490) | |
| | YLLPRRGPRL | HCV core protein 34-43 |
| | (SEQ. ID NO.:491) | |
| | LLPIFFCLWV | HBV env 378-387 |
| | (SEQ. ID NO.:492) | |
| | YMDDVVLGA | HBV Pol 538-546 |
| | (SEQ. ID NO.:493) | |
| | GTLGIVCPI | HPV16 E7 85-93 |
| | (SEQ. 1D NO.:494) | |
| | LLALLSCLTI | HCV MP 63-72 |
| | (SEQ. ID NO.:495) | |
| | MLDLQPETT | HPV 16 E7 12-20 |
| | (SEQ. ID NO.:496) | |
| | SLMAFTAAV | HCV NS4 174-182 |
| | (SEQ. ID NO.:497) | |
| | CINGVCWTV | HCV NS3 67-75 |
| | (SEQ. ID NO.:498) | |
| | VMNILLQYVV | Glutamic acid decarboxylase 114-123 |
| | (SEQ. ID NO.:499) | |
| | ILTVILGVL | Melan A/Mart- 32-40 |
| | (SEQ. ID NO.:500) | |
| | FLWGPRALV | MAGE-3 271-279 |
| | (SEQ. ID NO.:501) | |
| | LLCPAGHAV | HCV NS3 163-171 |
| | (SEQ. ID NO.:502) | |
| | ILDSFDPLV | HCV NSS 239-247 |
| | (SEQ. ID NO.:503) | |
| | LLLCLIFLL | HBV env 250-258 |
| | (SEQ. ID NO.:504) | |
| | LIDYQGMLPV | HBV env 260-269 |
| | (SEQ. ID NO.:505) | |
| | SIVSPFIPLL | HBV env 370-379 |
| | (SEQ. ID NO.:506) | |
| | FLLTRILTI | HBV env 183-191 |
| | (SEQ. ID NO.:507) | |
| | HLGNVKYLV | P. faciparum TRAP 3-11 |
| | (SEQ. ID NO.:508) | |
| | GIAGGLALL | P. faciparum TRAP 500-508 |
| | (SEQ. ID NO.:509) | |
| | ILAGYGAGV | HCV NS S4A 236-244 |
| | (SEQ. ID NO.:510) | |
| | GLQDCTMLV | HCV NS5 714-722 |
| | (SEQ. ID NO.:511) | |
| | TGAPVTYSTY | HCV NS3 281-290 |
| | (SEQ. ID NO.:512) | |
| | VIYQYMDDLV | HIV-1RT 179-187 |
| | (SEQ. ID NO.:513) | |
| | VLPDVFIRCV | N-acetylglucosaminyltransferase V Gnt-V intron |
| | (SEQ. ID NO.:514) | |
| | VLPDVFIRC | N-acetylglucosaminyltransferase V Gnt-V intron |
| | (SEQ. ID NO.:515) | |
| | AVGIGIAVV | Human CD9 |
| | (SEQ. ID NO.:516) | |
| | LVVLGLLAV | Human glutamyltransferase |
| | (SEQ. ID NO.:517) | |
| | ALGLGLLPV | Human G protein coupled receptor |
| | (SEQ. ID NO.:518) | |
| | 164-172 | |
| | GIGIGVLAA | HSV- I gp C 480-488 |
| | (SEQ. ID NO.:519) | |
| | GAGIGVAVL | HSV-2 gp C 446-454 |
| | (SEQ. ID NO.:520) | |
| | IAGIGILAI | Pseudorabies gpGIN 455-463 |
| | (SEQ. ID NO.:521) | |
| | LIVIGILIL | Adenovirus 3 E3 9kD 30-38 |
| | (SEQ. ID NO.:522) | |
| | LAGIGLIAA | S. Lincolnensis ImrA |
| | (SEQ. ID NO.:523) | |
| | VDGIGILTI | Yeast ysa-1 77-85 |
| | (SEQ. ID NO.:524) | |
| | GAGIGVLTA | B. polymyxa, βendoxylanase 149-157 |
| | (SEQ. ID NO.:525) | |
| | 157 | |
| | AAGIGIIQI | E. coli methionine synthase 590-598 |
| | (SEQ. ID NO.:526) | |
| | QAGIGILLA | E. coli hypothetical protein 4-12 |
| | (SEQ. ID NO.:527) | |
| | KARDPHSGHFV | CDK4wl 22.32 |
| | (SEQ. ID NO.:528) | |
| | KACDPI-ISGIIFV | CDK4-R24C 22-32 |
| | (SEQ. ID NO.:529) | |
| | ACDPFISGHFV | CDK4-R24C 23-32 |
| | (SEQ. ID NO.:530) | |
| | SLYNTVATL | HIV- I gag p 17 77-85 |
| | (SEQ. ID NO.:531) | |
| | ELVSEFSRV | HER-2, m>V substituted 971-979 |
| | (SEQ. ID NO.:532) | |
| | RGPGRAFVTI | HIV- I gp 160 315-329 |
| | (SEQ. ID NO.:533) | |
| | HMWNFISGI | HCV NS4A 149-157 |
| | (SEQ. ID NO.:534) | |
| | NLVPMVATVQ | HCMV pp65 495-504 |
| | (SEQ. ID NO.:535) | |
| | GLHCYEQLV | HPV 6b E7 21-30 |
| | (SEQ. ID NO.:536) | |
| | PLKQHFQIV | HPV 6b E7 47-55 |
| | (SEQ. ID NO.:537) | |
| | LLDFVRFMGV | EBNA-6 284-293 |
| | (SEQ. ID NO.:538) | |
| | AIMEKNIML | Influenza Alaska NS 1 122-130 |
| | (SEQ. ID NO.:539) | |
| | YLKTIQNSL | P. falciparum cp36 CSP |
| | (SEQ. ID NO.:540) | |
| | YLNKIQNSL | P. falciparurn cp39 CSP |
| | (SEQ. ID NO.:541) | |
| | YMLDLQPETT | HPV 16 E7 11-20* |
| | (SEQ. ID NO.:542) | |
| | LLMGTLGIV | HPV16 E7 82-90** |
| | (SEQ. ID NO.:543) | |
| | TLGIVCPI | HPV 16 E7 86-93 |
| | (SEQ. ID NO.:544) | |
| | TLTSCNTSV | HIV-1 gp120 197-205 |
| | (SEQ. ID NO.:545) | |
| | KLPQLCTEL | HPV 16 E6 18-26 |
| | (SEQ. ID NO.:546) | |
| | TIHDIILEC | HPV16 E6 29-37 |
| | (SEQ. ID NO.:547) | |
| | LGIVCPICS | HPV 16 E7 87-95 |
| | (SEQ. ID NO.:548) | |
| | VILGVLLLI | Melan A/Mart-1 35-43 |
| | (SEQ. ID NO.:549) | |
| | ALMDKSLHV | Melan A/Mart- 1 56-64 |
| | (SEQ. ID NO.:550) | |
| | GILTVILGV | Melan A/Mart- 1 31-39 |
| | (SEQ. ID NO.:551) | |
| T cell epitopes | MINAYLDKL | P. Falciparum STARP 523-531 |
| | (SEQ. ID NO.:552) | |
| | AAGIGILTV | Melan A/Mart- 127-35 |
| | (SEQ. ID NO.:553) | |
| | FLPSDFFPSV | HBV cAg 18-27 |
| | (SEQ. ID NO.:554) | |
| Motif unknown | SVRDRLARL | EBNA-3 464-472 |
| T cell epitopes | (SEQ. ID NO.:555) | |
| T cell epitopes | AAGIGILTV | Melan A/Mart-1 27-35 |
| | (SEQ. ID NO.:556) | |
| | FAYDGKDYI | Human MHC I-ot 140-148 |
| | (SEQ. ID NO.:557) | |
| T cell epitopes | AAGIGILTV | Melan A/Mart-1 27-35 |
| | (SEQ. ID NO.:558) | |
| | FLPSDFFPSV | HBV cAg 18-27 |
| | (SEQ. ID NO.:559) | |
| Motif unknown | AAGIGILTV | Meland A/Mart- 27-35 |
| T cell epitopes | (SEQ. ID NO.:560) | |
| | FLPSDFFPSV | HBV cAg 18-27 |
| | (SEQ. ID NO.:561) | |
| | AAGIGILTV | Melan A/Mart-1 27-35 |
| | (SEQ. ID NO.:562) | |
| | ALLAVGATK | Pmel17 gp 100 17-25 |
| | (SEQ. ID NO.:563) | |
| T cell epitopes | R L R D L L L I V T R | HIV-1 gp41 768-778 |
| | (SEQ. ID NO.:564) | |
| | QVPLRPMTYK | HIV-1 nef 73-82 |
| | (SEQ. ID NO.:565) | |
| | TVYYGVPVWK | HIV-1 gp120-36-45 |
| | (SEQ. ID NO.:566) | |
| | RLRPGGKKK | HIV-1 gag p 17 20-29 |
| | (SEQ. ID NO.:567) | |
| | ILRGSVAHK | Influenza NP 265-273 |
| | (SEQ. ID NO.:568) | |
| | RLRAEAGVK | EBNA-3 603-611 |
| | (SEQ. ID NO.:569) | |
| | RLRDLLLIVTR | HIV-1 gp41 770-780 |
| | (SEQ. ID NO.:570) | |
| | VYYGVPVWK | HIV- I GP 120 38-46 |
| | (SEQ. ID NO.:571) | |
| | RVCEKMALY | HCV NS5 575-583 |
| | (SEQ. ID NO.:572) | |
| Motif unknown | KIFSEVTLK | Unknown; muta melanoma peptide ted (p I 83L) 175-183 |
| T cell epitope | (SEQ. ID NO.:573) | |
| | YVNVNMGLK* | HBV cAg 88-96 |
| | (SEQ. ID NO.:574) | |
| T cell epitopes | IVTDFSVIK | EBNA-4 416-424 |
| | (SEQ. ID NO.:575) | |
| | ELNEALELK | P53 343-351 |
| | (SEQ. ID NO.:576) | |
| | VPLRPMTYK | HIV-1 NEF 74-82 |
| | (SEQ. ID NO.:577) | |
| | AIFQSSMTK | HIV- I gag p24 325-333 |
| | (SEQ. ID NO.:578) | |
| | QVPLRPMTYK | HIV-1 nef 73-82 |
| | (SEQ. ID NO.:579) | |
| | TINYTIFK HCV | NSI 238-246 |
| | (SEQ. ID NO.:580) | |
| | AAVDLSHFLKEK | HIV-1 nef 83-94 |
| | (SEQ. ID NO.:581) | |
| | ACQ G V G G P G G H K | HIV-1 II 1B p24 349-359 |
| | (SEQ. ID NO.:581) | |
| HLA-A24 | S Y L D S G I H F* | β-catenin, mutated (proto-onocogen) 29-37 |
| | (SEQ. ID NO.:582) | |
| T cell epitopes | RYLKDQQLL | HIV GP 41 583-591 |
| | (SEQ. ID NO.:583) | |
| | AYGLDFYIL | P15 melanoma Ag 10-18 |
| | (SEQ. ID NO.:584) | |
| | AFLPWHRLFL | Tyrosinase 206-215 |
| | (SEQ. ID NO.:585) | |
| | AFLPWHRLF | Tyrosinase 206-214 |
| | (SEQ. ID NO.:586) | |
| | RYSIFFDY | Ebna-3 246-253 |
| | (SEQ. ID NO.:587) | |
| T cell epitope | ETINEEAAEW | HIV-1 gag p24 203-212 |
| | (SEQ. ID NO.:588) | |
| T cell epitopes | STLPETTVVRR | HBV cAg 141-151 |
| | (SEQ. ID NO.:589) | |
| | MSLQRQFLR | ORF 3P-gp75 294-321 (bp) |
| | (SEQ. ID NO.:590) | |
| | LLPGGRPYR | TRP (tyrosinase rel.) 197-205 |
| | (SEQ. ID NO.:591) | |
| T cell epitope | IVGLNKIVR | HIV gag p24 267-267-275 |
| | (SEQ. ID NO.:592) | |
| | AAGIGILTV | Melan A/Mart- 127 35 |
| | (SEQ. ID NO.:593) | |

Table 3 sets forth additional antigens useful in the invention that are available from the Ludwig Cancer Institute. The Table refers to patents in which the identified antigens can be found. TRA refers to the tumor-related antigen and the LUD No. refers to the Ludwig Institute number.

**Table 3**

| **TRA** | **LUD No.** | **Patent No.** | **Date Patent Issued** | **Peptide (Antigen)** | **HLA** |
|---|---|---|---|---|---|
| MAGE-4 | 5293 | 5,405,940 | 11 April 1995 | EVDPASNTY | HLA-A1 |
| | | | | (SEQ. ID NO.:532) | |
| MAGE-41 | 5293 | 5,405,940 | 11 April 1995 | EVDPTSNTY | HLA-A1 |
| | | | | (SEQ ID NO:533) | |
| MAGE-5 | 5293 | 5,405,940 | 11 April 1995 | EADPTSNTY | HLA-A1 |
| | | | | (SEQ ID NO:534) | |
| MAGE-51 | 5293 | 5,405,940 | 11 April 1995 | EADPTSNTY | HLA-A1 |
| | | | | (SEQ ID NO:534) | |
| MAGE-6 | 5294 | 5,405,940 | 11 April 1995 | EVDPIGHVY | HLA-A1 |
| | | | | (SEQ ID NO:535) | |
| | 5299.2 | 5,487,974 | 30 January 1996 | MLLAVLYCLL | HLA-A2 |
| | | | | (SEQ ID NO:536) | |
| | 5360 | 5,530,096 | 25 June 1996 | MLLAVLYCL | HLA-844 |
| | | | | (SEQ ID NO:537) | |
| Tyrosinase | 5360.1 | 5,519,117 | 21 May 1996 | SEIWRDIDFA | HLA-B44 |
| | | | | (SEQ ID NO:538) | |
| | | | | SEIWRDIDF | |
| | | | | (SEQ ID NO:539) | |
| Tyrosinase | 5431 | 5,774,316 | 28 April 1998 | XEIWRDIDF | HLA-B44 |
| | | | | (SEQ ID NO:540) | |
| MAGE-2 | 5340 | 5,554,724 | 10 September 1996 | STLVEVTLGEV | HLA-A2 |
| | | | | (SEQ ID NO:541) | |
| | | | | LVEVTLGEV | |
| | | | | (SEQ ID NO:542) | |
| | | | | VIFSKASEYL | |
| | | | | (SEQ ID NO:543) | |
| | | | | IIVLAIIAI | |
| | | | | (SEQ ID NO:544) | |
| (Continued) | | | | KIWEELSMLEV | |
| | | | | (SEQ ID NO:545) | |
| | | | | LIETSYVKV | |
| | | | | (SEQ ID NO:546) | |
| | 5327 | 5,585,461 | 17 December 1996 | FLWGPRALV | HLA-A2 |
| | | | | (SEQ ID NO: 547) | |
| | | | | TLVEVTLGEV | |
| | | | | (SEQ ID NO:548) | |
| | | | | ALVETSYVKV | |
| | | | | (SEQ ID NO:549) | |
| MAGE-3 | 5344 | 5,554,506 | 10 September 1996 | KIWEELSVL | HLA-A2 |
| | | | | (SEQ ID NO:550) | |
| MAGE-3 | 5393 | 5,405,940 | 11 April 1995 | EVDPIGHLY | HLA-A1 |
| | | | | (SEQ ID NO:551) | |
| MAGE | 5293 | 5,405,940 | 11 April 1995 | EXDX5Y | HLA-A1 |
| | | | | (SEQ. ID NO.:552) | |
| | | | | (but not EADPTGHSY) | |
| | | | | (SEQ. ID NO.:553) | |
| | | | | E (A/V) D X5 Y | |
| | | | | (SEQ. ID NO.:554) | |
| | | | | E (A/V) D P X4 Y | |
| | | | | (SEQ. ID NO.:555) | |
| | | | | E (A/V) D P (1/A/T) X3 Y | |
| | | | | (SEQ. ID NO.:556) | |
| | | | | E (A/V) D P (I/A/T) (G/S) X2 Y | |
| | | | | (SEQ. ID NO.:557) | |
| | | | | E (A/V) D P (I/A/T) (G/S) (H/N) X Y | |
| | | | | E (A/V) DP (I/A/T) (G/S) (H/N) | |
| | | | | (L/T/V) Y | |
| | | | | (SEQ. II NO.:559) | |
| MAGE-1 | 5361 | 5,558.995 | 24 September 1996 | ELHSAYGEPRKLLTQD | HLA-C |
| | | | | (SEQ ID NO:560) | Clone 10 |
| | | | | EHSAYGEPRKLL | |
| | | | | (SEQ ID NO:561) | |
| | | | | SAYGEPRKL | |
| | | | | (SEQ ID NO:562) | |
| MAGE-1 | 5253.4 | TBA | TBA | EADPTGHSY | HLA-A1 |
| | | | | (SEQ ID NO:563) | |
| BAGE | 5310.1 | TBA | TBA | MAARAVFLALSAQLLQARLMKE | HLA-C |
| | | | | (SEQ ID NO:564) | Clone 10 |
| | | | | MAARAVFLALSAQLLQ | HLA-C |
| | | | | (SEQ ID NO:565) | Clone 10 |
| | | | | AARAVFLAL | HLA-C |
| | | | | (SEQ ID NO:566) | Clone 10 |
| GAGE | 5323.2 | 5,648,226 | 15 July 1997 | YRPRPRRY | HLA-CW6 |
| | | | | (SEQ. ID NO.:567) | |

**Table 4**

| **Source** | **Protein** | **AA Position** | **MHC molecules** | **T cell epitope MHC ligand (Antigen)** | **Ref.** |
|---|---|---|---|---|---|
| synthetic peptides | synthetic peptides | synthetic peptides | HLA-A2 ranking | ALFAAAAAV | Parker, et al., "Scheme for potential HLA-A2 binding peptides based on independent binding of individual peptide side-chains," J. Immunol. 152:163-175 |
| | | | " | GIFGGVGGV | " |
| | | | " | GLDKGGGV | " |
| | | | " | GLFGGFGGV | " |
| | | | " | GLFGGGAGV | " |
| | | | " | GLFGGGEGV | " |
| | | | " | GLFGGGFGV | " |
| | | | " | GLFGGGGGL | " |
| | | | " | GLFGGGGGV | " |
| | | | " | GLFGGGVGV | " |
| | | | " | GLFGGVGGV | " |
| | | | " | GLFGGVGKV | " |
| | | | " | GLFKGVGGV | " |
| | | | " | GLGGGGFGV | " |
| | | | " | GLLGGGVGV | " |
| | | | " | GLYGGGGGV | " |
| | | | " | GMFGGGGGV | " |
| | | | " | GMFGGVGGV | " |
| | | | " | GQFGGVGGV | " |
| | | | " | GVFGGVGGV | " |
| | | | " | KLFGGGGGV | " |
| | | | " | KLFGGVGGV | " |
| | | | " | AILGFVFTL | " |
| | | | " | GAIGFVFTL | " |
| | | | " | GALGFVFTL | " |
| | | | " | GELGFVFTL | " |
| | | | " | GIAGFVFTL | " |
| | | | " | GIEGFVFTL | " |
| | | | " | GILAFVFTL | " |
| | | | " | GILGAVFTL | " |
| | | | " | GILGEVFTL | " |
| | | | " | GILFGAFTL | " |
| | | | " | GILGFEFTL | " |
| | | | " | GILGFKFTL | " |
| | | | " | GILGFVATL | " |
| | | | " | GILGFVETL | " |
| | | | " | GILGFVFAL | " |
| | | | " | GILGFVFEL | " |
| | | | " | GILGFVFKL | " |
| | | | " | GILGFVFTA | " |
| | | | " | GILGFVFTL | " |
| | | | " | GILGFVFVL | " |
| | | | " | GILGFVKTL | " |
| | | | " | GILGKVFTL | " |
| | | | " | GILKFVFTL | " |
| | | | " | GILPFVFTL | " |
| | | | " | GIVGFVFTL | " |
| | | | " | GKLGFVFTL | " |
| | | | " | GLLGFVFTL | " |
| | | | " | GQLGFVFTL | " |
| | | | " | KALGFVFTL | " |
| | | | " | KILGFVFTL | " |
| | | | " | KILGKVFTL | " |
| | | | " | AILLGVFML | " |
| | | | " | AIYKRWIIL | " |
| | | | " | ALFFFDIDL | " |
| | | | " | ATVELLSEL | " |
| | | | " | CLFGYPVYV | " |
| | | | " | FIFPNYTIV | " |
| | | | " | IISLWDSQL | " |
| | | | " | ILASLFAAV | " |
| | | | " | ILESLFAAV | " |
| | | | " | KLGEFFNQM | " |
| | | | " | KLGEFYNQM | " |
| | | | " | LLFGYPVYV | " |
| | | | " | LLWKGEGAV | " |
| | | | " | LMFGYPVYV | " |
| | | | " | LNFGYPVYV | " |
| | | | " | LQFGYPVYV | " |
| | | | " | NIVAHTFKV | " |
| | | | " | NLPMVATV | " |
| | | | " | QMLLAIARL | " |
| | | | " | QMWQARLTV | " |
| | | | " | RLLQTGIHV | " |
| | | | " | RLVNGSLAL | " |
| | | | " | SLYNTVATL | " |
| | | | " | TLNAWVKVV | " |
| | | | " | WLYRETCNL | " |
| | | | " | YLFKRMIDL | " |
| | | | " | GAFGGVGGV | " |
| | | | " | GAFGGVGGY | " |
| | | | " | GEFGGVGGV | " |
| | | | " | GGFGGVGGV | " |
| | | | " | GIFGGGGGV | " |
| | | | " | GIGGFGGGL | " |
| | | | " | GIGGGGGGL | " |
| | | | " | GLDGGGGGV | " |
| | | | " | GLDGKGGGV | " |
| | | | " | GLDKKGGGV | " |
| | | | " | GLFGGGFGF | " |
| | | | " | GLFGGGFGG | " |
| | | | " | GLFGGGFGN | " |
| | | | " | GLFGGGFGS | " |
| | | | " | GLFGGGGGI | " |
| | | | " | GLFGGGGGM | " |
| | | | " | GLFGGGGGT | " |
| | | | " | GLFGGGGGY | " |
| | | | " | GLGFGGGGV | " |
| | | | " | GLGGFGGGV | " |
| | | | " | GLGGGFGGV | " |
| | | | " | GLGGGGGFV | " |
| | | | " | GLGGGGGGY | " |
| | | | " | GLGGGVGGV | " |
| | | | " | GLLGGGGGV | " |
| | | | " | GLPGGGGGV | " |
| | | | " | GNFGGVGGV | " |
| | | | " | GSFGGVGGV | " |
| | | | " | GTFGGVGGV | " |
| | | | " | AGNSAYEYV | " |
| | | | " | GLFPGQFAY | " |
| | | | " | HILLGVFML | " |
| | | | " | ILESLFRAV | " |
| | | | " | KKKYKLKHI | " |
| | | | " | MLASIDLKY | " |
| | | | " | MLERELVRK | " |
| | | | " | KLFGFVFTV | " |
| | | | " | ILDKKVEKV | " |
| | | | " | ILKEPVHGV | " |
| | | | " | ALFAAAAAY | " |
| | | | " | GIGFGGGGL | " |
| | | | " | GKFGGVGGV | " |
| | | | " | GLFGGGGGK | " |
| | | | " | EILGFVFTL | " |
| | | | " | GIKGFVFTL | " |
| | | | " | GQLGFVFTK | " |
| | | | " | ILGFVFTLT | " |
| | | | " | KILGFVFTK | " |
| | | | " | KKLGFVFTL | " |
| | | | " | KLFEKVYNY | " |
| | | | " | LRFGYPVYV | " |
| Human | HSP60 | 140-148 | HLA-B27 | IRRGVMLAV | Rammensee et al. 1997 |
| | | | | | 160 |
| " | " | 369-377 | " | KRIQEIIEQ | " |
| " | " | 469-477 | " | KRTLKIPAM | " |
| Yersinia | HSP60 | 35-43 | " | GRNVVLDKS | " |
| " | " | 117-125 | " | KRGIDKAVI | " |
| " | " | 420-428 | " | IRAASAITA | " |
| " | HSP 60 | 284-292 | HLA-B*2705 | RRKAMFEDI | 169 |
| P.falciparum | LSA-1 | 1850-1857 | HLA-B3501 | KPKDELDY | 170 |
| Influenza NP | | 379-387 | HLA-B*4402 | LELRSRYWA | 183 |
| | Tum-P35B | 4-13 | HLA-D^{d} | GPPHSNNFGY | 230 |
| Rotavirus | VP7 | 33-40 | | IIYRFLLI | 262 |
| | OGDH (F108Y) | 104-112 | H2-L^{d} | QLSPYPFDL | 253 |
| | TRP-2 | 181-188 | p287 | VYDFFVWL | 284 |
| | DEAD box p 68 | 547-554 | p287 | SNFVFAGI | 283 |
| | Vector "artefact" | | p287 | SVVEFSSL | 260 |
| | Epiope mimic of tumor Ag | | p287 | AHYLFRNL | 278 |
| | | | " | THYLFRNL | " |
| | Epitope mimic of H-3 miHAg" | | " | LIVIYNTL | 279 |
| | | | " | LIYEFNTL | " |
| | | | " | IPYTYNTL | " |
| | | | " | IIYIYHRL | " |
| | | | " | LIYIFNTL | " |
| | HBV cAg | 93-100 | " | MGLKFRQL | 280 |
| Human | autoantigen LA | 51-58 | " | IMIKFRNRL | 281 |
| Mouse | UTY protein | | H2D^{b} | WMHHNMDLI | 303 |
| Mouse | p53 | 232-240 | " | KYMCNSSCM | 302 |
| MURINE | MDM2 | 441-449 | " | GRPKNGCIV | 277 |
| | Epitope mimic of natural | | " | AQHPNAELL | 278 |
| | MuLV gag75K | 75-83 | " | CCLCLTVFL | 301 |
| P. Falciparum | CSP | 375-383 | p290 | YENDIEKK | 315 |
| " | " | 371-379 | " | DELDYENDI | 315 |
| HIV | -1RT | 206-214 | " | TEMEKEGKI | 316 |
| Rabies | NS | 197-205 | | VEAEIAHQI | 309,310 |
| Influenza A NS1 | | 152-160 | " | EEGAIGEI | 304 |
| Murine | SMCY | | p291 | TENSGKDI | 317 |
| | MHC class 1 leader | 3-11 | p293 | AMAPRTLLL | 318 |
| | ND1 alpha | 1-12 | p293 | FFINILTLLVP | 323 |
| | ND Beta | 1-12 | p293 | FFINILTLLVP | 323 |
| | ND alpha | 1-17 | " | FFINILTLLVPILIAM | 324 |
| | ND Beta | 1-17 | " | FFINALTLLVPILIAM | " |
| | COI mitochondri al | 1-6 | " | FINRW | 325 |
| L. monocytoge nes | LemA | 1-6 | " | IGWII | 326 |
| | SIV gag p11C | 179-190 | Mamu-A*01 | EGCTPYDINQML | 334 |
| | | | | | |
| | MAGE-3 | | HLA-A2 | ALSRKVAEL | 5,554,506 |
| | | | " | IMPKAGLLI | " |
| | | | " | KIWEELSVL | " |
| | | | " | ALVETSYVKV | " |
| | | | " | ThrLeuValGluValThrL euGlyGluVal | " |
| | | | " | AlaLeuSerArgLysValA laGluLeu | " |
| | | | " | IleMetProLysAlaGlyLe uLeuIle | " |
| | | | " | LysIleTrpGluGluLeuSe rValLeu | " |
| | | | " | AlaLeuValGluThrSerT yrValLysVal | " |
| | | | | | |
| | peptides which bind to MHCs | | HLA-A2 | Lys Gly Ile Leu Gly Phe Val Phe Thr Leu Thr Val | 5,989,565 |
| | | | " | Gly Ile Ile Gly Phe Val Phe Thr Ile | " |
| | | | " | Gly Ile Ile Gly Phe Val Phe Thr Leu | " |
| | | | " | Gly Ile Leu Gly Phe Val Phe Thr Leu | " |
| | | | " | Gly Leu Leu Gly Phe Val Phe Thr Leu | " |
| | | | " | XXTVXXGVX, X=Leu or Ile (6-37) | " |
| | | | " | Ile Leu Thr Val Ile Leu Gly Val Leu | " |
| | | | " | Tyr Leu Glu Pro Gly Pro Val Thr Ala | " |
| | | | " | Gln Val Pro Leu Arg Pro Met Thr Tyr Lys | " |
| | | | " | Asp Gly Leu Ala Pro Pro Gln His Leu Ile Arg | " |
| | | | " | Leu Leu Gly Arg Asn Ser Phe Glu Val | " |
| | | | | | |
| | Peptides from MAGE-1 | | HLA-C clone 10 | GluHisSerAlaTyrGlyG luProArgLysLeuLeuTh rGlnAspLeu | 5,558,995 |
| | | | " | GluHisSerAlaTyrGlyG luProArgLysLeuLeu | " |
| | | | " | SerAlaTyrGlyGluProA rgLysLeu | " |
| | | | | | |
| | GAGE | | HLA-Cw6 | TyrArgProArgProArgA rgTyr | 5,648,226 |
| | | | " | ThrTyrArgProArgProA rgArgTyr | " |
| | | | " | TyrArgProArgProArgA rgTyrVal | " |
| | | | " | ThrTyrArgProArgProA rgArgTyrVal | " |
| | | | " | ArgProArgProArgArgT yrValGlu | " |
| | | | " | MetSerTrpArgGlyArgS erThrTyrArgProArgPro ArgArg | " |
| | | | " | ThrTyrArgProArgProA rgArgTyrValGluProPro GluMetIle | " |
| | MAGE | | HLA-A1, primarily | Isolated nonapeptide having Glu at its N terminal, Tyr at its C-terminal, and Asp at the third residue from its N terminal, with the proviso that said isolated nonapeptide is not Glu Ala Asp Pro Thr Gly His Ser Tyr (SEQ ID NO: 1), and wherein said isolated nonapeptide binds to a human leukocyte antigen molecule on a cell to form a complex, said complex provoking lysis of said cell by a cytolytic T cell specific to said complex | 5,405,940 |
| | | | " | GluValValProIleSerHis LeuTyr | " |
| | | | " | GluValValArgIleGlyHi sLeuTyr | " |
| | | | " | GluValAspProIleGlyHi sLeuTyr | " |
| | | | " | GluValAspProAlaSerA snThrTyr | " |
| | | | " | GluValAspProThrSerA snThrTyr | " |
| | | | " | GluAlaAspProThrSerA snThrTyr | " |
| | | | " | GluValAspProIleGlyHi sValTyr | " |
| | | | " | GAAGTGGTCCCCAT CAGCCACTTGTAC | " |
| | | | " | GAAGTGGTCCGCA TCGGCCACTTGTAC | " |
| | | | " | GAAGTGGACCCCA TCGGCCACTTGTAC | " |
| | | | " | GAAGTGGACCCCG CCAGCAACACCTAC | " |
| | | | " | GAAGTGGACCCCA CCAGCAACACCTAC | " |
| | | | " | GAAGCGGACCCCA CCAGCAACACCTAC | " |
| | | | " | GAAGCGGACCCCA CCAGCAACACCTAC | " |
| | | | " | GAAGTGGACCCCA TCGGCCACGTGTAC | " |
| | | | " | GluAlaAspProThrGly HisSer | " |
| | | | " | AlaAspProTrpGlyHisS erTyr | " |
| | MAGE peptides | | HLA-A2 | SerThrLeuValGluValT hrLeuGlyGluVal | 5,554,724 |
| | " | | " | LeuValGluValThrLeu GlyGluVal | " |
| | " | | " | LysMetValGluLeuVal HisPheLeu | " |
| | " | | " | ValIlePheSerLysAlaSe rGluTyrLeu | " |
| | " | | " | TyrLeuGlnLeuValPhe GlyIleGluVal | " |
| | " | | " | GlnLeuValPheGlyIleGl uValVal | " |
| | " | | " | GlnLeuValPheGlyIleGl uValValGluVal | " |
| | " | | " | IleIleValLeuAlaIleIleA laIle | " |
| | " | | " | LysIleTrpGluGluLeuSe rMetLeuGluVal | " |
| | " | | " | AlaLeuIleGluThrSerTy rValLysVal | " |
| | " | | " | LeuIleGluThrSerTyrVa ILysVal | " |
| | " | | " | GlyLeuGluAlaArgGly GluAlaLeuGlyLeu | " |
| | " | | " | GlyLeuGluAlaArgGly GluAlaLeu | " |
| | " | | " | AlaLeuGlyLeuValGly AlaGlnAla | " |
| | " | | " | GlyLeuValGlyAlaGln AlaProAla | " |
| | " | | " | AspLeuGluSerGluPhe GlnAlaAla | " |
| | " | | " | AspLeuGluSerGluPhe GlnAlaAlaIle | " |
| | " | | " | AlalleSerArgLysMetV alGluLeuVal | " |
| | " | | " | AlalleSerArgLysMetV alGluLeu | " |
| | " | | " | LysMetValGluLeuVal HisPheLeuLeu | " |
| | " | | " | LysMetValGluLeuVal HisPheLeuLeuLeu | " |
| | " | | " | LeuLeuLeuLysTyrArg AlaArgGluProVal | " |
| | " | | " | LeuLeuLysTyrArgAla ArgGluProVal | " |
| | " | | " | ValLeuArgAsnCysGln AspPhePheProVal | " |
| | " | | " | TyrLeuGlnLeuValPhe GlyIleGluValVal | " |
| | " | | " | GlyIleGluValValGluV alValProIle | " |
| | " | | " | ProIleSerHisLeuTyrlle LeuVal | " |
| | " | | " | HisLeuTyrIleLeuValTh rCysLeu | " |
| | " | | " | HisLeuTyrIleLeuValTh rCysLeuGlyLeu | " |
| | " | | " | TyrIleLeuValThrCysLe uGlyLeu | " |
| | " | | " | CysLeuGlyLeuSerTyr AspGlyLeu | " |
| | " | | " | CysLeuGlyLeuSerTyr AspGlyLeuLeu | " |
| | " | | " | ValMetProLysThrGlyL euLeuIle | " |
| | " | | " | ValMetProLysThrGlyL euLeuIleIle | " |
| | " | | " | ValMetProLysThrGlyL euLeuIlelleVal | " |
| | " | | " | GlyLeuLeuIleIleValLe uAlaIle | " |
| | " | | " | GlyLeuLeuIleIleValLe uAlaIleIle | " |
| | " | | " | GlyLeuLeuIleIleValLe uAlaIlelleAla | " |
| | " | | " | LeuLeuIleIleVaILeuAl aIleIle | " |
| | " | | " | LeuLeuIleIleValLeuAl aIleIleAla | " |
| | " | | " | LeuLeuIleIlevalLeuAl aIleIleAlaIle | " |
| | " | | " | LeuIleIleValLeuAlaIleI leAla | " |
| | " | | " | LeuIleIleValLeuAlaIleI leAlaIle | " |
| | " | | " | IleIleAlaIleGluGlyAsp CysAla | " |
| | " | | " | LysIleTrpGluGluLeuSe rMetLeu | " |
| | " | | " | LeuMetGlnAspLeuVal GlnGluAsnTyrLeu | " |
| | " | | " | PheLeuTrpGlyProArg AlaLeuIle | " |
| | " | | " | LeuIleGluThrSerTyrVa ILysVal | " |
| | " | | " | AlaLeuIleGluThrSerTy rValLysValLeu | " |
| | " | | " | ThrLeuLysIleGlyGlyGl uProHisIle | " |
| | " | | " | HisIleSerTyrProProLeu HisGluArgAla | " |
| | " | | " | GlnThrAlaSerSerSerSe rThrLeu | " |
| | " | | " | GlnThrAlaSerSerSerSe rThrLeuVal | " |
| | " | | " | ValThrLeuGlyGluValP roAlaAla | " |
| | " | | " | ValThrLysAlaGluMetL euGluSerVal | " |
| | " | | " | ValThrLysAlaGluMetL euGluSerValLeu | " |
| | " | | " | ValThrCysLeuGlyLeu SerTyrAspGlyLeu | " |
| | " | | " | LysThrGlyLeuLeuIleIl eValLeu | " |
| | " | | " | LysThrGlyLeuLeuIleIl eValLeuAla | " |
| | " | | " | LysThrGlyLeuLeuIleIl cValLcuAlaIle | " |
| | " | | " | HisThrLeuLysIleGlyGl yGluProHisIle | " |
| | " | | " | MetLeuAspLeuGlnPro GluThrThr | " |
| | | | | | |
| | Mage-3 peptides | | HLA-A2 | GlyLeuGluAlaArgGly GluAlaLeu | 5,585,461 |
| | " | | " | AlaLeuSerArgLysValA laGluLeu | " |
| | " | | " | PheLeuTrpGlyProArg AlaLeuVal | " |
| | " | | " | ThrLeuValGluValThrL euGlyGluVal | " |
| | " | | " | AlaLeuSerArgLysValA laGluLeuVal | " |
| | " | | " | AlaLeuValGluThrSerT yrValLysVal | " |
| | | | | | |
| | Tyrosinase | | HLA-A2 | TyrMetAsnGlyThrMet SerGlnVal | 5,487,974 |
| | " | | " | MetLeuLeuAlaValLeu TyrCysLeuLeu | " |
| | | | | | |
| | Tyrosinase | | HLA-A2 | MetLeuLeuAlaValLeu TyrCysLeu | 5,530,096 |
| | " | | " | LeuLeuAlaValLeuTyr CysLeuLeu | " |
| | Tyrosinase | | HLA-A2 and HLA-B44 | SerGluIleTrpArgAspIle AspPheAlaHisGluAla | 5,519,117 |
| | " | | " | SerGluIleTrpArgAspIle AspPhe | " |
| | " | | " | GluGluAsnLeuLeuAsp PheValArgPhe | " |
| | Melan | | | EAAGIGILTV | Jäger, E. et al. |
| | A/MART-1 | | | | Granulocyte-macrophage-colony-stimulating Factor Enhances Immune Responses To Melanoma-'associated Peptides in vivo Int. J Cancer 67, 54-62 (1996) |
| | Tyrosinase | | | MLLAVLYCL | " |
| | " | | | YMDGTMSQV | " |
| | gp 100/Pmel 17 | | | YLEPGPVTA | " |
| | " | | | LLDGTATLRL | " |
| | Influenza matrix | | | GILGFVFTL | " |
| | MAGE-1 | | | EADPTGHSY | " |
| | | | | | |
| | MAGE-1 | | HLA-A1 | EADPTGHSY | DIRECTLY FROM DAVID'S LIST |
| | BAGE | | HLA-C | MAARAVFLALSAQL LQARLMKE | " |
| | " | | " | MAARAVFLALSAQL LQ | " |
| | " | | " | AARAVFLAL | " |
| Influenza | PR8 NP | 147-154 | K^{d} | IYQRIRALV | Falk et al., Allele-specific motifs revealed by sequencing of self-peptides eluted from MHC molecules |
| SELF PEPTIDE | P815 | | " | SYFPEITHI | " |
| Influenza | Jap HA 523-549 | | " | " IYATVAGSL | " |
| " | " | | " | VYQILAIYA | " |
| " | " | | " | IYSTVASSL | " |
| " | JAP HA 202-221 | | " | LYQNVGTYV | " |
| | HLA-A24 | | " | RYLENQKRT | " |
| | HLA-Cw3 | | " | RYLKNGKET | " |
| | P815 | | " | KYQAVTTTL | " |
| Plasmodium berghen | CSP | | " | SYIPSAEKI | " |
| Plasmodium yoelli | CSP | | " | SYVPSAFQI | " |
| Vesicular stomatitis viruse | NP 52-59 | | K^{b} | RGYVYQGL | " |
| Ovalbumin | | | " | SIINFEKL | " |
| Sandal Virus | NP 321-332 | | " | APGNYPAL | " |
| | | | | VPYGSFKHV | Morel et al., Processing of some antigens by the standard proteasome but not by the immunoproteaso me results in poor presentation by dendritic cells, Immunity, vol. 12:107-117, 2000. |

| | | | **MOTIFS** | | |
|---|---|---|---|---|---|
| influenza | PR8 NP | | K^{d} restricted peptide motif | TYQRTRALV | 5,747,269 |
| self peptide | P815 | | " | SYFPEITHI | " |
| influenza | JAP HA | | " | IYATVAGSL | " |
| influenza | JAP HA | | " | VYQILAIYA | " |
| influenza | PR8 HA | | " | IYSTVASSL | " |
| influenza | JAP HA | | " | LYQNVGTYV | " |
| | | | HLA-A24 | RYLENGKETL | " |
| | | | HLA-Cw3 | RYLKNGKETL | " |
| | P815 tumour antigen | | " | KYQAVTTTL | " |
| Plasmodium berghei | CSP | | " | SYIPSAEKI | " |
| Plasmodium yoeli | CSP | | " | SYVPSAEQI | " |
| influenza | NP | | D^{b} - restricted peptide motif | ASNENMETM | " |
| adenovirus | E1A | | " | SGPSNTPPEI | " |
| lymphocytic choriomenin gitis | | | " | SGVENPGGYCL | " |
| simian virus | 40 T | | " | SAINNY ... | " |
| HIV | reverse transcriptase | | HLA-A2.1-restricted peptide motif | ILKEPVHGV | " |
| | influenza matrix protein | | " | GILGFVFTL | " |
| influenza | influenza matrix protein | | " | ILGFVFTLTV | " |
| HIV | Gag protein | | | FLQSRPEPT | " |
| HIV | Gag protein | | | AMQMLKE .. | " |
| HIV | Gag protein | | | PIAPGQMRE | " |
| HIV | Gag protein | | | QMKDCTERQ | " |
| | | | HLA-A*0205-restricted peptide motif | VYGVIQK | " |

**Table 5**

| |
|---|
| VSV-NP peptide (49-62) |
| LCMV-NP peptide (118-132) |
| LCMV glycoprotein peptide. 3341 |
| ISNQLTLDSNTKYFHKLN |
| ISNQLTLDSNTKYFHKL |
| ISNQLTLDSNTKYFHK |
| VDTFLEDVKNLYHSEA |
| KPRAIVVDPVHGFMY |
| KQTISPDYRNMI |
| YIDFIMDPKEKDKV |
| NIQLINDQEVARFD |
| LLSFVRDLNQYRADI |
| LPKPPKPVSKMRMATPL |
| LPKPPKPVSKMRMATPLLMQALP |
| LPKPPKPVSKMRMATPLLMQALPM |
| PKPPKPVSKMRMATPL |
| PKPPKPVSKMRMATPLLMQA |
| KPPKPVSKMRMATPLLMQ |
| KPPKPVSKMRMATPLLMQALPM |
| VDDTQFVRFDSDAASQ |
| ATKYGNMTEDHVMHLLQNA |
| VFLLLLADKVPETSLS |
| LNKILLDEQAQWK |
| GPPKLDIRKEEKQIMIDIFH |
| GPPKLDIRKEEKQIMIDIFHP |
| GFKAIRPDKKSNPIIRTV |
| YANILLDRRVPQTDMTF |
| NLFLKSDGRIKYTLNKNSLK |
| IPDNLFLKSDGRIKYTLNKN |
| IPDNLFLKSDGRIKYTLNK |
| IPDNLFLKSDGRIKYTLN |
| IPDNLFLKSDGRIKYTL |
| NLFLKSDGRIKYTLNK |
| NLFLKSDGRIKYTLN |
| VTTLNSDLKYNALDLTN |
| VGSDWRFLRGYHQYA |

Also described herein are methods, uses, therapies and compositions related to epitopes with specificity for MHC, including, for example, those listed in Tables 6-10. Also described are of the MHCs listed in Tables 6-10, including combinations of the same, while other embodiments specifically exclude any one or more of the MHCs or combinations thereof. Tables 8-10 include frequencies for the listed HLA antigens.

**Table 6**

| Class I MHC Molecules |
|---|
| **Class I** |
| **Human** |
| HLA-A1 |
| HLA-A*0101 |
| HLA-A*0201 |
| HLA-A*0202 |
| HLA-A*0203 |
| HLA-A*0204 |
| HLA-A*0205 |
| HLA-A*0206 |
| HLA-A*0207 |
| HLA-A*0209 |
| HLA-A*0214 |
| HLA-A3 |
| HLA-A*0301 |
| HLA-A*1101 |
| HLA-A23 |
| HLA-A24 |
| HLA-A25 |
| HLA-A*2902 |
| HLA-A*3101 |
| HLA-A*3302 |
| HLA-A*6801 |
| HLA-A*6901 |
| HLA-B7 |
| HLA-B*0702 |
| HLA-B*0703 |
| HLA-B*0704 |
| HLA-B*0705 |
| HLA-B8 |
| HLA-B13 |
| HLA-B14 |
| HLA-B*1501 (B62) |
| HLA-B17 |
| HLA-B18 |
| HLA-B22 |
| HLA-B27 |
| HLA-B*2702 |
| HLA-B*2704 |
| HLA-B*2705 |
| HLA-B*2709 |
| HLA-B35 |
| HLA-B*3501 |
| HLA-B*3502 |
| HLA-B*3701 |
| HLA-B*3801 |
| HLA-B*39011 |
| HLA-B*3902 |
| HLA-B40 |
| HLA-B*40012 (B60) |
| HLA-B*4006 (B61) |
| HLA-B44 |
| HLA-B*4402 |
| HLA-B*4403 |
| HLA-B*4501 |
| HLA-B*4601 |
| HLA-B51 |
| HLA-B*5101 |
| HLA-B*5102 |
| HLA-B*5103 |
| HLA-B*5201 |
| HLA-B*5301 |
| HLA-B*5401 |
| HLA-B*5501 |
| HLA-H*5502 |
| HLA-B*5601 |
| HLA-B*5801 |
| HLA-B*6701 |
| HLA-B*7301 |
| HLA-B*7801 |
| HLA-Cw*0102 |
| HLA-Cw*0301 |
| HLA-Cw*0304 |
| HLA-Cw*0401 |
| HLA-Cw*0601 |
| HLA-Cw*0602 |
| HLA-Cw*0702 |
| HLA-Cw8 |
| HLA-Cw*1601 M |
| HLA-G |

| **Murine** |
|---|
| H2-K^{d} |
| H2-D^{d} |
| H2-L^{d} |
| H2-K^{b} |
| H2-D^{b} |
| H2-K^{k} |
| H2-K^{kml} |
| Qa-1^{a} |
| Qa-2 |
| H2-M3 |

| **Rat** |
|---|
| RT1.A^{a} |
| RT1.A¹ |

| **Bovine** |
|---|
| Bota-A11 |
| Bota-A20 |

| **Chicken** |
|---|
| B-F4 |
| B-F12 |
| B-F15 |
| B-F19 |

| **Chimpanzee** |
|---|
| Patr-A*04 |
| Patr-A* 11 |
| Patr-B*01 |
| Patr-B*13 |
| Patr-B*16 |

| **Baboon** |
|---|
| Papa-A*06 |

| **Macaque** |
|---|
| Mamu-A*01 |

| **Swine** |
|---|
| SLA (haplotype d/d) |

| **Virus homolog** |
|---|
| hCMV class I homolog UL18 |

**Table 7**

| Class I MHC Molecules |
|---|
| **Class I** |
| **Human** |
| HLA-A1 |
| HLA-A*0101 |
| HLA-A*0201 |
| HLA-A*0202 |
| HLA-A*0204 |
| HLA-A*0205 |
| HLA-A*0206 |
| HLA-A*0207 |
| HLA-A*0214 |
| HLA-A3 |
| HLA-A*1101 |
| HLA-A24 |
| HLA-A*2902 |
| HLA-A*3101 |
| HLA-A*3302 |
| HLA-A*6801 |
| HLA-A*6901 |
| HLA-B7 |
| HLA-B*0702 |
| HLA-B*0703 |
| HLA-B*0704 |
| HLA-B*0705 |
| HLA-B8 |
| HLA-B14 |
| HLA-B*1501 (B62) |
| HLA-B27 |
| HLA-B*2702 |
| HLA-B*2705 |
| HLA-B35 |
| HLA-B*3501 |
| HLA-B*3502 |
| HLA-B*3701 |
| HLA-B*380 |
| HLA-B*39011 |
| HLA-B*3902 |
| HLA-B40 |
| HLA-B*40012 (B60) |
| HLA-B*4006 (B61) |
| HLA-B44 |
| HLA-B*4402 |
| HLA-B*4403 |
| HLA-B*4601 |
| HLA-B51 |
| HLA-B*5101 |
| HLA-B*5102 |
| HLA-B*5103 |
| HLA-B*520 |
| HLA-B*5301 |
| HLA-B*5401 |
| HLA-B*5501 |
| HLA-B*5502 |
| HLA-B*5601 |
| HLA-B*5801 |
| HLA-B*670 |
| HLA-B*7301 |
| HLA-B*7801 |
| HLA-Cw*0102 |
| HLA-Cw*0301 |
| HLA-Cw*0304 |
| HLA-Cw*0401 |
| HLA-Cw*0601 |
| HLA-Cw*0602 |
| HLA-Cw*0702 |
| HLA-G |
| **tMurine** |
| H2-K^{d} |
| H2-D^{d} |
| H2-L^{d} |
| H2-K^{b} |
| H2-D^{b} |
| H2-K^{k} |
| H2-K^{kml} |
| Qa-2 |

| **Rat** |
|---|
| RT1.A^{a} |
| RT1.A¹ |

| **Bovine** |
|---|
| Bota-A11 |
| Bota-A20 |
| **Chicken** |
| B-F4 |
| B-F12 |
| B-F15 |
| B-F19 |

| **Virus homolog** |
|---|
| hCMV class I homolog UL18 |

**Table 8**

| Estimated gene frequencies of HLA-A antigens | | | | | | | | | | |
|---|---|---|---|---|---|---|---|---|---|---|
| Antigen | CAU | | R | | ASI | | LAT | | NAT | |
| | Gf^{a} | SE^{b} | Gf | SE | Gf | SE | Gf | SE | Gf | SE |
| A1 | 15.1843 | 0.0489 | 5.7256 | 0.0771 | 4.4818 | 0.0846 | 7.4007 | 0.0978 | 12.0316 | 0.2533 |
| A2 | 28.6535 | 0.0619 | 18.8849 | 0.1317 | 24.6352 | 0.1794 | 28.1198 | 0.1700 | 29.3408 | 0.3585 |
| A3 | 13.3890 | 0.0463 | 8.4406 | 0.0925 | 2.6454 | 0.0655 | 8.0789 | 0.1019 | 11.0293 | 0.2437 |
| A28 | 4.4652 | 0.0280 | 9.9269 | 0.0997 | 1.7657 | 0.0537 | 8.9446 | 0.1067 | 5.3856 | 0.1750 |
| A36 | 0.0221 | 0.0020 | 1.8836 | 0.0448 | 0.0148 | 0.0049 | 0.1584 | 0.0148 | 0.1545 | 0.0303 |
| A23 | 1.8287 | 0.0181 | 10.2086 | 0.1010 | 0.3256 | 0.0231 | 2.9269 | 0.0628 | 1.9903 | 0.1080 |
| A24 | 9.3251 | 0.0395 | 2.9668 | 0.0560 | 22.0391 | 0.1722 | 13.2610 | 0.1271 | 12.6613 | 0.2590 |
| A9 unsplit | 0.0809 | 0.0038 | 0.0367 | 0.0063 | 0.0858 | 0.0119 | 0.0537 | 0.0086 | 0.0356 | 0.0145 |
| A9 total | 11.2347 | 0.0429 | 13.2121 | 0.1128 | 22.4505 | 0.1733 | 16.2416 | 0.1382 | 14.6872 | 0.2756 |
| A25 | 2.1157 | 0.0195 | 0.4329 | 0.0216 | 0.0990 | 0.0128 | 1.1937 | 0.0404 | 1.4520 | 0.0924 |
| A26 | 3.8795 | 0.0262 | 2.8284 | 0.0547 | 4.6628 | 0.0862 | 3.2612 | 0.0662 | 2.4292 | 0.1191 |
| A34 | 0.1508 | 0.0052 | 3.5228 | 0.0610 | 1.3529 | 0.0470 | 0.4928 | 0.0260 | 0.3150 | 0.0432 |
| A43 | 0.0018 | 0.0006 | 0.0334 | 0.0060 | 0.0231 | 0.0062 | 0.0055 | 0.0028 | 0.0059 | 0.0059 |
| A66 | 0.0173 | 0.0018 | 0.2233 | 0.0155 | 0.0478 | 0.0089 | 0.0399 | 0.0074 | 0.0534 | 0.0178 |
| A10 unsplit | 0.0790 | 0.0038 | 0.0939 | 0.0101 | 0.1255 | 0.0144 | 0.0647 | 0.0094 | 0.0298 | 0.0133 |
| A10 total | 6.2441 | 0.0328 | 7.1348 | 0.0850 | 6.311 | 0.0993 | 5.0578 | 0.0816 | 4.2853 | 0.1565 |
| A29 | 3.5796 | 0.0252 | 3.2071 | 0.0582 | 1.1233 | 0.0429 | 4.5156 | 0.0774 | 3.4345 | 0.1410 |
| A30 | 2.5067 | 0.0212 | 13.0969 | 0.1129 | 2.2025 | 0.0598 | 4.4873 | 0.0772 | 2.5314 | 0.1215 |
| A31 | 2.7386 | 0.0221 | 1.6556 | 0.0420 | 3.6005 | 0.0761 | 4.8328 | 0.0800 | 6.0881 | 0.1855 |
| A32 | 3.6956 | 0.0256 | 1.5384 | 0.0405 | 1.0331 | 0.0411 | 2.7064 | 0.0604 | 2.5521 | 0.1220 |
| A33 | 1.2080 | 0.0148 | 6.5607 | 0.0822 | 9.2701 | 0.1191 | 2.6593 | 0.0599 | 1.0754 | 0.0796 |
| A74 | 0.0277 | 0.0022 | 1.9949 | 0.0461 | 0.0561 | 0.0096 | 0.2027 | 0.0167 | 0.1068 | 0.0252 |
| A19 unsplit | 0.0567 | 0.0032 | 0.2057 | 0.0149 | 0.0990 | 0.0128 | 0.1211 | 0.0129 | 0.0475 | 0.0168 |
| A19 total | 13.8129 | 0.0468 | 28.2593 | 0.1504 | 17.3846 | 0.1555 | 19.5252 | 0.1481 | 15.8358 | 0.2832 |
| AX | 0.8204 | 0.0297 | 4.9506 | 0.0963 | 2.9916 | 0.1177 | 1.6332 | 0.0878 | 1.8454 | 0.1925 |

| | | | | | | | | | | |
|---|---|---|---|---|---|---|---|---|---|---|
| ^{a}Gene frequency. ^{b}Standard error. | | | | | | | | | | |

**Table 9**

| Estimated gene frequencies for HLA-B antigens | | | | | | | | | | |
|---|---|---|---|---|---|---|---|---|---|---|
| Antigen | CAU | | AFR | | ASI | | LAT | | NAT | |
| | Gf^{a} | SE^{b} | Gf | SE | Gf | SE | Gf | SE | Gf | SE |
| B7 | 12.1782 | 0.0445 | 10.5960 | 0.1024 | 4.2691 | 0.0827 | 6.4477 | 0.0918 | 10.9845 | 0.2432 |
| B8 | 9.4077 | 0.0397 | 3.8315 | 0.0634 | 1.3322 | 0.0467 | 3.8225 | 0.0715 | 8.5789 | 0.2176 |
| B13 | 2.3061 | 0.0203 | 0.8103 | 0.0295 | 4.9222 | 0.0886 | 1.2699 | 0.0416 | 1.7495 | 0.1013 |
| B14 | 4.3481 | 0.0277 | 3.0331 | 0.0566 | 0.5004 | 0.0287 | 5.4166 | 0.0846 | 2.9823 | 0.1316 |
| B18 | 4.7980 | 0.0290 | 3.2057 | 0.0582 | 1.1246 | 0.0429 | 4.2349 | 0.0752 | 3.3422 | 0.1391 |
| B27 | 4.3831 | 0.0278 | 1.2918 | 0.0372 | 2.2355 | 0.0603 | 2.3724 | 0.0567 | 5.1970 | 0.1721 |
| B35 | 9.6614 | 0.0402 | 8.5172 | 0.0927 | 8.1203 | 0.1122 | 14.6516 | 0.1329 | 10.1198 | 0.2345 |
| B37 | 1.4032 | 0.0159 | 0.5916 | 0.0252 | 1.2327 | 0.0449 | 0.7807 | 0.0327 | 0.9755 | 0.0759 |
| B41 | 0.9211 | 0.0129 | 0.8183 | 0.0296 | 0.1303 | 0.0147 | 1.2818 | 0.0418 | 0.4766 | 0.0531 |
| B42 | 0.0608 | 0.0033 | 5.6991 | 0.0768 | 0.0841 | 0.0118 | 0.5866 | 0.0284 | 0.2856 | 0.0411 |
| B46 | 0.0099 | 0.0013 | 0.0151 | 0.0040 | 4.9292 | 0.0886 | 0.0234 | 0.0057 | 0.0238 | 0.0119 |
| B47 | 0.2069 | 0.0061 | 0.1305 | 0.0119 | 0.0956 | 0.0126 | 0.1832 | 0.0159 | 0.2139 | 0.0356 |
| B48 | 0.0865 | 0.0040 | 0.1316 | 0.0119 | 2.0276 | 0.0575 | 1.5915 | 0.0466 | 1.0267 | 0.0778 |
| B53 | 0.4620 | 0.0092 | 10.9529 | 0.1039 | 0.4315 | 0.0266 | 1.6982 | 0.0481 | 1.0804 | 0.0798 |
| B59 | 0.0020 | 0.0006 | 0.0032 | 0.0019 | 0.4277 | 0.0265 | 0.0055 | 0.0028 | 0^{c} | ___ |
| B67 | 0.0040 | 0.0009 | 0.0086 | 0.0030 | 0.2276 | 0.0194 | 0.0055 | 0.0028 | 0.0059 | 0.0059 |
| B70 | 0.3270 | 0.0077 | 7.3571 | 0.0866 | 0.8901 | 0.0382 | 1.9266 | 0.0512 | 0.6901 | 0.0639 |
| B73 | 0.0108 | 0.0014 | 0.0032 | 0.0019 | 0.0132 | 0.0047 | 0.0261 | 0.0060 | 0^{c} | |
| B51 | 5.4215 | 0.0307 | 2.5980 | 0.0525 | 7.4751 | 0.1080 | 6.8147 | 0.0943 | 6.9077 | 0.1968 |
| B52 | 0.9658 | 0.0132 | 1.3712 | 0.0383 | 3.5121 | 0.0752 | 2.2447 | 0.0552 | 0.6960 | 0.0641 |
| B5 unsplit | 0.1565 | 0.0053 | 0.1522 | 0.0128 | 0.1288 | 0.0146 | 0.1546 | 0.0146 | 0.1307 | 0.0278 |
| B5 total | 6.5438 | 0.0435 | 4.1214 | 0.0747 | 11.1160 | 0.1504 | 9.2141 | 0.1324 | 7.7344 | 0.2784 |
| B44 | 13.4838 | 0.0465 | 7.0137 | 0.0847 | 5.6807 | 0.0948 | 9.9253 | 0.1121 | 11.8024 | 0.2511 |
| B45 | 0.5771 | 0.0102 | 4.8069 | 0.0708 | 0.1816 | 0.0173 | 1.8812 | 0.0506 | 0.7603 | 0.0670 |
| B12 unsplit | 0.0788 | 0.0038 | 0.0280 | 0.0055 | 0.0049 | 0.0029 | 0.0193 | 0.0051 | 0.0654 | 0.0197 |
| B12 total | 14.1440 | 0.0474 | 11.8486 | 0.1072 | 5.8673 | 0.0963 | 11.8258 | 0.1210 | 12.6281 | 0.2584 |
| B62 | 5.9117 | 0.0320 | 1.5267 | 0.0404 | 9.2249 | 0.1190 | 4.1825 | 0.0747 | 6.9421 | 0.1973 |
| B63 | 0.4302 | 0.0088 | 1.8865 | 0.0448 | 0.4438 | 0.0270 | 0.8083 | 0.0333 | 0.03738 | 0.0471 |
| B75 | 0.0104 | 0.0014 | 0.0226 | 0.0049 | 1.9673 | 0.0566 | 0.1101 | 0.0123 | 0.0356 | 0.0145 |
| B76 | 0.0026 | 0.0007 | 0.0065 | 0.0026 | 0.0874 | 0.0120 | 0.0055 | 0.0028 | 0^{c} | ___ |
| B77 | 0.0057 | 0.0010 | 0.0119 | 0.0036 | 0.0577 | 0.0098 | 0.0083 | 0.0034 | 0.0059 | 0.0059 |
| B15 unsplit | 0.1305 | 0.0049 | 0.0691 | 0.0086 | 0.4301 | 0.0266 | 0.1820 | 0.0158 | 0.0715 | 0.0206 |
| B15 total | 6.4910 | 0.0334 | 3.5232 | 0.0608 | 12.2112 | 0.1344 | 5.2967 | 0.0835 | 7.4290 | 0.2035 |
| B38 | 2.4413 | 0.0209 | 0.3323 | 0.0189 | 3.2818 | 0.0728 | 1.9652 | 0.0517 | 1.1017 | 0.0806 |
| B39 | 1.9614 | 0.0188 | 1.2893 | 0.0371 | 2.0352 | 0.0576 | 6.3040 | 0.0909 | 4.5527 | 0.1615 |
| B16 unsplit | 0.0638 | 0.0034 | 0.0237 | 0.0051 | 0.0644 | 0.0103 | 0.1226 | 0.0130 | 0.0593 | 0.0188 |
| B16 total | 4.4667 | 0.0280 | 1.6453 | 0.0419 | 5.3814 | 0.0921 | 8.3917 | 0.1036 | 5.7137 | 0.1797 |
| B57 | 3.5955 | 0.0252 | 5.6746 | 0.0766 | 2.5782 | 0.0647 | 2.1800 | 0.0544 | 2.7265 | 0.1260 |
| B58 | 0.7152 | 0.0114 | 5.9546 | 0.0784 | 4.0189 | 0.0803 | 1.2481 | 0.0413 | 0.9398 | 0.0745 |
| B17 unsplit | 0.2845 | 0.0072 | 0.3248 | 0.0187 | 0.3751 | 0.0248 | 0.1446 | 0.0141 | 0.2674 | 0.0398 |
| B17 total | 4.5952 | 0.0284 | 11.9540 | 0.1076 | 6.9722 | 0.1041 | 3.5727 | 0.0691 | 3.9338 | 0.1503 |
| B49 | 1.6452 | 0.0172 | 2.6286 | 0.0528 | 0.2440 | 0.0200 | 2.3353 | 0.0562 | 1.5462 | 0.0953 |
| B50 | 1.0580 | 0.0138 | 0.8636 | 0.0304 | 0.4421 | 0.0270 | 1.8883 | 0.0507 | 0.7862 | 0.0681 |
| B21 unsplit | 0.0702 | 0.0036 | 0.0270 | 0.0054 | 0.0132 | 0.0047 | 0.0771 | 0.0103 | 0.0356 | 0.0145 |
| B21 total | 2.7733 | 0.0222 | 3.5192 | 0.0608 | 0.6993 | 0.0339 | 4.3007 | 0.0755 | 2.3680 | 0.1174 |
| B54 | 0.0124 | 0.0015 | 0.0183 | 0.0044 | 2.6873 | 0.0660 | 0.0289 | 0.0063 | 0.0534 | 0.0178 |
| B55 | 1.9046 | 0.0185 | 0.4895 | 0.0229 | 2.2444 | 0.0604 | 0.9515 | 0.0361 | 1.4054 | 0.0909 |
| B56 | 0.5527 | 0.0100 | 0.2686 | 0.0170 | 0.8260 | 0.0368 | 0.3596 | 0.0222 | 0.3387 | 0.0448 |
| B22 unsplit | 0.1682 | 0.0055 | 0.0496 | 0.0073 | 0.2730 | 0.0212 | 0.0372 | 0.0071 | 0.1246 | 0.0272 |
| B22 total | 2.0852 | 0.0217 | 0.8261 | 0.0297 | 6.0307 | 0.0971 | 1.3771 | 0.0433 | 1.9221 | 0.1060 |
| B60 | 5.2222 | 0.0302 | 1.5299 | 0.0404 | 8.3254 | 0.1135 | 2.2538 | 0.0553 | 5.7218 | 0.1801 |
| B61 | 1.1916 | 0.0147 | 0.4709 | 0.0225 | 6.2072 | 0.0989 | 4.6691 | 0.0788 | 2.6023 | 0.1231 |
| B40 unsplit | 0.2696 | 0.0070 | 0.0388 | 0.0065 | 0.3205 | 0.0230 | 0.2473 | 0.0184 | 0.2271 | 0.0367 |
| B40 total | 6.6834 | 0.0338 | 2.0396 | 0.0465 | 14.8531 | 0.1462 | 7.1702 | 0.0963 | 8.5512 | 0.2168 |
| BX | 1.0922 | 0.0252 | 3.5258 | 0.0802 | 3.8749 | 0.0988 | 2.5266 | 0.0807 | 1.9867 | 0.1634 |

| | | | | | | | | | | |
|---|---|---|---|---|---|---|---|---|---|---|
| ^{a}Gene frequency. ^{b}Standard error. ^{c}The observed gene count was zero. | | | | | | | | | | |

**Table 10.**

| Estimated gene frequencies of HLA-DR antigens | | | | | | | | | | |
|---|---|---|---|---|---|---|---|---|---|---|
| Antigen | CAU | | AFR | | ASI | | LAT | | NAT | |
| | Gf^{a} | SE^{b} | Gf | SE | Gf | SE | Gf | SE | Gf | SE |
| DR1 | 10.2279 | 0.0413 | 6.8200 | 0.0832 | 3.4628 | 0.0747 | 7.9859 | 0.1013 | 8.2512 | 0.2139 |
| DR2 | 15.2408 | 0.0491 | 16.2373 | 0.1222 | 18.6162 | 0.1608 | 11.2389 | 0.1182 | 15.3932 | 0.2818 |
| DR3 | 10.8708 | 0.0424 | 13.3080 | 0.1124 | 4.7223 | 0.0867 | 7.8998 | 0.1008 | 10.2549 | 0.2361 |
| DR4 | 16.7589 | 0.0511 | 5.7084 | 0.0765 | 15.4623 | 0.1490 | 20.5373 | 0.1520 | 19.8264 | 0.3123 |
| DR6 | 14.3937 | 0.0479 | 18.6117 | 0.1291 | 13.4471 | 0.1404 | 17.0265 | 0.1411 | 14.8021 | 0.2772 |
| DR7 | 13.2807 | 0.0463 | 10.1317 | 0.0997 | 6.9270 | 0.1040 | 10.6726 | 0.1155 | 10.4219 | 0.2378 |
| DR8 | 2.8820 | 0.0227 | 6.2673 | 0.0800 | 6.5413 | 0.1013 | 9.7731 | 0.1110 | 6.0059 | 0.1844 |
| DR9 | 1.0616 | 0.0139 | 2.9646 | 0.0559 | 9.7527 | 0.1218 | 1.0712 | 0.0383 | 2.8662 | 0.1291 |
| DR10 | 1.4790 | 0.0163 | 2.0397 | 0.0465 | 2.2304 | 0.0602 | 1.8044 | 0.0495 | 1.0896 | 0.0801 |
| DR11 | 9.3180 | 0.0396 | 10.6151 | 0.1018 | 4.7375 | 0.0869 | 7.0411 | 0.0955 | 5.3152 | 0.1740 |
| DR12 | 1.9070 | 0.0185 | 4.1152 | 0.0655 | 10.1365 | 0.1239 | 1.7244 | 0.0484 | 2.0132 | 0.1086 |
| DR5 unsplit | 1.2199 | 0.0149 | 2.2957 | 0.0493 | 1.4118 | 0.0480 | 1.8225 | 0.0498 | 1.6769 | 0.0992 |
| DR5 total | 12.4449 | 0.0045 | 17.0260 | 0.1243 | 16.2858 | 0.1516 | 10.5880 | 0.1148 | 9.0052 | 0.2218 |
| DRX | 1.3598 | 0.0342 | 0.8853 | 0.0760 | 2.5521 | 0.1089 | 1.4023 | 0.0930 | 2.0834 | 0.2037 |

| | | | | | | | | | | |
|---|---|---|---|---|---|---|---|---|---|---|
| ^{a}Gene frequency. ^{b}Standard error. | | | | | | | | | | |

It can be desirable to express housekeeping peptides in the context of a larger protein. Processing can be detected even when a small number of amino acids are present beyond the terminus of an epitope. Small peptide hormones are usually proteolytically processed from longer translation products, often in the size range of approximately 60-120 amino acids. This fact has led some to assume that this is the minimum size that can be efficiently translated. In some embodiments, the housekeeping peptide can be embedded in a translation product of at least about 60 amino acids, in others 70, 80, 90 amino acids, and in still others 100, 110 or 120 amino acids, for example. In other embodiments the housekeeping peptide can be embedded in a translation product of at least about 50, 30, or 15 amino acids.

Due to differential proteasomal processing, the immunoproteasome of the pAPC produces peptides that are different from those produced by the housekeeping proteasome in peripheral body cells. Thus, in expressing a housekeeping peptide in the context of a larger protein, it is preferably expressed in the pAPC in a context other than its full-length native sequence, because, as a housekeeping epitope, it is generally only efficiently processed from the native protein by the housekeeping proteasome, which is not active in the pAPC. In order to encode the housekeeping epitope in a DNA sequence encoding a larger polypeptide, it is useful to find flanking areas on either side of the sequence encoding the epitope that permit appropriate cleavage by the immunoproteasome in order to liberate that housekeeping epitope. Such a sequence promoting appropriate processing is referred to hereinafter as having substrate or liberation sequence function. Altering flanking amino acid residues at the N-terminus and C-terminus of the desired housekeeping epitope can facilitate appropriate cleavage and generation of the housekeeping epitope in the pAPC. Sequences embedding housekeeping epitopes can be designed *de novo* and screened to determine which can be successfully processed by immunoproteasomes to liberate housekeeping epitopes.

Alternatively, another strategy is very effective for identifying sequences allowing production of housekeeping epitopes in APC. A contiguous sequence of amino acids can bc generated from head to tail arrangement of one or more Housekeeping epitopes. A construct expressing this sequence is used to immunize an animal, and the resulting T cell response is evaluated to determine its specificity to one or more of the epitopes in the array. These immune responses indicate housekeeping epitopes that are processed in the pAPC effectively. The necessary flanking areas around this epitope are thereby defined. The use of flanking regions of about 4-6 amino acids on either side of the desired peptide can provide the necessary information to facilitate proteasome processing of the housekeeping epitope by the immunoproteasome. Therefore, a substrate or liberation sequence of approximately 16-22 amino acids can be inserted into, or fused to, any protein sequence effectively to result in that housekeeping epitope being produced in an APC. In some embodiments, a broader context of a substrate sequence can also influence processing. In such embodiments, comparisons of a liberaton sequence in a variety of contexts can be useful in further optimizing a particular substrate sequence. In alternate embodiments the whole head-to-tail array of epitopes, or just the epitopes immediately adjacent to the correctly processed housekeeping epitope can be similarly transferred from a tes construct to a vaccine vector.

It is also described herein that the housekeeping epitopes can be embedded between known immune epitopes, or segments of such, thereby providing an appropriate context for processing. The abutment of housekeeping and immune epitopes can generate the necessary context to enable the immunoproteasome to liberate the housekeeping epitope, or a larger fragment, preferably including a correct C-terminus. It can be useful to screen constructs to verify that the desired epitope is produced. The abutment of housekeeping epitopes can generate a site cleavable by the immunoproteasome. Some embodiments of the invention employ known epitopes to flank housekeeping epitopes in test substrates; in others, screening as described below is used, whether the flanking regions are arbitrary sequences or mutants of the natural flanking sequence, and whether or not knowledge of proteasomal cleavage preferences are used in designing the substrates.

Cleavage at the mature N-terminus of the epitope, while advantageous, is not required, since a variety of N-terminal trimming activities exist in the cell that can generate the mature N-terminus of the epitope subsequent to proteasomal processing. It is preferred that such N-terminal extension be less than about 25 amino acids in length and it is further preferred that the extension have few or no proline residues. Preferably, in screening, consideration is given not only to cleavage at the ends of the epitope (or at least at its C-terminus), but consideration also can be given to ensure limited cleavage within the epitope.

Shotgun approaches can be used in designing test substrates and can increase the efficiency of screening. In one embodiment multiple epitopes can be assembled one after the other, with individual epitopes possibly appearing more than once. The substrate can be screened to determine which epitopes can be produced. In the case where a particular epitope is of concern, a substrate can be designed in which it appears in multiple different contexts. When a single epitope appearing in more than one context is liberated from the substrate additional secondary test substrates, in which individual instances of the epitope are removed, disabled, or are unique, can be used to determine which are being liberated and truly confer substrate or liberation sequence function.

Several readily practicable screens exist. A preferred *in vitro* screen utilizes proteasomal digestion analysis, using purified immunoproteasomcs, to determine if the desired housekeeping epitope can be liberated from a synthetic peptide embodying the sequence in question. The position of the cleavages obtained can be determined by techniques such as mass spectrometry, HPLC, and N-terminal pool sequencing; as described in greater detail in U.S. Patent Application Nos. 09/561,074, 09/560,465 and 10/117,937, and Provisional U.S. Patent Application Nos. 60/282,211, 60/337,017, and 60/363, 210.

Alternatively, *in vivo* and cell-based screens such as immunization or target sensitization can be employed. For immunization a nucleic acid construct capable of expressing the sequence in question is used. Harvested CTL can be tested for their ability to recognize target cells presenting the housekeeping epitope in question. Such targets cells are most readily obtained by pulsing cells expressing the appropriate MHC molecule with synthetic peptide embodying the mature housekeeping epitope. Alternatively, immunization can be carried out using cells known to express housekeeping proteasome and the antigen from which the housekeeping epitope is derived, either endogenously or through genetic engineering. To use target sensitization as a screen, CTL, or preferably a CTL clone, that recognizes the housekeeping epitope can be used. In this case it is the target cell that expresses the embedded housekeeping epitope (instead of the pAPC during immunization) and it must express immunoproteasome. Generally, the cell or target cell can be transformed with an appropriate nucleic acid construct to confer expression of the embedded housekeeping epitope. Loading with a synthetic peptide embodying the embedded epitope using peptide loaded liposomes, or complexed with cationic lipid protein transfer reagents such as BIOPORTER™ (Gene Therapy Systems, San Diego, CA), represents an alternative.

Once sequences with substrate or liberation sequence function are identified they can be encoded in nucleic acid vectors, chemically synthesized, or produced recombinantly. In any of these forms they can be incorporated into immunogenic compositions. Such compositions can be used *in vitro* in vaccine development or in the generation or expansion of CTL to be used in adoptive immunotherapy. *In vivo* they can be used to induce, amplify or sustain and active immune response. The uptake of polypeptides for processing and presentation can be greatly enhanced by packaging with cationic lipid, the addition of a tract of cationic amino acids such as poly-L-lysine (Ryser, H.J. et al., J. Cell Physiol. 113:167-178, 1982; Shen, W.C. & Ryser, H.J., Proc. Natl. Aced. Sci. USA 75:1872-1876, 1978), the incorporation into branched structures with importation signals (Sheldon, K. et al., Proc. Natl. Aced. Sci. USA 92:2056-2060, 1995), or mixture with or fusion to polypeptides with protein transfer function including peptide carriers such as pep-1 (Morris, M.C., et al., Nat. Biotech. 19:1173-1176, 2001), the PreS2 translocation motif of hepatitis B virus surface antigen, VP22 of herpes viruses, and HIV-TAT protein (Oess, S. & Hildt, E., Gene Ther. 7:750-758, 2000; Ford, K.G., et al., Gene Ther. 8:1-4, 2001; Hung, C.F. et al., J. Virol. 76:2676-2682, 2002; Oliveira, S.C., et a;. Hum. Gene Ther. 12:1353-1359, 2001; Normand, N. et al., J. Biol. Chem. 276:15042-15050, 2001; Schwartz, J.J. & Zhang, S., Curr. Opin. Mol. Ther. 2:162-167, 2000; Elliot G., 7 Hare, P. Cell 88:223-233, 1997), among other methodologies. Particularly for fusion proteins the immunogen can be produced in culture and the purified protein administered or, in the alternative, the nucleic acid vector can be administered so that the immunogen is produced and secreted by cells transformed *in vivo.* In either scenario the transport function of the fusion protein facilitates uptake by pAPC.

### EXAMPLES

### Example 1

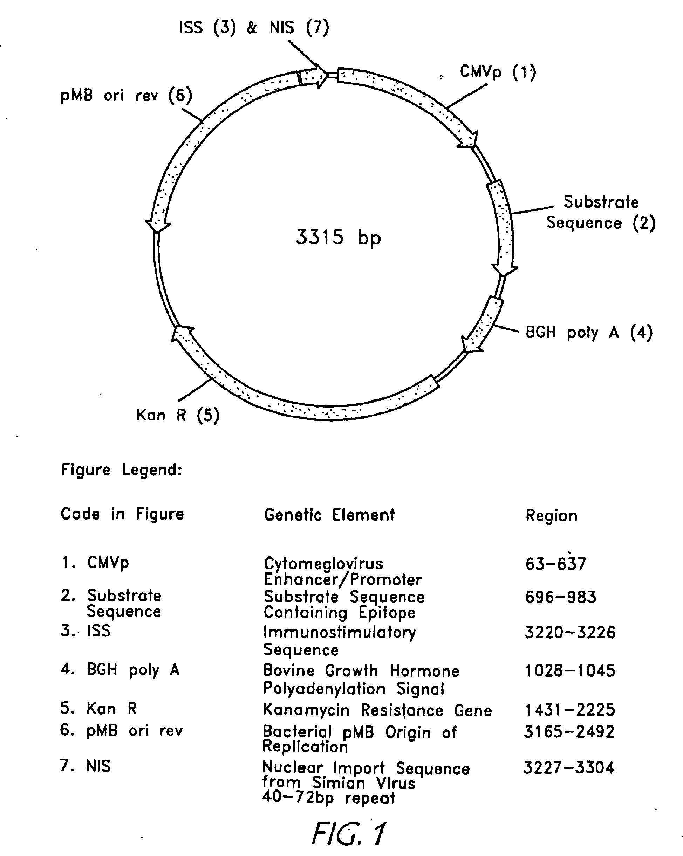

A recombinant DNA plasmid vaccine, pMA2M, which encodes one polypeptide with an HLA A2-specific CTL epitope ELAGIGILTV (SEQ ID NO. 1) from melan-A (26-35A27L), and a portion (amino acids 31-96) of melan-A (SEQ ID NO. 2) including the epitope clusters at amino acids 31-48 and 56-69, was constructed. These clusters were previously disclosed in U.S. Patent Application No. 09/561,571 entitled EPITOPE CLUSTERS. Flanking the defined melan-A CTL epitope are short amino acid sequences derived from human tyrosinase (SEQ ID NO. 3) to facilitate liberation of the melan-A housekeeping epitope by processing by the immunoproteasome. In addition, these amino acid sequences represent potential CTL epitopes themselves. The cDNA sequence for the polypeptide in the plasmid is under the control of promoter/enhancer sequence from cytomegalovirus (CMVp) (see Figure 1), which allows efficient transcription of messenger for the polypeptide upon uptake by APCs. The bovine growth hormone polyadenylation signal (BGH polyA) at the 3' end of the encoding sequence provides a signal for polyadenylation of the messenger to increase its stability as well as for translocation out of nucleus into the cytoplasm for translation. To facilitate plasmid transport into the nucleus after uptake, a nuclear import sequence (NIS) from simian virus 40 (SV40) has been inserted in the plasmid backbone. The plasmid carries two copies of a CpG immunostimulatory motif, one in the NIS sequence and one in the plasmid backbone. Lastly, two prokaryotic genetic elements in the plasmid are responsible for amplification in *E.coli,* the kanamycin resistance gene (Kan R) and the pMB 1 bacterial origin of replication.

### SUBSTRATE or LIBERATION sequence

The amino acid sequence of the encoded polypeptide (94 amino acid residues in length) (SEQ ID NO. 4) containing a 28 amino acid substrate or liberation sequence at its N-terminus (SEQ ID NO. 5) is given below:

MLLAVLYCL-ELAGIGILTV-YMDGTMSQV-
GILTVILGVLLLIGCWYCRRRNGYRALMDKSLHVGTQCALTRRCPQEGFDHRDSKVSLQEK
NCEPV

The first 9 amino acid residues are derived from tyrosinase₁₋₉ (SEQ ID NO. 6), the next ten constitute melan-A (26-35A27L) (SEQ ID NO. 1), and amino acid residues 20 to 29 are derived from tyrosinasc₃₆₉₋₃₇₇ (SEQ ID NO. 7). These two tyrosinase nonamer sequences both represent potential HLA A2-specific CTL epitopes. Amino acid residues 10-19 constitute melan-A (26-35A27L) an analog of an HLA A2-specific CTL epitope from melan-A, EAAGIGILTV (SEQ ID NO. 8), with an elevated potency in inducing CTL responses during *in vitro* immunization of human PBMC and *in vivo* immunization in mice. The segment of melan-A constituting the rest of the polypeptide (amino acid residues 30 to 94) contain a number of predicted HLA A2-specific epitopes, including the epitope clusters cited above, and thus can be useful in generating a response to immune epitopes as described at length in the patent applications 'Epitope Synchronization in Antigen Presenting Cells' and 'Epitope Clusters'. This region was also included to overcome any difficulties that can be associated with the expression of shorter sequences. A drawing of pMA2M is shown in Figure 1.

### Plasmid construction

A pair of long complementary oligonucleotides was synthesized which encoded the first 30 amino acid residues. In addition, upon annealing, these oligonucleotides generated the cohensive ends of *Afl* II at the 5' end and that of *EcoR* I at the 3' end. The melan A₃₁₋₉₆ region was amplified with PCR using oligonucleotides carrying restriction sites for *EcoR* I at the 5' end and *Not* I at the 3' end. The PCR product was digested with *EcoR* I and *Not* I and ligated into the vector backbone, described in Example 1, that had been digested with *Afl* II and *Not* I, along with the annealed oligonucleotides encoding the amino terminal region in a three-fragment ligation. The entire coding sequence was verified by DNA sequencing. The sequence of the entire insert, from the *Afl* II site at the 5' end to the *Not* I site at the 3' end is disclosed as SEQ ID NO. 9. Nucleotides 12-293 encode the polypeptide.

### Example 2

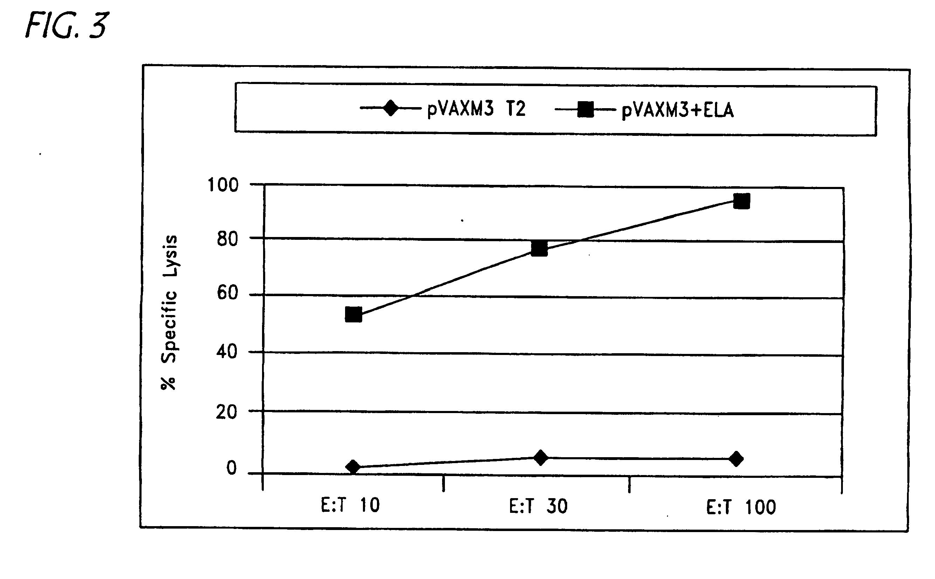

Three vectors containing melan-A (26-35A27L) (SEQ ID NO. 1) as an embedded housekeeping epitope were tested for their ability to induce a CTL response to this epitope in HLA-A2 transgenic HID mice (Pascolo et al. J. Exp. Med. 185:2043-2051, 1997). One of the vectors was pMA2M described above (called pVAXM3 in Figure 3). In pVAXM2 the same basic group of 3 epitopes was repeated several times with the flanking epitopes truncated by differing degrees in the various repeats of the array. Specifically the cassette consisted of:

M-Tyr(5-9)-ELA-Tyr(369-373)-Tyr(4-9)-ELA-Tyr(369-374)-Tyr(3-9)-ELA-
Tyr(369-375)-Tyr(2-9)-ELA
(SEQ ID NO. 10)

where ELA represents melan-A (26-35A27L) (SEQ ID NO. 1). This cassette was inserted in the same plasmid backbone as used for pVAXM3. The third, pVAXM1 is identical to pVAXM2 except that the epitope array is followed by an IRES (internal ribosome entry site for encephalomyocarditis virus) linked to a reading frame encoding melan-A 31-70.

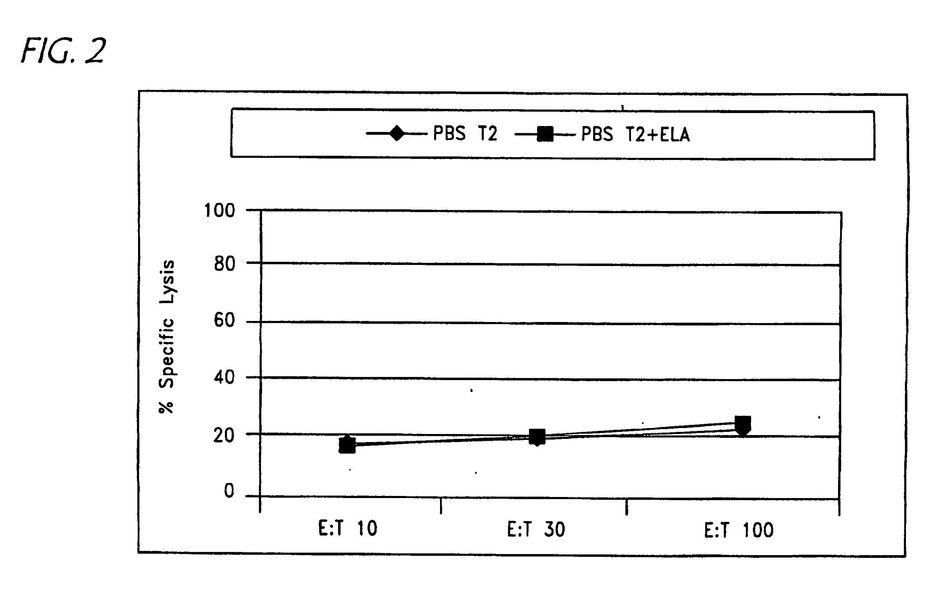

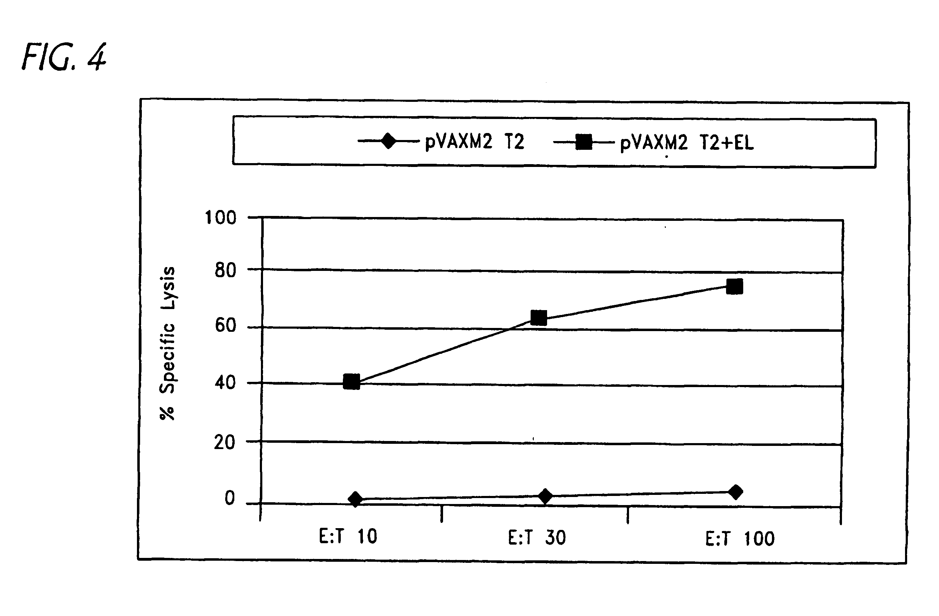

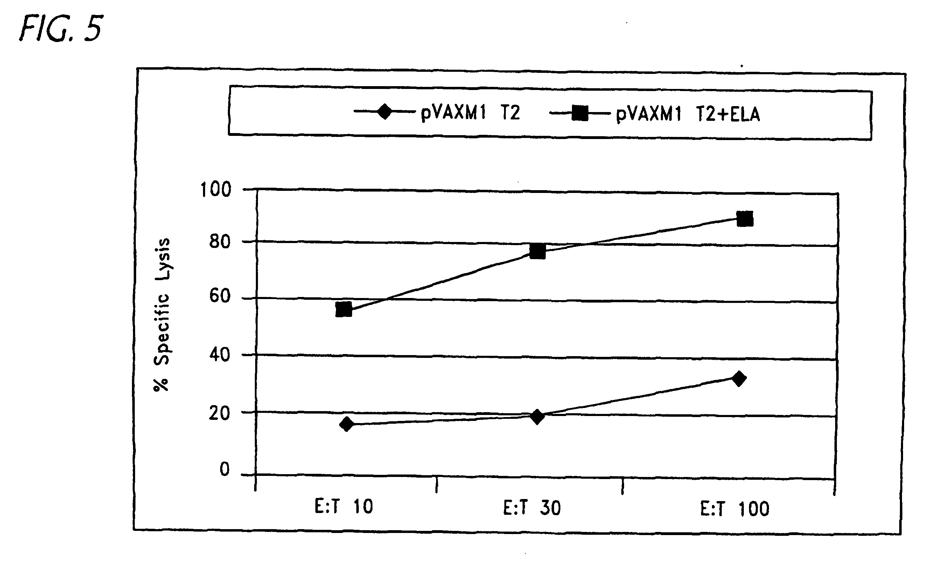

Four groups of three HHD A2.1 mice were injected intranodally in surgically exposed inguinal lymph nodes with 25 µl of I mg/ml plasmid DNA in PBS on days 0, 3, and 6, each group receiving one of the three vectors or PBS alone. On day 14 the spleens were harvested and restimulated *in vitro* one time with 3-day LPS blasts pulsed with peptide (melan-A (26-35A27L)(SEQ ID NO. 1)). The *in vitro* cultures were supplemented with Rat T-Stim (Collaborative Biomedical Products) on the 3^{rd} day and assayed for cytolytic activity on the 7^{th} day using a standard ⁵¹Cr-release assay. Figures 2 to 5 show % specific lysis obtained using the cells immunized with PBS, pVAXM1, pVAXM2, and pVAXM3, respectively on T2 target cells and T2 target cells pulsed with melan-A (26-35A27L) (ELA) (SEQ ID NO. 1). All three vectors generated strong CTL responses. These data indicated that the plasmids have been taken up by APCs, the encoded polypeptide has been synthesized and proteolytically processed to produce the decamer epitope in question (that is, it had substrate or liberation sequence function), and that the epitope became HLA-A2 bound for presentation. Also, an isolated variant of pVAXM2, that terminates after the 55^{th} amino acid, worked similarly well as the full length version (data not shown). Whether other potential epitopes within the expression cassette can also be produced and be active in inducing CTL responses can be determined by testing for CTL activity against target cells pulsed with corresponding synthetic peptides.

### Example 3

### An NY-ESO-1 (SEO ID NO. 11) SUBSTRATE/LIBERATION Sequence

Six other epitope arrays were tested leading to the identification of a substrate/liberation sequence for the housekeeping epitope NY-ESO-1₁₅₇₋₁₆₅ (SEQ ID NO. 12). The component epitopes of the arrays were:

| | | |
|---|---|---|
| SSX-2₄₁₋₄₉: | KASEKIFYV (SEQ ID NO. 13) | Array element A |
| NY-ESO-1₁₅₇₋₁₆₅: | SLLMWITQC (SEQ ID NO. 12) | Array element B |
| NY-ESO-1₁₆₃₋₁₇₁: | TQCFLPVFL (SEQ ID NO. 14) | Array element C |
| PSMA₂₈₈₋₂₉₇: | GLPSIPVHPI (SEQ ID NO. 15) | Array element D |
| TYR₄₋₉: | AVLYCL (SEQ ID NO. 16) | Array element E |

The six arrays had the following arrangements of elements after starting with an initiator methionine:

| | |
|---|---|
| pVAX-PC-A: | B-A-D-D-A-B-A-A |
| pVAX-PC-B: | D-A-B-A-A-D-B-A |
| pVAX-PC-C: | E-A-D-B-A-B-E-A-A |
| pVAX-HC-A: | B-A-C-B-A-A-C-A |
| pVAX-BC-B: | C-A-B-C-A-A-B-A |
| pVAX-BC-C: | E-A-A-B-C-B-A-A |

These arrays were inserted into the same vector backbone described in the examples above. The plasmid vectors were used to immunize mice essentially as described in Example 2 and the resulting CTL were tested for their ability to specifically lyse target cells pulsed with the peptide NY-ESO-1 157-165, corresponding to element B above. Both pVAX-PC-A and pVAX-BC-A were found to induce specific lytic activity. Comparing the contexts of the epitope (element B) in the various arrays, and particularly between pVAX-PC-A and pVAX-BC-A, between pVAX-PC-A and pVA3C-PC-B, and between pVAX-BC-A and pVAX-BC-C, it was concluded that it was the first occurrence of the epitope in pVAX-PC-A and pVAX-BC-A that was being correctly processed and presented. In other words an initiator methionine followed by elements B-A constitute a substrate/liberation sequence for the presentation of element B. On this basis a new expression cassette for use as a vaccine was constructed encoding the following elements:

An initiator methionine,

NY-ESO-1₁₅₇₋₁₆₅ (**bold**) - housekeeping epitope,

SSX2₄₁₋₄₉ (*italic*) - providing appropriate context for processing, and

NY-ESO-1₇₇₋₁₈₀ - to avoid "short sequence" problems and provide immune epitopes.

Thus the construct encodes the amino acid sequence:

**M-SLLMWITQC**-*KASEKIFYV-*RCGARGPESRLLEFYLAMPFATPMEAELARRSLAQDAPPLPVPGVLLKEFTVSGNILTIRLTA ADHRQLQLSISSCLQQLSLLMWTTQCFLPVFLAQPPSGQRR (SEQ ID NO. 17) and MSLLMWITQCKASEKIFYV (SEQ ID NO. 18) constitutes the liberation or substrate sequence. A polynucleotide encoding SEQ ID NO. 17 (SEQ ID NO. 19: nucleotides 12-380) was inserted into the same plasmid backbone as used for pMA2M generating the plasmid pN157.

### Example 4

A construct similar to pN157 containing the whole epitope array from pVAX-PC-A was also made and designated pBPL. Thus the encoded amino acid sequence in pBPL is:

M-SLLMWITQC-KASEKIFYV-GLPSIPVHPI-GLPSIPVHPI-KASEKIFYV-
SLLMWITQC-KASEKIFYV-KASEKIFYV-
RCGARGPESRLLEFYLAMPFATPMEAELARRSLAQDAPPLPVPGVLLKEFTVSGNILTIRLTA
ADHRQLQLSISSCLQQLSLLMWITQCFLPVFLAQPPSGQRR (SEQ ID NO. 20).

SEQ ID NO. 21 is the polynucleotide encoding SEQ ID NO. 20 used in pBPL.

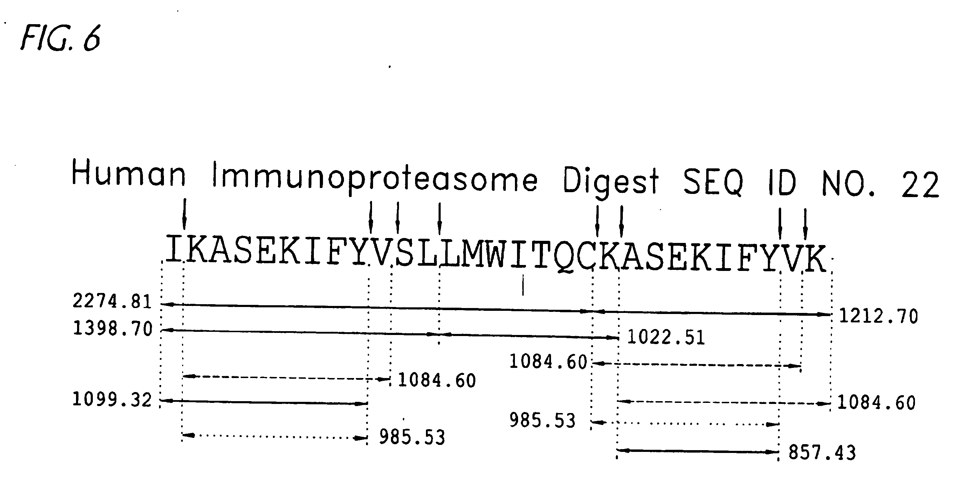

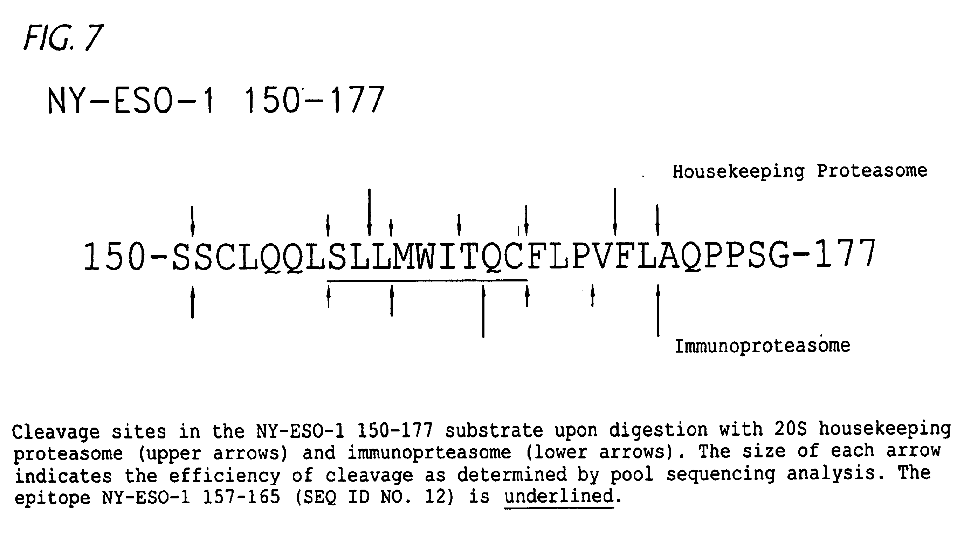

A portion of SEQ ID NO. 20, I*KASEKIFYV***SLLMITQC***KASEKIFYVK* (SEQ ID NO. 22) was made as a synthetic peptide and subjected to *in vitro* proteasomal digestion analysis with human immunoproteasome, utilizing both mass spectrometry and N-terminal pool sequencing. The identification of a cleavage after the C residue indicates that this segment of the construct can function as a substrate or liberation sequence for NY-ESO-I₁₅₇₋₁₆₅ (SEQ ID NO. 12) epitope (see Figure 6). Figure 7 shows the differential processing of the SLLMWITQC epitope (SEQ ID NO. 12) in its native context where the cleavage following the C is more efficiently produced by housekeeping than immunoproteasome. The immunoproteasome also produces a major cleavage internal to the epitope, between the T and the Q when the epitope is in its native context, but not in the context of SEQ ID NO. 22 (compare fig. 6 and 7).

### Example 5

Screening of further epitope arrays led to the identification of constructs promoting the expression of the epitope SSX-₂₄₁₋₄₉ (SEQ ID NO. 13). In addition to some of the array elements defined in Example 3, the following additional elements were also used:

SSX-4₅₇₋₆₅:VMTKLGFKV (SEQ ID NO. 23) Array element F.

PSMA₇₃₀₋₇₃₉: RQIYVAAFTV (SEQ ID NO. 24) Array element G.

A construct, denoted CTLA02, encoding an initiator methionine and the.array F-A-G-D-C-F-G-A, was found to successfully immunize HLA-A2 transgenic mice to generate a CTL response recognizing the peptide SSX-2₄₁₋₄₉ (SEQ ID NO. 13).

As described above, it can be desirable to combine a sequence with substrate or liberation sequence function with one that can be processed into immune epitopes. Thus SSX-2₁₅₋₁₈₃ (SEQ ID NO. 25) was combined with all or part of the array as follows:

| | |
|---|---|
| CTLS1: | F-A-G-D-C-F-G-A- SSX-2₁₅₋₁₈₃ (SEQ ID NO. 26) |
| CTLS2: | SSX-2₁₅₋₁₈₃ - F-A-G-D-C-F-G-A (SEQ ID NO. 27) |
| CTLS3: | F-A-G-D- SSX-2₁₅₋₁₈₃ (SEQ ID NO. 28) |
| CTLS4: | SSX-2₁₅₋₁₈₃-C-F-G-A (SEQ ID NO. 29). |

All of the constructs except CTL3 were able to induce CTL recognizing the peptide SSX-2₄₁₋₄₉ (SEQ ID NO. 13). CTLS3 was the only one of these four constructs which did not include the second element A from CTLA02 suggesting that it was this second occurrence of the element that provided substrate or liberation sequence function. In CTLS2 and CTLS4 the A element is at the C-terminal end of the array, as in CTLA02. In CTLS1 the A element is immediately followed by the SSX-2₁₅₋₁₈₃ segment which begins with an alanine, a residue often found after proteasomal cleavage sites (Toes, R.E.M., et al., J. Exp. Med. 194:1-12, 2001). SEQ ID NO. 30 is the polynucleotide sequence encoding SEQ ID NO. 26 used in CTLS1, also called pCBP.

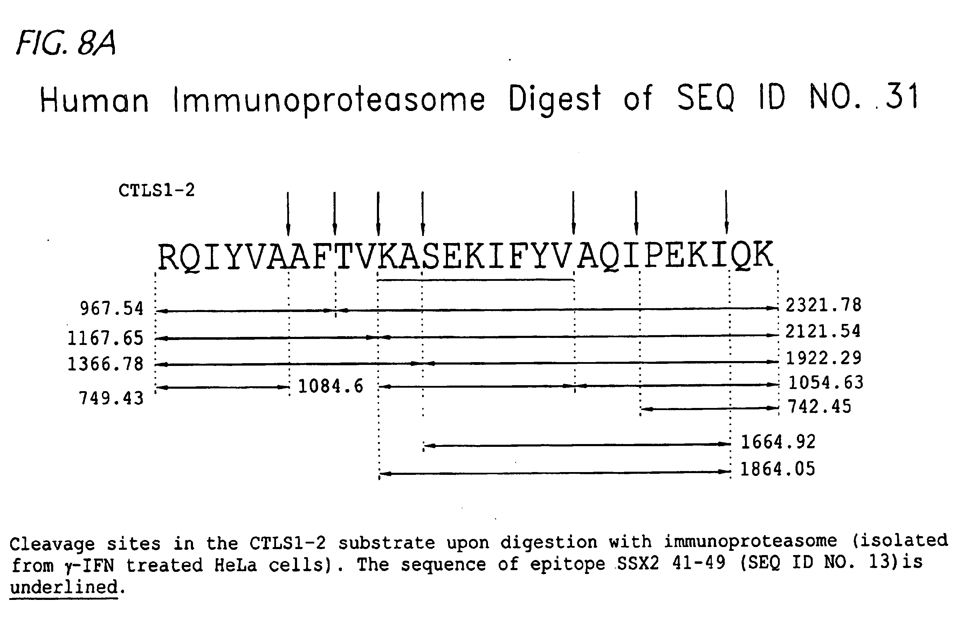

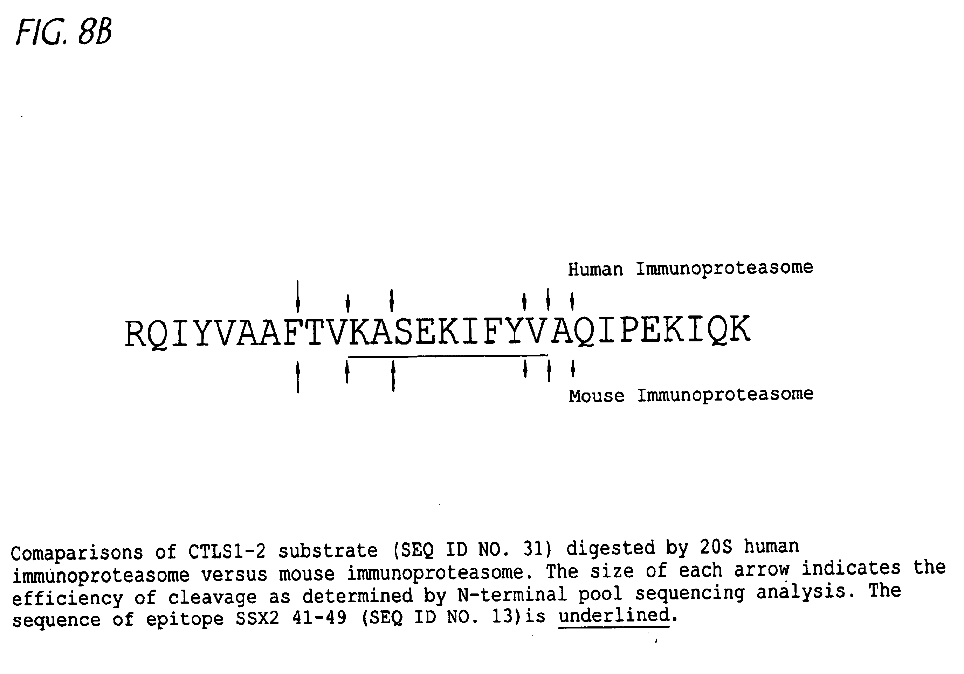

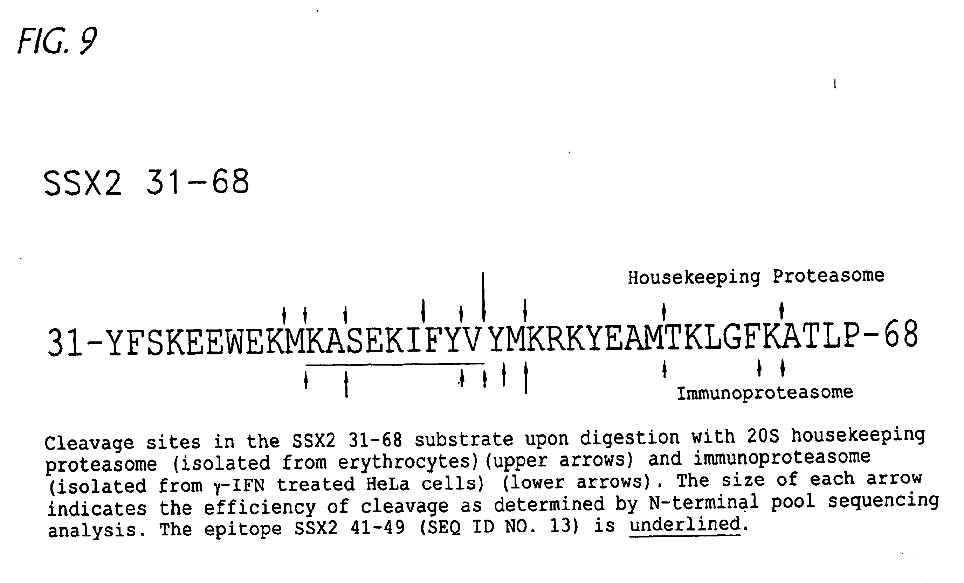

A portion of CTLS1 (SEQ ID NO. 26), encompassing array elements F-A-SSX-2₁₅₋₂₃ with the sequence RQIYVAAFTV-*KASEKIFYV*-AQIPEKIQK (SEQ ID NO. 31), was made as a synthetic peptide and subjected to *in vitro* proteasumal digestion analysis with human immunoproteasome, utilizing both mass spectrometry and N-terminal pool sequencing. The observation that the C-terminus of the SSX-2₄₁₋₄₉ epitope (SEQ ID NO. 13) was generated (see Figure 8) provided further evidence in support of substrate or liberation sequence function. The data in Figure 9 showed the differential processing of the SSX-2₄₁₋₄₉ epitope, *KASEKIFYV* (SEQ ID NO. 13), in its native context, where the cleavage following the V was the predominant cleavage produced by housekeeping proteasome, while the immunoproteasome had several major cleavage sites elsewhere in the sequence. By moving this epitope into the context provided by SEQ ID NO. 31 the desired cleavage became a major one and its relative frequency compared to other immunoproteasome cleavages was increased (compare figs. 8 and 9). The data in figure 8ß also showed the similarity in specificity of mouse and human immunoproteasome lending support to the usefulness of the transgenic mouse model to predict human antigen processing.

### Example 6

Screening also revealed substrate or liberation sequence function for a tyrosinase epitope, Tyr₂₀₇₋₂₁₅ (SEQ ID NO. 32), as part of an array consisting of the sequence [Tyr₁₋₁₇- Tyr₂₀₇₋₂₁₅]₄, [MLLAVLYCLLWSFQTSA-FLPWHRLFL]₄, (SEQ ID NO. 33). The same vector backbone described above was used to express this array. This array differs from those of the other examples in that the Tyr₁₋₁₇ segment, which was included as a source of immune epitopes, is used as a repeated element of the array. This is in contrast with the pattern shown in the other examples where sequence included as a source of immune epitopes and/or length occurred a single time at the beginning or end of the array, the remainder of which was made up of individual epitopes or shorter sequences. Plasmid construction

The polynucleotide encoding SEQ ID NO. 33 was generated by assembly of annealed synthetic oligonucleotides. Four pairs of complementary oligonucleotides were synthesized which span the entire coding sequence with cohesive ends of the restriction sites of Afl II and EcoR I at either terminus. Each complementary pair of oligonucleotides were first annealed, the resultant DNA fragments were ligated stepwise, and the assembled DNA fragment was inserted into the same vector backbone described above pre-digested with Afl II/EcoR I. The construct was called CTLT2/pMEL and SEQ ID NO. 34 is the polynucleotide sequence used to encode SEQ ID NO. 33.

### Example 7

### Administration of a DNA plasmid formulation of a immunotherapeutic for melanoma to humans.

An MA2M melanoma vaccine with a sequence as described in Example 1 above, was formulated in 1% Benzyl alcohol, 1% ethyl alcohol, 0.5mM EDTA, citrate-phosphate, pH 7.6. Aliquots of 200, 400, and 600 µg DNA/ml were prepared for loading into MINIMED 407C infusion pumps. The catheter of a SILHOUETTE infusion set was placed into an inguinal lymph node visualized by ultrasound imaging. The pump and infusion set assembly was originally designed for the delivery of insulin to diabetics. The usual 17mm catheter was substituted with a 31mm catheter for this application. The infusion set was kept patent for 4 days (approximately 96 hours) with an infusion rate of about 25 µl/hour resulting in a total infused volume of approximately 2.4 ml. Thus the total administered dose per infusion was approximately 500, and 1000 µg; and can be 1500 µg, respectively, for the three concentrations described above. Following an infusion, subjects were given a 10 day rest period before starting a subsequent infusion. Given the continued residency of plasmid DNA in the lymph node after administration and the usual kinetics of CTL response following disappearance of antigen, this schedule will be sufficient to maintain the immunologic CTL response.

### Example 8

SEQ ID NO. 22 is made as a synthetic peptide and packaged with a cationic lipid protein transfer reagent. The composition is infused directly into the inguinal lymph node (see example 7) at a rate of 200 to 600 µg of peptide per day for seven days, followed by seven days rest. An initial treatment of 3-8 cycles are conducted.

### Example 9

A fusion protein is made by adding SEQ ID NO. 34 to the 3' end of a nucleotide sequence encoding herpes simplex virus 1 VP22 (SEQ ID NO. 42) in an appropriate mammalian expression vector; the vector used above is suitable. The vector is used to transform HEK 293 cells and 48 to 72 hours later the cells are pelleted, lysed and a soluble extract prepared. The fusion protein is purified by affinity chromatagraphy using an anti-VP22 monoclonal antibody. The purified fusion protein is administered intranodally at a rate of 10 to 100 µg per day for seven days, followed by seven days rest. An initial treatment of 3-8 cycles are conducted.

Further, the present invention can utilize various aspects of the following: U.S. Patent Application Nos. 09/380,534, filed on September 1, 1999, entitled A METHOD OF INDUCING A CTL RESPONSE; 09/776,232, filed on February 2, 2001, entitled METHOD OF INDUCING A CTL RESPONSE; 09/715,835, filed on November 16, 2000, entitled AVOIDANCE OF UNDESIRABLE REPLICATION INTERMEDIATES IN PLASMID PROPOGATION; 09/999,186, filed on November 7, 2001, entitled METHODS OF COMMERCIALIZING AN ANTIGEN; and Provisional U.S. Patent Application No 60/274,063, filed on March 7, 2001, entitled ANTI-NEOVASCULAR VACCINES FOR CANCER.

**Table 11**

| Partial listing of SEQ ID NOS. | | |
|---|---|---|
| 1 | ELAGIGILTV | melan-A 26-35 (A27L) |
| 2 | Melan -A protein | Accession number: NP 005502 |
| 3 | Tyrosinase protein | Accession number: P14679 |
| 4 | MLLAVLYCLELAGIGILTVYMDGTMSQVGILT VILGVLLLIGCWYCRRRNGYRALMDKSLHVG TQCALTRRCPQEGFDHRDSKVSLQEKNCEPV | pMA2M expression product |
| 5 | MLLAVLYCLELAGIGILTVYMDGTMSQV | Liberation or substrate sequence for SEQ ID NO. 1 from pMA2M |
| 6 | MLLAVLYCL | tyrosinase 1-9 |
| 7 | YMDGTMSQV | tyrosinase 369-377 |
| 8 | EAAGIGILTV | melan-A 26-35 |
| 9 | cttaagccaccatgttactagctgttttgtactgcctggaact agcagggatcggcatattgacagtgtatatgga tggaacaatgtcccaggtaggaattctgacagtgatcctggga gtcttactgctcatcggctgttggtattgtaga agacgaaatggatacagagccttgatggataaaagtcttcatg ttggcactcaatgtgccttaacaagaagatgcc cacaagaagggtttgatcatcgggacagcaaagtgtctcttca agagaaaaactgtgaacctgtgtagtgagcggc cgc | pMA2M insert |
| 10 | MVLYCLELAGIGILTVYMDGTAVLYCLELAGI | Epitope array from pVAXM2 and |
| | GILTVYMDGTMLAVLYCLELAGIGILTVYMD GTMSLLAVLYCLELAGIGILTV | pVAXM1 |
| 11 | NY-ESO-1 protein | Accession number: P78358 |
| 12 | SLLMWITQC | NY-ESO-1 157-165 |
| 13 | KASEKIFYV | SSX-241-49 |
| 14 | TQCFLPVFL | NY-ESO-1 163-171 |
| 15 | GLPSIPVHPI | PSMA 288-297 |
| 16 | AVLYCL | tyrosinase 4-9 |
| 17 | MSLLMWITQCKASEKIFYVRCGARGPESRLLE FYLAMPFATPMEAELARRSLAQDAPPLPVPGV LLKEFTVSGNILTIRLTAADHRQLQLSISSCLQ QLSLLMWITQCFLPVFLAQPPSGQRR | pN 157 expression product |
| 18 | MSLLMWITQCKASEKIFYV | liberation or substrate sequence for SEQ ID NO. 12 from pN157 |
| 19 | cttaagccaccatgtccctgttgatgtggatcacgcagtgcaa agcttcggagaaaatcttctacgtacggtgcgg tgccagggggccggagagccgcctgcttgagttctacctcgcc atgcctttcgcgacacccatggaagcagagctg gcccgcaggagcctggcccaggatgccccaccgcttcccgtgc caggggtgcttctgaaggagttcactgtgtccg gcaacatactgactatccgactgactgctgcagaccaccgcca actgcagctctccatcagctcctgtctccagca gctttccctgttgatgtggatcacgcagtgctttctgcccgtg tttttggctcagcctccctcagggcagaggcgc tagtgagaattc | Insert for pN157 |
| 20 | MSLLMWITQCKASEKIFYVGLPSIPVHPIGLPSI PVHPIKASEKIFYVSLLMWITQCKASEKIFYVK ASEKIFYVRCGARGPESRLLEFYLAMPFATPM EAELARRSLAQDAPPLPVPGVLLKEFTVSGNIL TIRLTAADHRQLQLSISSCLQQLSLLMWITQCF LPVFLAOPPSGORR | pBPL expression product |
| 21 | atgtccctgttgatgtggatcacgcagtgcaaagcttcggaga aaatcttctatgtgggtcttccaagtattcctg ttcatccaattggtcttccaagtattcctgttcatccaattaa agcttcggagaaaatcttctatgtgtccctgtt gatgtggatcacgcagtgcaaagcttcggagaaaatcttctat gtgaaagcttcggagaaaatcttctacgtacgg tgcggtgccaggggggccggagagccgcctgcttgagttctacc tcgccatgcctttcgcgacacccatggaagcag agctggcccgcaggagcctggcccaggatgccccaccgcttcc cgtgccaggggtgcttctgaaggagttcactgt gtccggcaacatactgactatccgactgactgctgcagaccac cgccaactgcagctctccatcagctcctgtctc cagcagctttccctgttgatgtggatcacgcagtgctttctgc ccgtgtttttggctcagcctccctcagggcaga ggcgctagtga | pBPL insert coding region |
| 22 | IKASEKIFYV**SLLMWITQC**KASEKIFYVK | Substrate in Fig. 6 |
| 23 | VMTKLGFKV | SSX-4₅₇₋₆₅ |
| 24 | RQIYVAAFTV | PSMA₇₃₀₋₇₃₉ |
| 25 | AQIPEKIQKAFDDIAKYFSKEEWEKMKASEKIF YVYMKRKYEAMTKLGFKATLPPFMCNKRAE DFQGNDLDNDPNRGNQVERPQMTFGRLQGIS PKIMPKKPAEEGNDSEEVPEASGPQNDGKELC PPGKPTTSEKIHERSGPKRGEHAWTHRLRERK QLVIYEEISDP | SSX-2₁₅₋₁₈₃ |
| 26 | | CTLS1/pCBP expression product |
| 27 | | CTLS2 expression product |
| 28 | MVMTKLGFKVKASEKIFYVRQIYVAAFTV GLPSIPVHPIAQIPEKIQKAFDDIAKYFSKEEWE KMKASEKIFYVYMKRKYEAMTKLGFKATLPP FMCNKRAEDFQGNDLDNDPNRGNQVERPQM TFGRLQGISPKIMPKKPAEEGNDSEEVPEASGP QNDGKELCPPGKPTTSEKIHERSGPKRGEHAW THRLRERKQLVIYEEISDP | CTLS3 expression product |
| 29 | MAQIPEKIQKAFDDIAKYFSKEEWEKMKASE KIFYVYMKRKYEAMTKLGFKATLPPFMCNKR AEDFQGNDLDNDPNRGNQVERPQMTFGRLQ GISPKIMPKKPAEEGNDSEEVPEASGPQNDGK ELCPPGKPTTSEKIHERSGPKRGEHAWTHRLR ERKQLVIYEEISDPTQCFLPVFLVMTKLGFKVR QIYVAAFTVKASEKIFYV | CTLS4 expression product |
| 30 | atggtcatgactaaactaggtttcaaggtcaaagcttcggaga aaatcttctatgtgagacagatttatgttgcag ccttcacagtgggtcttccaagtattcctgttcatccaattac gcagtgctttctgcccgtgtttttggtcatgac taaactaggtttcaaggtcagacagatttatgttgcagccttc acagtgaaagcttcggagaaaatcttctacgta gctcaaataccagagaagatccaaaaggccttcgatgatattg ccaaatacttctctaaggaagagtgggaaaaga tgaaagcctcggagaaaatcttctatgtgtatatgaagagaaa gtatgaggctatgactaaactaggtttcaaggc cacccctcccacctttcatgtgtaataaacgggccgaagacttc caggggaatgatttggataatgaccctaaccgt gggaatcaggttgaacgtcctcagatgactttcggcaggctcc agggaatctccccgaagatcatgcccaagaagc cagcagaggaaggaaatgattcggaggaagtgccagaagcatc tggcccacaaaatgatgggaaagagctgtgccc cccgggaaaaccaactacctctgagaagattcacgagagatct ggacccaaaaggggggaacatgcctggacccac agactgcgtgagagaaaacagctggtgatttatgaagagatca gcgacccttagtga | pCBP insert coding region |
| 31 | RQIYVAAFTVKASEKJFYVAQIPEKIQK | Fig. 8 substrate/ CTLS1-2 |
| 32 | FLPWHRLFL | TYR₂₀₇₋₂₁₅ |
| 33 | MLLAVLYCLLWSFQTSAFLPWHRLFLMLLAV LYCLLWSFQTSAFLPWHRLFLMLLAVLYCLL | CTLT2/pMEL expression product |
| | WSFQTSAFLPWHRLFLMLLAVLYCLLWSFQT SAFLPWHRLFL | |
| 34 | atgctcctggctgttttgtactgcctgctgtggagtttccaga cctccgcttttctgccttggcatagactcttct tgatgctcctggctgttttgtactgcctgctgtggagtttcca gacctccgcttttctgccttggcatagactctt cttgatgctcctggctgttttgtactgcctgctgtggagtttc cagacctccgcttttctgccttggcatagactc ttcttgatgctcctggctgttttgtactgcctgctgtggagtt tccagacctccgcttttctgccttggcatagac tcttcttgtagtga | CTLT2/pMEL insert coding region |
| | | |
| 35 | MELAN-A cDNA | Accession number: NM 005511 |
| 36 | Tyrosinase cDNA | Accession number: NM 000372 |
| 37 | NY-ESO-1 cDNA | Accession number: U87459 |
| 38 | PSMA protein | Accession number: NP 004467 |
| 39 | PSMA cDNA | Accession number: NM 004476 |
| 40 | SSX-2 protein | Accession number: NP 003138 |
| 41 | SSX-2 cDNA | Accession number: NM 003147 |
| 42 | atgacctctcgccgctccgtgaagtcgggtccgcgggaggttccgcgc gatgagtacgaggatctgtactacaccccgtcttcaggtatggcgagtcc cgatagtccgcctgacacctcccgccgtggcgccctacagacacgctc gcgccagagggcgaggtccgtttcgtccagtacgacgagtcggattat gccctctacgggggctcgtcatccgaagacgacgaacacccggaggt cccccggacgcggcgtcccgtttccggggcggttttgtccggcccggg gcctgcgcgggcgcctccgccacccgctgggtccggagggggccgga cgcacacccaccaccgcccccccgggcccccgaacccagcgggtgg cgactaaggcccccgcggccccggcggcggagaccacccgcggca ggaaatcggcccagccagaatccgccgcactcccagacgcccccgcg tcgacggcgccaacccgatccaagacacccgcgcaggggctggcca gaaagctgcactttagcaccgccccccccaaaccccgacgcgccatgga cccccccgggtggccggctttaacaagcgcgtcttctgcgccgcggtcg ggcgcctggcggccatgcatgcccggatggcggcggtccagctctgg gacatgtcgcgtccgcgcacagacgaagacctcaacgaactccttggc atcaccaccatccgcgtgacggtctgcgagggcaaaaacctgcttcag cgcgccaacgagttggtgaatccagacgtggtgcaggacgtcgacgcg gccacggcgactcgagggcgttctgcggcgtcgcgccccaccgagcg acctcgagccccagcccgctccgcttctcgccccagacggcccgtcga g | From accession number: D10879 Herpes Simplex virus 1 UL49 coding sequence (VP22) |
| 43 | MTSRRSVKSGPREVPRDEYEDLYYTPSSGMAS PDSPPDTSRRGALFTQTRSRQRGEVRFVQYDE SDYALYGGSSSEDDEHPEVPRTRRPVSGAVLS GPGPARAPPPFTPAGSGGAGRTPTTAPRAPRT QRVATKAPAAPAAETTRGRKSAQPESAALPD APASTAPTFTRSKTPAQGLARKLHFSTAPPNP DAPWTPRVAGFNKRVFCAAVGRLAAMHARM AAVQLWDFTMSRPRTDEDLNELLGITTIRVTV CEGKNLLQRANELVNPDVVQDVDAATATRG RSAASRFTPTERPRAPARSASRPRRPVE | Accession number: P10233 Herpes Simplex virus 1 UL49/VP22 protein sequence |

### Melan-A mRNA sequence

LOCUS NM_005511 1524 bp mRNA PRI 14-OCT-2001
DEFINITION Homo sapiens melan-A (MLANA), mRNA.
ACCESSION NM_005511
VERSION NM_005511.1 GI:5031912

(SEQ ID NO. 2)
/translation="MPREDAHFIYGYPKKGHGHSYTTAEEAAGIGILTVILGVLLLIGCWYCRRRNGY
RALMDKSLHVGTQCALTRRCPQEGFDHRDSKVSLQEKNCEPVVPNAPPAYEKLSAEQSPPP
YSP"

(SEQ ID NO. 35)
ORIGIN
1 agcagacaga ggactctcat taaggaaggt gtcctgtgcc ctgaccctac aagatgccaa
61 gagaagatgc tcacttcatc tatggttacc ccaagaaggg gcacggccac tcttacacca
121 cggctgaaga ggccgctggg atcggcatcc tgacagtgat cctgggagtc ttactgctca
181 tcggctgttg gtattgtaga agacgaaatg gatacagagc cttgatggat aaaagtcttc
241 atgttggcac tcaatgtgcc ttaacaagaa gatgcccaca agaagggttt gatcatcggg
301 acagcaaagt gtctcttcaa gagaaaaact gtgaacctgt ggttcccaat gctccacctg
361 cttatgagaa actctctgca gaacagtcac caccacctta ttcaccttaa gagccagcga
421 gacacctgag acatgctgaa attatttctc tcacactttt gcttgaattt aatacagaca
481 tctaatgttc tcctttggaa tggtgtagga aaaatgcaag ccatctctaa taataagtca
541 gtgttaaaat tttagtaggt ccgctagcag tactaatcat gtgaggaaat gatgagaaat
601 attaaattgg gaaaactcca tcaataaatg ttgcaatgca tgatactatc tgtgccagag
661 gtaatgttag taaatccatg gtgttatttt ctgagagaca gaattcaagt gggtattctg
721 gggccatcca atttctcttt acttgaaatt tggctaataa caaactagtc aggttttcga
781 accttgaccg acatgaactg tacacagaat tgttccagta ctatggagtg ctcacaaagg
841 atacttttac aggttaagac aaagggttga ctggcctatt tatctgatca agaacatgtc
901 agcaatgtct ctttgtgctc taaaattcta ttatactaca ataatatatt gtaaagatcc
961 tatagctctt tttttttgag atggagtttc gcttttgttg cccaggctgg agtgcaatgg
1021 cgcgatcttg gctcaccata acctccgcct cccaggttca agcaattctc ctgccttagc
1081 ctcctgagta gctgggatta caggcgtgcg ccactatgcc tgactaattt tgtagtttta
1141 gtagagacgg ggtttctcca tgttggtcag gctggtctca aactcctgac ctcaggtgat

1201 ctgcccgcct cagcctccca aagtgctgga attacaggcg tgagccacca cgcctggctg
1261 gatcctatat cttaggtaag acatataacg cagtctaatt acatttcact tcaaggctca
1321 atgctattct aactaatgac aagtattttc tactaaacca gaaattggta gaaggattta
1381 aataagtaaa agctactatg tactgcctta gtgctgatgc ctgtgtactg ccttaaatgt
1441 acctatggca atttagctct cttgggttcc caaatccctc tcacaagaat gtgcagaaga
1501 aatcataaag gatcagagat tctg

### Tyrosinase mRNA sequence

LOCUS NM_000372 1964 bp mRNA PRI 31-OCT-2000
DEFINITION Homo sapiens tyrosinase (oculocutaneous albinism IA) (TYR), mRNA.
ACCESSION NM_000372
VERSION NM_000372.1 GI:4507752

(SEQ ID NO. 3)
/translation="







"

(SEQ ID NO. 36)
ORIGIN
1 atcactgtag tagtagctgg aaagagaaat ctgtgactcc aattagccag ttcctgcaga
61 ccttgtgagg actagaggaa gaatgctcct ggctgttttg tactgcctgc tgtggagttt
121 ccagacctcc gctggccatt tccctagagc ctgtgtctcc tctaagaacc tgatggagaa
181 ggaatgctgt ccaccgtgga gcggggacag gagtccctgt ggccagcttt caggcagagg
241 ttcctgtcag aatatccttc tgtccaatgc accacttggg cctcaatttc ccttcacagg
301 ggtggatgac cgggagtcgt ggccttccgt cttttataat aggacctgcc agtgctctgg

361 caacttcatg ggattcaact gtggaaactg caagtttggc ttttggggac caaactgcac
421 agagagacga ctcttggtga gaagaaacat cttcgatttg agtgccccag agaaggacaa
481 attttttgcc tacctcactt tagcaaagca taccatcagc tcagactatg tcatccccat
541 agggacctat ggccaaatga aaaatggatc aacacccatg tttaacgaca tcaatattta
601 tgacctcttt gtctggatgc attattatgt gtcaatggat gcactgcttg gggatctga
661 aatctggaga gacattgatt ttgcccatga agcaccagct tttctgcctt ggcatagact
721 cttcttgttg cggtgggaac aagaaatcca gaagctgaca ggagatgaaa acttcactat
781 tccatattgg gactggcggg atgcagaaaa gtgtgacatt tgcacagatg agtacatggg
841 aggtcagcac cccacaaatc ctaacttact cagcccagca tcattcttct cctcttggca
901 gattgtctgt agccgattgg aggagtacaa cagccatcag tctttatgca atggaacgcc
961 cgagggacct ttacggcgta atcctggaaa ccatgacaaa tccagaaccc caaggctccc
1021 ctcttcagct gatgtagaat tttgcctgag tttgacccaa tatgaatctg gttccatgga
1081 taaagctgcc aatttcagct ttagaaatac actggaagga tttgctagtc cacttactgg
1141 gatagcggat gcctctcaaa gcagcatgca caatgccttg cacatctata tgaatggaac
1201 aatgtcccag gtacagggat ctgccaacga tcctatcttc cttcttcacc atgcatttgt
1261 tgacagtatt tttgagcagt ggctccgaag gcaccgtcct cttcaagaag tttatccaga
1321 agccaatgca cccattggac ataaccggga atcctacatg gttcctttta taccactgta
1381 cagaaatggt gatttcttta tttcatccaa agatctgggc tatgactata gctatctaca
1441 agattcagac ccagactctt ttcaagacta cattaagtcc tatttggaac aagcgagtcg
1501 gatctggtca tggctccttg gggcggcgat ggtaggggcc gtcctcactg ccctgctggc
1561 agggcttgtg agcttgctgt gtcgtcacaa gagaaagcag cttcctgaag aaaagcagcc
1621 actcctcatg gagaaagagg attaccacag cttgtatcag agccatttat aaaaggctta
1681 ggcaatagag tagggccaaa aagcctgacc tcactctaac tcaaagtaat gtccaggttc
1741 ccagagaata tctgctggta tttttctgta aagaccattt gcaaaattgt aacctaatac
1801 aaagtgtagc cttcttccaa ctcaggtaga acacacctgt ctttgtcttg ctgttttcac
1861 tcagcccttt taacattttc ccctaagccc atatgtctaa ggaaaggatg ctatttggta
1921 atgaggaact gttatttgta tgtgaattaa agtgctctta tttt

### NY-ESO-1 mRNA sequence

LOCUS HSU87459 752 bp mRNA PRI 22-DEC-1999
DEFINITION Human autoimmunogenic cancer/testis antigen NY-ESO-1 mRNA, complete cds.
ACCESSION U87459
VERSION U87459.1 GI:1890098

(SEQ ID NO. 11)
/translation="MQAEGRGTGGSTGDADGPGGPGIPDGPGGNAGGPGEAGATGGRGPRGAGAAR
ASGPGGGAPRGPHGGAASGLNGCCRCGARGPESRLLEFYLAMPFATPMEAELARRSLAQDA
PPLPVPGVLLKEFTVSGNILTIRLTAADHRQLQLSISSCLQQLSLLM
WITQCFLPVFLAQPPSGQRR"

(SEQ ID NO. 37)
ORIGIN
1 atcctcgtgg gccctgacct tctctctgag agccgggcag aggctccgga gccatgcagg
61 ccgaaggccg gggcacaggg ggttcgacgg gcgatgctga tggcccagga ggccctggca
121 ttcctgatgg cccagggggc aatgctggcg gcccaggaga ggcgggtgcc acgggcggca
181 gaggtccccg gggcgcaggg gcagcaaggg cctcggggcc gggaggaggc gccccgcggg
241 gtccgcatgg cggcgcggct tcagggctga atggatgctg cagatgcggg gccagggggc
301 cggagagccg cctgcttgag ttctacctcg ccatgccttt cgcgacaccc atggaagcag
361 agctggcccg caggagcctg gcccaggatg ccccaccgct tcccgtgcca ggggtgcttc
421 tgaaggagtt cactgtgtcc ggcaacatac tgactatccg actgactgct gcagaccacc
481 gccaactgca gctctccatc agctcctgtc tccagcagct ttccctgttg atgtggatca
541 cgcagtgctt tctgcccgtg tttttggctc agcctccctc agggcagagg cgctaagccc
601 agcctggcgc cccttcctag gtcatgcctc ctcccctagg gaatggtccc agcacgagtg
661 gccagttcat tgtgggggcc tgattgtttg tcgctggagg aggacggctt acatgtttgt
721 ttctgtagaa aataaaactg agctacgaaa aa

### PSMA cDNA sequence

LOCUS NM_004476 2653 bp mRNA PRI 01-NOV-2000
DEFINITION Homo sapiens folate hydrolase (prostate-specific membrane antigen) I (FOLH1), mRNA.
ACCESSION NM_004476
VERSION NM_004476.1 GI:4758397

(SEQ ID NO. 38)
/translation="MWNLLHETDSAVATARRPRWLCAGALVLAGGFFLLGFLFGWFIKSSNEATNIT
PKHNMKAFLDELKAENIKKFLYNFTQIPHLAGTEQNFQLAKQIQSQWKEFGLDSVELAHYD
VLLSYPNKTHPNYISIINEDGNEIFNTSLFEPPPPGYENVSDIVPPFSAFSPQGMPEGDLVYVNY
ARTEDFFKLERDMKINCSGKIVIARYGKVFRGNKVKNAQLAGAKGVILYSDPADYFAPGVK
SYPDGWNLPGGGVQRGNILNLNGAGDPLTPGYPANEYAYRRGIAEAVGLPSIPVHPIGYYDA
QKLLEKMGGSAPPDSSWRGSLKVPYNVGPGFTGNFSTQKVKMHIHSTNEVTRIYNVIGTLRG
AVEPDRYVILGGHRDSWVFGGIDPQSGAAVVHEIVRSFGTLKKEGWRPRRTILFASWDAEEF
GLLGSTEWAEENSRLLQERGVAYINADSSIEGNYTLRVDCTPLMYSLVHNLTKELKSPDEGF
EGKSLYESWTKKSPSPEFSGMPRISKLGSGNDFEVFFQRLGIASGRARYTKNWETNKFSGYPL
YHSVYETYELVEKFYDPMFKYHLTVAQVRGGMVFELANSIVLPFDCRDYAVVLRKYADKIY
SISMKHPQEMKTYSVSFDSLFSAVKNFTEIASKFSERLQDFDKSNPIVLRMMNDQLMFLERAF
IDPLGLPDRPFYRHVIYAPSSHNKYAGESFPGIYDALFDIESKVDPSKAWGEVKRQIYVAAFT
VQAAAETLSEVA"

(SEQ ID NO. 39)
ORIGIN
1 ctcaaaaggg gccggatttc cttctcctgg aggcagatgt tgcctctctc tctcgctcgg
61 attggttcag tgcactctag aaacactgct gtggtggaga aactggaccc caggtctgga
121 gcgaattcca gcctgcaggg ctgataagcg aggcattagt gagattgaga gagactttac
181 cccgccgtgg tggttggagg gcgcgcagta gagcagcagc acaggcgcgg gtcccgggag
241 gccggctctg ctcgcgccga gatgtggaat ctccttcacg aaaccgactc ggctgtggcc
301 accgcgcgcc gcccgcgctg gctgtgcgct ggggcgctgg tgctggcggg tggcttcttt
361 ctcctcggct tcctcttcgg gtggtttata aaatcctcca atgaagctac taacattact
421 ccaaagcata atatgaaagc atttttggat gaattgaaag ctgagaacat caagaagttc
481 ttatataatt ttacacagat accacattta gcaggaacag aacaaaactt tcagcttgca
541 aagcaaattc aatcccagtg gaaagaattt ggcctggatt ctgttgagct agcacattat
601 gatgtcctgt tgtcctaccc aaataagact catcccaact acatctcaat aattaatgaa
661 gatggaaatg agattttcaa cacatcatta tttgaaccac ctcctccagg atatgaaaat
721 gtttcggata ttgtaccacc tttcagtgct ttctctcctc aaggaatgcc agagggcgat
781 ctagtgtatg ttaactatgc acgaactgaa gacttcttta aattggaacg ggacatgaaa

841 atcaattgct ctgggaaaat tgtaattgcc agatatggga aagttttcag aggaaataag
901 gttaaaaatg cccagctggc aggggccaaa ggagtcattc tctactccga ccctgctgac
961 tactttgctc ctggggtgaa gtcctatcca gatggttgga atcttcctgg aggtggtgtc
1021 cagcgtggaa atatcctaaa tctgaatggt gcaggagacc ctctcacacc aggttaccca
1081 gcaaatgaat atgcttatag gcgtggaatt gcagaggctg ttggtcttcc aagtattcct
1141 gttcatccaa ttggatacta tgatgcacag aagctcctag aaaaaatggg tggctcagca
1201 ccaccagata gcagctggag aggaagtctc aaagtgccct acaatgttgg acctggcttt
1261 actggaaact tttctacaca aaaagtcaag atgcacatcc actctaccaa tgaagtgaca
1321 agaatttaca atgtgatagg tactctcaga ggagcagtgg aaccagacag atatgtcatt
1381 ctgggaggtc accgggactc atgggtgttt ggtggtattg accctcagag tggagcagct
1441 gttgttcatg aaattgtgag gagctttgga acactgaaaa aggaagggtg gagacctaga
1501 agaacaattt tgtttgcaag ctgggatgca gaagaatttg gtcttcttgg ttctactgag
1561 tgggcagagg agaattcaag actccttcaa gagcgtggcg tggcttatat taatgctgac
1621 tcatctatag aaggaaacta cactctgaga gttgattgta caccgctgat gtacagcttg
1681 gtacacaacc taacaaaaga gctgaaaagc cctgatgaag gctttgaagg caaatctctt
1741 tatgaaagtt ggactaaaaa aagtccttcc ccagagttca gtggcatgcc caggataagc
1801 aaattgggat ctggaaatga ttttgaggtg ttcttccaac gacttggaat tgcttcaggc
1861 agagcacggt atactaaaaa ttgggaaaca aacaaattca gcggctatcc actgtatcac
1921 agtgtctatg aaacatatga gttggtggaa aagttttatg atccaatgtt taaatatcac
1981 ctcactgtgg cccaggttcg aggagggatg gtgtttgagc tagccaattc catagtgctc
2041 ccttttgatt gtcgagatta tgctgtagtt ttaagaaagt atgctgacaa aatctacagt
2101 atttctatga aacatccaca ggaaatgaag acatacagtg tatcatttga ttcacttttt
2161 tctgcagtaa agaattttac agaaattgct tccaagttca gtgagagact ccaggacttt
2221 gacaaaagca acccaatagt attaagaatg atgaatgatc aactcatgtt tctggaaaga
2281 gcatttattg atccattagg gttaccagac aggccttttt ataggcatgt catctatgct
2341 ccaagcagcc acaacaagta tgcaggggag tcattcccag gaatttatga tgctctgttt
2401 gatattgaaa gcaaagtgga cccttccaag gcctggggag aagtgaagag acagatttat
2461 gttgcagcct tcacagtgca ggcagctgca gagactttga gtgaagtagc ctaagaggat
2521 tctttagaga atccgtattg aatttgtgtg gtatgtcact cagaaagaat cgtaatgggt
2581 atattgataa attttaaaat tggtatattt gaaataaagt tgaatattat atataaaaaa
2641 aaaaaaaaaa aaa

| NM 003147 | **Homo sapiens synovial sarcoma, X breakpoint 2 (SSX2), mRNA** |
|---|---|
| LOCUS | NM_003147 766 bp mRNA PRI 14-MAR-2001 |
| DEFINITION | Homo sapiens synovial sarcoma, X breakpoint 2 (SSX2), mRNA. |
| ACCESSION | NM_003147 |
| VERSION | NM_003147.1 GI:10337582 |

SEQ ID NO. 40
/translation="MNGDDAFARRPTVGAQIPEKIQKAFDDIAKYFSKEEWEKMKASE
KIFYVYMKRKYEAMTKLGFKATLPPFMCNKRAEDFQGNDLDNDPNRGNQVERPQMTFG
RLQGISPKIMPKKPAEEGNDSEEVPEASGPQNDGKELCPPGKPTTSEKIHERSGPKRG
EHAWTHRLRERKQLVIYEEISDPEEDDE"

SEQ ID NO 41
1 ctctctttcg attcttccat actcagagta cgcacggtct gattttctct ttggattctt
61 ccaaaatcag agtcagactg ctcccggtgc catgaacgga gacgacgcct ttgcaaggag
121 acccacggtt ggtgctcaaa taccagagaa gatccaaaag gccttcgatg atattgccaa
181 atacttctct aaggaagagt gggaaaagat gaaagcctcg gagaaaatct tctatgtgta
241 tatgaagaga aagtatgagg ctatgactaa actaggtttc aaggccaccc tcccaccttt
301 catgtgtaat aaacgggccg aagacttcca ggggaatgat ttggataatg accctaaccg
361 tgggaatcag gttgaacgtc ctcagatgac tttcggcagg ctccagggaa tctccccgaa
421 gatcatgccc aagaagccag cagaggaagg aaatgattcg gaggaagtgc cagaagcatc
481 tggcccacaa aatgatggga aagagctgtg cccccccgga aaaccaacta cctctgagaa
541 gattcacgag agatctggac ccaaaagggg ggaacatgcc tggacccaca gactgcgtga
601 gagaaaacag ctggtgattt atgaagagat cagcgaccct gaggaagatg acgagtaact
661 cccctcaggg atacgacaca tgcccatgat gagaagcaga acgtggtgac ctttcacgaa
721 catgggcatg gctgcggacc cctcgtcatc aggtgcatag caagtg

## Claims

1. A polypeptide comprising SEQ ID NO. 4 or SEQ ID NO. 5.

2. A polypeptide according to claim 1 for use in immunizing a mammal.

3. A vector encoding the polypeptide of claim 1, the vector comprising the sequence of SEQ ID NO. 9.

4. A vector encoding the polypeptide of claim 1 comprising the sequence of SEQ ID NO. 4.

5. A cell transformed with a vector according to claims 3 or 4.

6. An in vitro method of activating a T cell comprising contacting the vector of claim 3 or 4 with an APC and contacting said APC with a T cell.

7. The vector according to claim 3 or 4 for use in immunizing a mammal.

## Patentansprüche

1. Polypeptid umfassend SEQ ID NO. 4 oder SEQ ID NO. 5.

2. Polypeptid nach Anspruch 1 zur Verwendung in der Immunisierung eines Säugetiers.

3. Vektor, der für das Polypeptid nach Anspruch 1 kodiert und die Sequenz von SEQ ID NO. 9 umfasst.

4. Vektor, der für das Polypeptid nach Anspruch 1, umfassend die Sequenz von SEQ ID NO. 4, kodiert.

5. Zelle, die mit einem Vektor nach einem der Ansprüche 3 oder 4 transformiert ist.

6. In vitro-Verfahren zur Aktivierung einer T-Zelle, umfassend das Inkontaktbringen des Vektors nach einem der Ansprüche 3 oder 4 mit einer Antigen-präsentierenden Zelle (APC) und das Inkontaktbringen dieser Antigen-präsentierenden Zelle mit einer T-Zelle.

7. Vektor nach einem der Ansprüche 3 oder 4 zur Verwendung in der Immunisierung eines Säugetiers.

## Revendications

1. Un polypeptide comprenant SEQ ID n° 4 ou SEQ ID n° 5.

2. Un polypeptide selon la revendication 1, destiné à être utilisé dans l'immunisation d'un mammifère.

3. Un vecteur codant le polypeptide de la revendication 1, le vecteur comprenant la séquence de SEQ ID n° 9.

4. Un vecteur codant le polypeptide de la revendication 1, comprenant la séquence de SEQ ID n° 4.

5. Une cellule transformée au moyen d'un vecteur selon la revendication 3 ou 4.

6. Un procédé in vitro d'activation d'une cellule T comportant la mise en contact du vecteur de la revendication 3 ou 4 avec une APC et la mise en contact de ladite APC avec une cellule T.

7. Le vecteur selon la revendication 3 ou 4, destiné à être utilisé dans l'immunisation d'un mammifère.
